# EUROPÄISCHE PATENTSCHRIFT

(11) **EP 1 836 180 B1**
(45) Veröffentlichungstag und Bekanntmachung des Hinweises auf die Patenterteilung: **13.06.2012**
(21) Anmeldenummer: 05822649.9
(22) Anmeldetag: 21.12.2005
(51) Int. Cl.: C07D 295/22, C07D 401/12, C07D 401/14, C07D 405/04, C07D 405/14, C07D 409/04, C07D 409/12, C07D 413/04, C07D 413/12, C07D 413/14, A61K 31/4025, A61K 31/497, A61P 3/12, A61P 9/00, C07D 295/26

(54) **SULFONYLPYRROLIDINE, VERFAHREN ZU IHRER HERSTELLUNG UND IHRE VERWENDUNG ALS ARZNEIMITTEL**
SULFONYL PYRROLIDINES, METHOD FOR PRODUCING THE SAME AND THEIR USE AS DRUGS
SULFONYLPYRROLIDINES, PROCEDE POUR LES PRODUIRE ET LEUR UTILISATION COMME MEDICAMENTS

(30) Priorität: 04.01.2005 DE 102005000666
(43) Veröffentlichungstag der Anmeldung: 26.09.2007
(73) Patentinhaber: SANOFI, 75008 Paris (FR)
(72) Erfinder: KEIL, Stefanie, 65719 Hofheim (DE); SCHAEFER, Hans-Ludwig, 65239 Hochheim (DE); GLIEN, Maike, 65195 Wiesbaden (DE); GUESSREGEN, Stefan, 65205 Wiesbaden (DE); WENDLER, Wolfgang, 65618 Selters (DE); ESSWEIN, Marion, 65719 Hofheim (DE)
(74) Vertreter: Fischer, Hans-Jürgen
(86) Internationale Anmeldenummer: PCT/EP2005/013772
(87) Internationale Veröffentlichungsnummer: WO 2006/072393

(56) Entgegenhaltungen:
- WO-A-00/02850
- WO-A-00/02851
- WO-A-94/07496
- WO-A-2005/040109

## Beschreibung

Sulfonylpyrrolidine, Verfahren zu ihrer Herstellung und ihre Verwendung als Arzneimittel

Die Erfindung betrifft substituierte Sulfonylpyrrolidine sowie deren physiologisch verträgliche Salze.

Es sind bereits strukturähnliche Verbindungen als Oxitocin Rezeptor Antagonisten in WO 94/07496 beschrieben.

Der Erfindung lag die Aufgabe zugrunde, Verbindungen zur Verfügung zu stellen, mit denen eine Prävention und Behandlung von Dyslipidämie, Erkrankungen des Herz-Kreislaufsystems und atherosklerotischen Erkrankungen möglich ist. Insbesondem sollten sich die Verbindungen zur Prävention und Behandlung von niedrigen HDL-Spiegeln (HDL bedeutet High Density Lipoproteins) eignen.

Die Erfindung betrifft daher Verbindungen der Formel I, 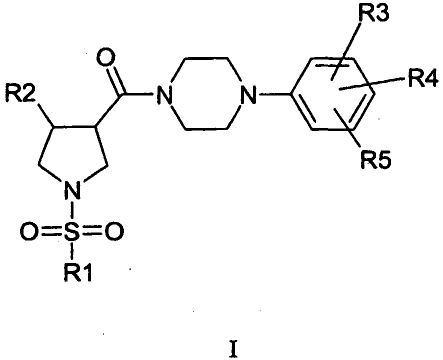 worin bedeuten
- R1: (C₁-C₆)-Alkyl, wobei in dem Alkylrest ein oder mehrere Wasserstoffe durch Fluor ersetzt sein können, Phenyl, (C₁-C₈)-Alkylen-Phenyl, Heterocyclus, (C₁-C₈)-Alkylen-Heterocyclus, wobei der Phenyl- oder Heterocyclus-rest ein oder mehrfach substituiert sein kann mit F, Cl, Br, NO₂, COO-(C₁-C₆)-Alkyl, (C₁-C₆)-, Alkylen-O-(C₁-C₆)-alkyl, OCF₃ (C₁-C₆)-Alkyl, CO-(C₁-C₆)-Alkyl, Phenyl, SCF₃, SF₅;
- R2: (C₁-C₆)-Alkyl, Phenyl, (C₁-C₈)-Alkylen-Phenyl, Heterocyclus, (C₁-C₈)-Alkylen-Heterocyclus, wobei der Phenyl- oder Heterocyclus-rest ein oder mehrfach substituiert sein kann mit F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
- R3, R4, R5: unabhängig voneinander H, F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
wobei die Verbindungen der folgenden vier Formeln ausgeschlossen sind 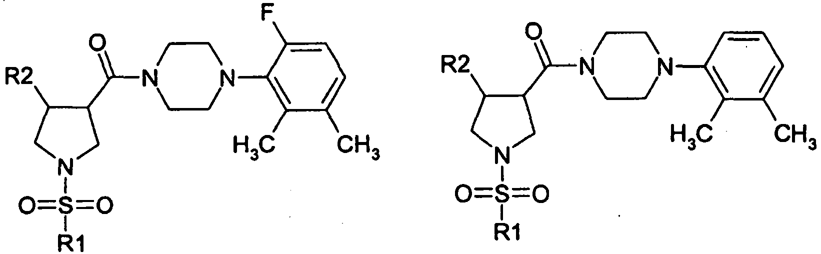 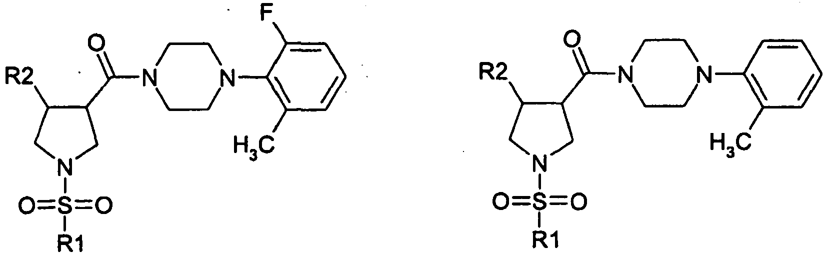 sowie deren physiologisch verträgliche Salze.

Bevorzugt sind Verbindungen der Formel I worin ein oder mehrere Reste folgende Bedeutung haben:
- R1: (C₁-C₆)-Alkyl, wobei in dem Alkylrest ein oder mehrere Wasserstoffe durch Fluor ersetzt sein können, Phenyl, (C₁-C₈)-Alkylen-Phenyl, Heterocyclus, (C₁-C₈)-Alkylen-Heterocyclus, wobei der Phenyl- oder Heterocyclus-rest ein oder mehrfach substituiert sein kann mit F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
- R2: Phenyl, Heterocyclus, wobei der Phenyl- oder Heterocyclus-rest ein oder mehrfach substituiert sein kann mit F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
- R3, R4, R5: unabhängig voneinander H, F, Cl, Br, NO₂, COO-(C)-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
wobei die Verbindungen der folgenden vier Formeln ausgeschlossen sind 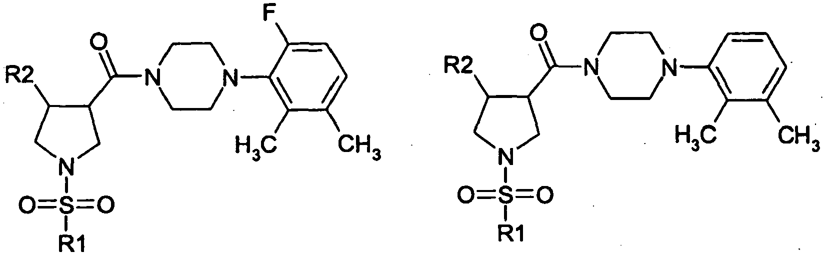 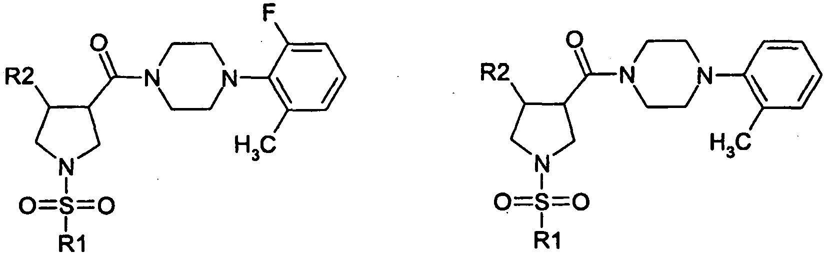 sowie deren physiologisch verträgliche Salze.

Besonders bevorzugt sind Verbindungen der Formel I, die die Struktur Ia besitzen: 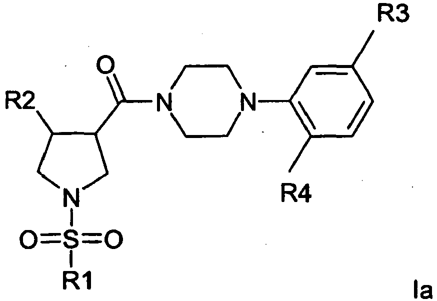 worin ein oder mehrere Reste folgende Bedeutung haben:
- R1: (C₁-C₆)-Alkyl, wobei in dem Alkylrest ein oder mehrere Wasserstoffe durch Fluor ersetzt sein können, Phenyl, (C₁-C₈)-Alkylen-Phenyl, Heterocyclus, (C₁- C₈)-Alkylen-Heterocyclus, wobei der Phenyl- oder Heterocyclus-rest ein oder mehrfach substituiert sein kann mit F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
- R2: Phenyl, Heterocyclus, wobei der Phenyl- oder Heterocyclus-rest ein oder mehrfach substituiert sein kann mit F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
- R3, R4: unabhängig voneinander H, F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
wobei die Verbindung der folgenden Formel ausgeschlossen ist 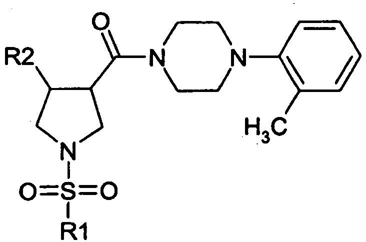 sowie deren physiologisch verträgliche Salze.

Ganz besonders bevorzugt sind Verbindungen der Formel I, die die Struktur Ia besitzen: 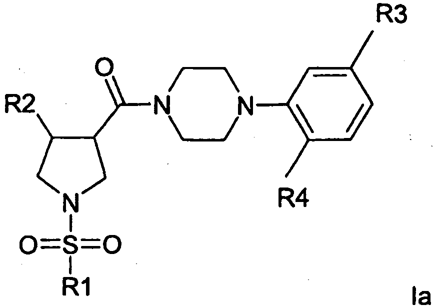 worin ein oder mehrere Reste folgende Bedeutung haben:
- R1: (C₁-C₆)-Alkyl, wobei in dem Alkylrest ein oder mehrere Wasserstoffe durch Fluor ersetzt sein können, Phenyl, (C₁-C₈)-Alkylen-Phenyl, Heterocyclus, (C₁-C₈)-Alkylen-Heterocyclus, wobei der Phenyl- oder Heterocyclus-rest ein oder mehrfach substituiert sein kann mit F, Cl, Br, NO₂, COO=(C₁-C₆)-Alkyl, (C₁-C₆)-Alkylen-O-(C₁-C₆)-alkyl, OCF₃, (C₁-C₆)-Alkyl, CO-(C₁-C6)-Alkyl, Phenyl, SCF₃, SF₅ und der Heterocylus aus der Gruppe Thiophen, Quinolin, Oxadiazol, Isoxazol und Pyridin ausgewählt ist und mit einem Benzolring anneliert sein kann.
- R2: Phenyl, Heterocyclus, wobei der Phenyl- oder Heterocyclus-rest ein oder mehrfach substituiert sein kann mit F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅ und der Heterocylus aus der Gruppe Dioxol, Tetrahydrofuran, Isoxazol, Oxazin, Thiophen und Pyridin ausgewählt ist und mit einem Benzolring anneliert sein kann.
- R3, R4: unabhängig voneinander H, F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
wobei die Verbindung der folgenden Formel ausgeschlossen ist 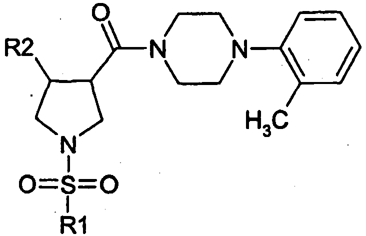 sowie deren physiologisch verträgliche Salze.

Die Erfindung bezieht sich auf Verbindungen der Formel I, in Form ihrer Racemate, racemischen Mischungen und reinen Enantiomere sowie auf ihre Diastereomere und Mischungen davon.

Können Reste oder Substituenten mehrfach in den Verbindungen der Formel I auftreten, so können sie alle unabhängig voneinander die angegebenen Bedeutungen haben und gleich oder verschieden sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Ein weiterer Aspekt der Erfindung sind die physiologisch funktionellen Derivate der Verbindungen der Formel I. Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Unter einem Alkylrest wird eine geradkettige oder verzweigte Kohlenwasserstoffkette mit einem oder mehreren Kohlenstoffen verstanden, wie z.B. Methyl, Ethyl, iso-Propyl, tert.-Butyl, Hexyl.

Die Alkylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)A1kyl, CON[(C₁-C₆)Alkyl]₂, Cycloalkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH2)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH2)ₙ-Aryl)₂, , SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)-Alkyl, N(C₁-C₆)-Alkyl -COO-(C₁-C₆)-Alkyl, N(C₁-C₆)-Alkyl - CO-Aryl, N(C₁-C₆)-Alkyl -CO-Heterocyclus, N(C₁-C₆)-Alkyl -COO-Aryl, N(C₁-C₆)-Alkyl - COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Alkenylrest wird eine geradkettige oder verzweigte Kohlenwasserstoffkette mit zwei oder mehreren Kohlenstoffen sowie einer oder mehreren Doppelbindungen verstanden, wie z.B. Vinyl, Allyl, Pentenyl.

Die Alkenylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, , SO₂-N((CH₂)ₙ-Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C6)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)-Alkyl, N(C₁-C₆)-Alkyl -COO-(C₁-C₆)-Alkyl, N(C₁-C₆)-Alkyl - CO-Aryl, N(C₁-C₆)-Alkyl -CO-Heterocyclus, N(C₁-C₆)-Alkyl -COO-Aryl, N(C₁-C₆)-Alkyl - COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Alkinylrest wird eine geradkettige oder verzweigte Kohlenwasserstoffkette mit zwei oder mehreren Kohlenstoffen sowie einer oder mehreren Dreifachbindungen verstanden, wie z.B. Ethinyl, Propinyl, Hexinyl.

Die Alkinylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₁-C₁₀)-Alkyl, O-(C₁-C₆)-Alkyl O-CO-(C₁-C₆)-Alkyl, O-CO-(C₁-C₆)-Aryl, O-CO-(C₁-C₆)-Heterocyclus;
PO₃H₂, SO₃H, SO₂-NH₂, SO₂NH(C₁-C₆)-Alkyl, SO₂N[(C₁-C₆)-Alkyl)₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, , SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C6)-Alkyl, N(C₁-C6)-Alkyl -COO-(C₁-C₆)-Alkyl, N(C₁-C₆)-Alkyl ₋ CO-Aryl, N(C₁-C₆)-Alkyl -CO-Heterocyclus, N(C₁-C₆)-Alkyl -COO-Aryl, N(C₁-C₆)-Alkyl - COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heteroeyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Arylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl)₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus,; PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH2)ₙ-Aryl, SO₂-N(C₁-C₆)-Alkyl)(CH₂)ₙ-Heterocyclus, SO₂-N((CH₂)ₙ-Aryl)₂, , SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)-Alkyl, N(C₁-C₆)-Alkyl-COO-(C₁-C₆)-Alkyl, N(C₁-C₆)-Alkyl - CO-Aryl, N(C₁-C₆)-Alkyl -CO-Heterocyclus, N(C₁-C₆)-Alkyl -COO-Aryl, N(C₁-C₆)-Alkyl - COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.

Die Cycloalkylrestereste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus; PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH2)ₙ-Aryl, SO₂-N(C₁-C₆)-Alkyl)(CH₂)ₙ-Heterocyclus, SO₂-N((CH₂)ₙ-Aryl)₂, , SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)-Alkyl, N(C₁-C₆)-Alkyl -COO-(C₁-C₆)-Alkyl, N(C₁-C₆)-Alkyl - CO-Aryl, N(C₁-C₆)-Alkyl -CO-Heterocyclus, N(C₁-C₆)-Alkyl -COO-Aryl, N(C₁-C₆)-Alkyl - COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heteroeyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter Heterocyclus bzw. Heterocyclischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der Heterocyclische Rest mit Benzolkernen kondensiert ist.

Geeignete "Heterocyclen" bzw. "Heterocyclische Reste" sind Acridinyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-pyridyl.

Umfasst sind weiterhin ein oder mehrfach benzoannelierte Derivate dieser Heterocyclen.

Die Heterocyclen bzw. Heterocyclischen Reste können ein oder mehrfach mit geeigneten Gruppen substituiert sein, wie z.B: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, , SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH2), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl-CO-(C₁-C₆)-Alkyl, N(C₁-C₆)-Alkyl -COO-(C₁-C₆)-Alkyl, N(C₁-C₆)-Alkyl-CO-Aryl, N(C₁-C₆)-Alkyl -CO-Heterocyclus, N(C₁-C₆)-Alkyl -COO-Aryl, N(C₁-C₆)-Alkyl - COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Kilogramm Körpergewicht pro Tag, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die Verbindungen der Formel I können allein, aber oder auch in Kombination mit weiteren Wirkstoffen verabreicht werden. Als weitere Wirkstoffe für die Kombinationspräparate sind geeignet:

Alle Antidiabetika, die in der Roten Liste 2004, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder Apidra^{®}, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulfonylfhamstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken (PPAR = peroxisome proliferator activated receptor, PXR = pregnane X receptor, ATP = adenosine triphosphat).

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht (HMGCoA = 3-hydroxy-3-methylglutaryl Coenzyme A).

Bei einer Ausführungsform der Erfindung werden die Verbindungen der Formel I in Kombination mit einem Cholesterinresorptionsinhibitor, wie z.B. Ezetimibe, Tiqueside, Pamaqueside, oder mit einer Verbindung, wie in PCT/EP 2004/00269, WO 2004/000804, WO 2004/000803, WO 2004/000805, EP 0114531, US 6,498,156 beschrieben, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alfa Agonist, wie z.B. GW 9578, GW 7647, verabreicht.

Bei einer Ausführungsform der Erfindung werden die Verbindungen der Formel I in Kombination mit einem gemischten PPAR alfa/gamma Agonisten, wie z.B. GW 1536, AVE 8042, AVE 8134, AVE 0847, oder wie in WO 00/64888, WO 00/64876, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide , BMS-201038, R-103757, verabreicht (MTP = Microsomal triglyceride transfer protein).

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705, verabreicht (CETP = cholesteryl ester transfer protein).

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht (LDL = Low Density Lipids).

Bei einer Ausführungsform der Erfindung werden die Verbindungen der Formel I in Kombination mit einem ACAT-Inhibitor, wie z.B. Avasimibe, verabreicht (ACAT = acyl-Coenzyme A:cholesterol acyltransferase).

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulfonylhamstoff, wie z.B. Tolbutamid, Glibenclamid, Glipizid oder Glimepirid verabreicht. Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxi]phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Adenosin A1 Agonisten, wie z. B. solchen, die in der WO 2004/003002 beschrieben sind, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.: Hormone and Metabolic Research (2001), 33(9), 554-558), NPY-Antagonisten (NPY = Neuropeptid Y, z.B. Naphthalin-1-sulfonsäure-{4-[(4-amino-quinazolin-2-ylamino)-methyl]-cyclohexylmethyl}-amid Hydrochlorid (CGP 71683A)), MC4-Agonisten (MC4 = Melanocortin 4 receptor, z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff Hydrochlorid (SB-334867-A)), H3-Agonisten (H3 = Histamin Rezeptor, z.B. 3-Cyclohexyl-1-(4,4-dimethyl-1,4,6,7-tetrahydro-imidazo[4,5-c]pyridin-5-yl)-propan-1-on Oxalsäuresalz (WO 00/63208)); TNF-Agonisten (TNF = Tumor Necrosis Factor), CRF-Antagonisten (CRF = corticotropin releasing factor, z.B. [2-Methyl-9-(2,4,6-trimethylphenyl)-9H-1,3,9-triaza-fluoren-4-yl]-dipropyl-amin (WO 00/66585)), CRF BP-Antagonisten (CRF-BP = corticotropin releasing factor -binding protein, z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxi)-ethylamino]-ethanol Hydrochlorid (WO 01/83451)), CB1 (Cannabinoid Rezeptor 1) Rezeptor Antagonisten (zB. Rimonabant oder die in WO 02/28346 genannten Wirkstoffe, MSH (Melanocyt-stimulierendes Hormon)-Agonisten, CCK-A (CCK-A = cholecystokinin-A) Agonisten (z.B. {2-[4-(4-Chloro-2,5-dimethoxi-phenyl)-5-(2-cyclohexylethyl)-thiazol-2-ylcarbamoyl]-5,7-dimethyl-indol-1-yl}-essigsäure Trifluoressigsäuresalz (WO 99/15525)), Serotonin-Wiederaufnahme-Inhibitoren (z.B. Dexfenfluramine), gemischte Sertonin- und noradrenerge Verbindungen (z.B. WO 00/71549), 5HT-Agonisten (Serotoninmimetika), z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxi-1-(2-diisopropylamino-ethylcarbamoyl)-3,4-dihydro-1H-isochinolin-2-carbonsäuretertiärbutylester (WO 01/85695)), TRH-Agonisten (TRH = TSH Releasing Hormone; TSH = thyroidstimulating hormone; thyrotropin), siehe z.B. EP 0 462 884 entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (DA = Dopamine Autoreceptor, wie z.B. Bromocriptin, Doprexin), Lipase/Amylase-Inhibitoren (z.B. WO 00/40569), PPAR-Modulatoren (z.B. WO 00/78312), RXR- (RXR = Retinoid X Receptor) Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer weiteren Ausführungsform ist der weitere Wirkstoff Rimonabant.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.); Caromax ist ein Carob enthaltendes Produkt der Fa. Nutrinova, Nutrition Specialties & Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

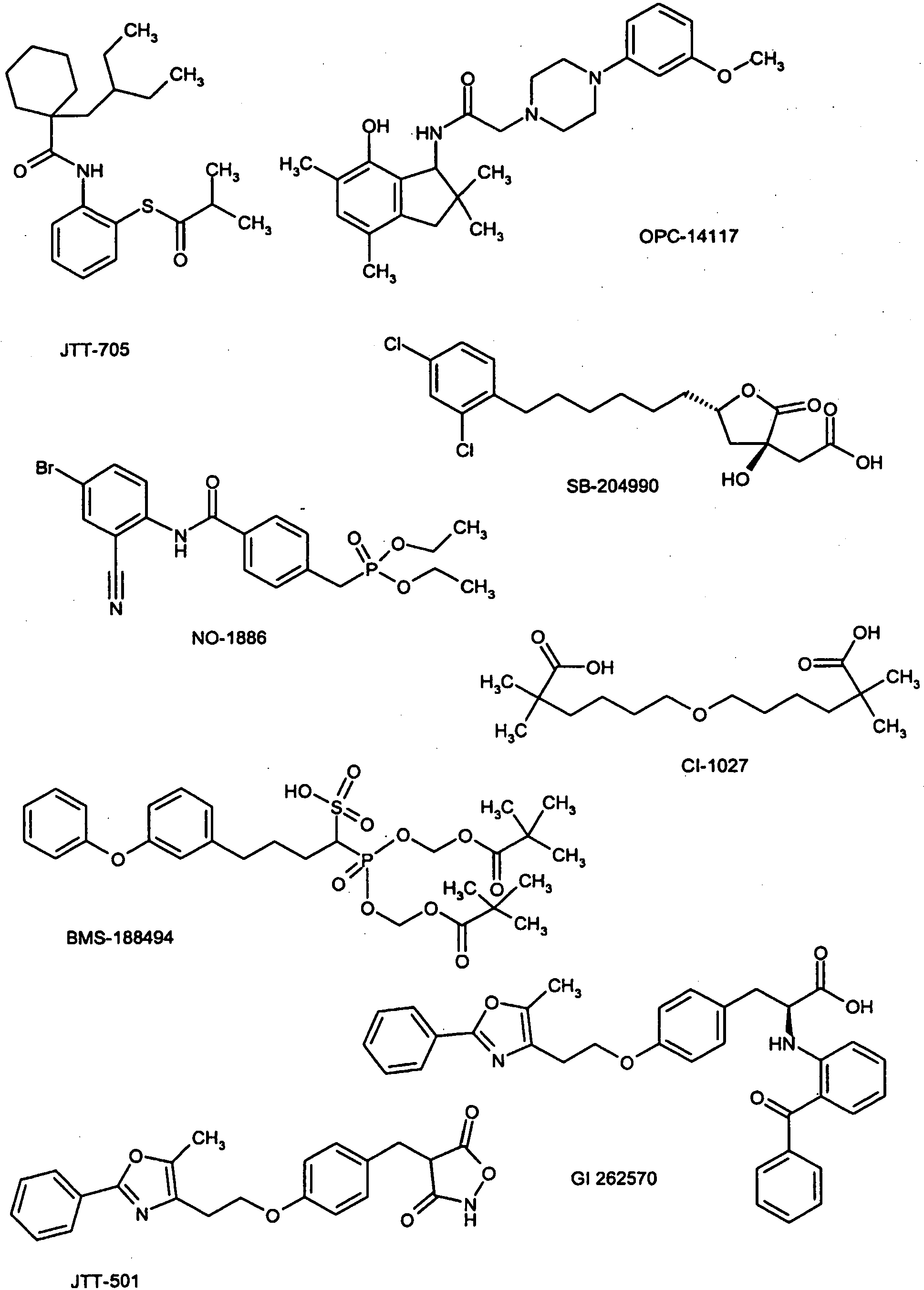

Die nachfolgend aufgeführten Beispiele dienen zur Erläuterung der Erfindung.

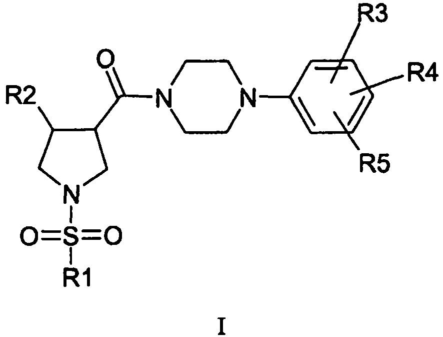

Die relative Konfiguration gibt die Stellung der Substituenten in 3 und 4 Position am Pyrrolidinring an, gestrichelte Linien geben die Anknüpfung an.

| **Beispiel** | **relative Konfiguration** | **R1** | **R2** | **R3** | **R4** | **R5** |
|---|---|---|---|---|---|---|
| **1** | trans | 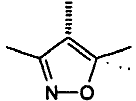 | 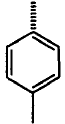 | o-CH3 | m'-CH3 | H |
| **2** | trans | 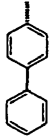 | 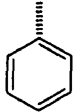 | H | o-CH3 | H |
| **3** | trans | 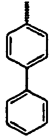 | 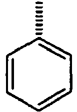 | H | p-NO2 | H |
| **4** | trans | 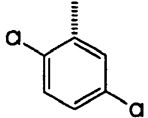 | 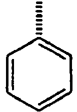 | H | m-CF3 | H |
| **5** | trans | 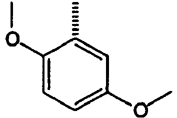 | 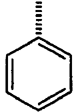 | H | H | H |
| **6** | trans | 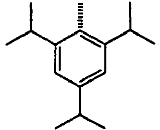 | 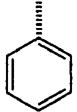 | H | H | H |
| **7** | trans | 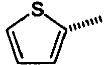 | 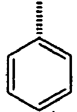 | H | p-COCH3 | H |
| **8** | trans | 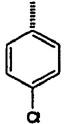 | 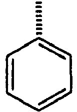 | o-CH3 | m'-CH3 | H |
| **9** | trans | 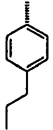 | 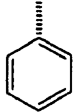 | o-CH3 | m-CH3 | H |
| **10** | trans | 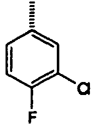 | 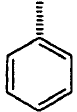 | o-CH3 | m'-CH3 | H |
| **11** | trans | 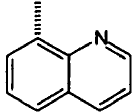 | 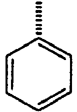 | H | o-Cl | H |
| **12** | trans | 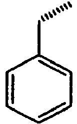 | 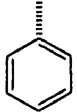 | H | p-Cl | H |
| **13** | trans | 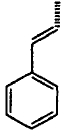 | 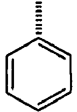 | H | p-OCH3 | H |
| **14** | trans | 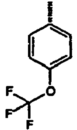 | 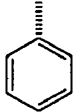 | H | o-Cl | H |
| **15** | trans | 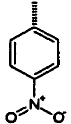 | 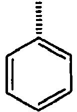 | H | p-Cl | H |
| **16** | trans | 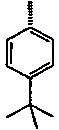 | 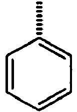 | H | p-OCH3 | H |
| **17** | trans | 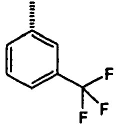 | 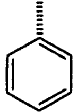 | H | p-OCH3 | H |
| **18** | trans | 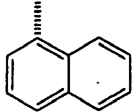 | 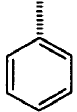 | H | o-OCH2CH3 | H |
| **19** | trans | 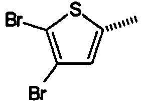 | 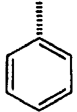 | H | o-F | H |
| **20** | trans | 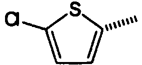 | 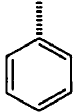 | H | H | H |
| **21** | trans | 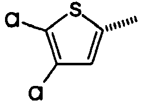 | 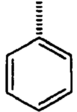 | H | H | H |
| **22** | trans | 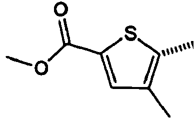 | 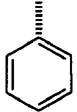 | H | H | H |
| **23** | trans | 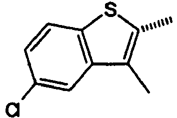 | 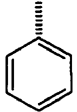 | H | H | H |
| **24** | trans | 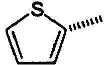 | 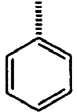 | H | o-OCH3 | H |
| **25** | trans | 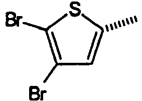 | 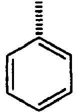 | H | o-OCH3 | H |
| **26** | trans | 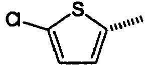 | 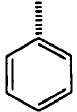 | H | o-OCH3 | H |
| **27** | trans | 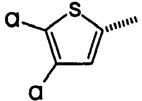 | 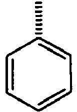 | H | o-OCH3 | H |
| **28** | trans | 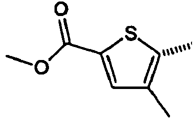 | 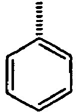 | H | o-OCH3 | H |
| **29** | trans | 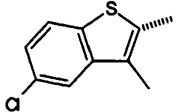 | 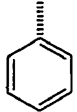 | H | o-OCH3 | H |
| **30** | trans | 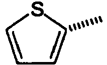 | 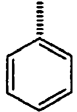 | H | m-CF3 | H |
| **31** | trans | 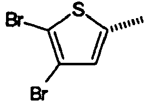 | 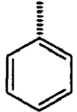 | H | m-CF3 | H |
| **32** | trans | 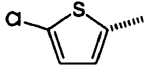 | 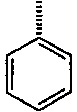 | H | m-CF3 | H |
| **33** | trans | 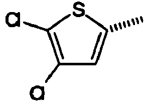 | 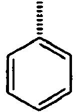 | H | m-CF3 | H |
| **34** | trans | 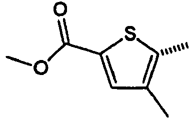 | 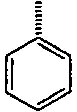 | H | m-CF3 | H |
| **35** | trans | 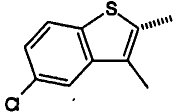 | 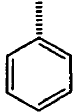 | H | m-CF3 | H |
| **36** | trans | 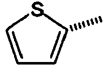 | 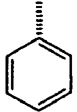 | o-CH3 | p-CH3 | H |
| **37** | trans | 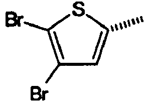 | 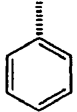 | o-CH3 | p-CH3 | H |
| **38** | trans | 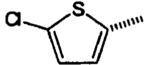 | 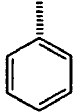 | o-CH3 | p-CH3 | H |
| **39** | trans | 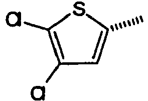 | 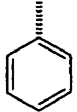 | o-CH3 | p-CH3 | H |
| **40** | trans | 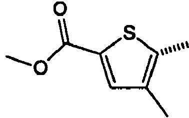 | 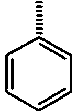 | o-CH3 | p-CH3 | H |
| **41** | trans | 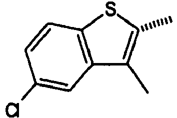 | 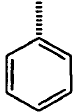 | o-CH3 | p-CH3 | H |
| **42** | trans | 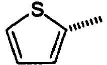 | 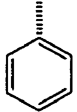 | m-CH3 | p-CH3 | H |
| **43** | trans | 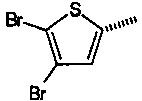 | 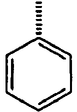 | m-CH3 | p-CH3 | H |
| **44** | trans | 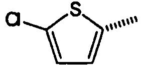 | 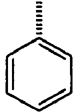 | m-CH3 | p-CH3 | H |
| **45** | trans | 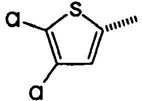 | 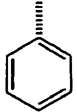 | m-CH3 | p-CH3 | H |
| **46** | trans | 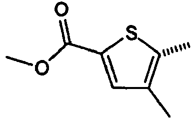 | 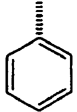 | m-CH3 | p-CH3 | H |
| **47** | trans | 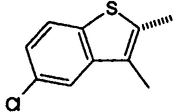 | 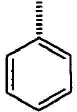 | m-CH3 | p-CH3 | H |
| **48** | trans | 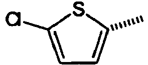 | 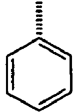 | o-CH3 | m-CH3 | H |
| **49** | trans | 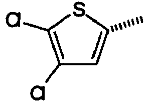 | 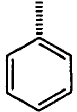 | o-CH3 | m'-CH3 | H |
| **50** | trans | 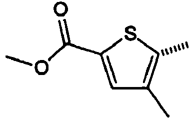 | 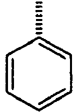 | o-CH3 | m'-CH3 | H |
| **51** | trans | 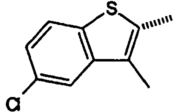 | 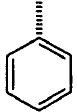 | o-CH3 | m'-CH3 | H |
| **52** | trans | 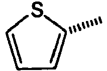 | 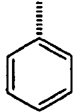 | o-CH3 | m-CH3 | H |
| **53** | trans | 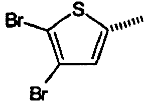 | 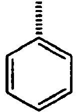 | o-CH3 | m-CH3 | H |
| **54** | trans | 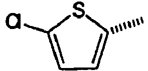 | 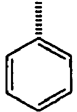 | o-CH3 | m-CH3 | H |
| **55** | trans | 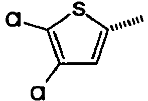 | 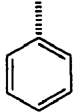 | o-CH3 | m-CH3 | H |
| **56** | trans | 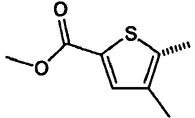 | 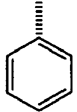 | o-CH3 | m-CH3 | H |
| **57** | trans | 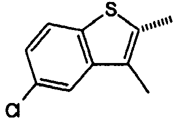 | 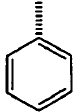 | o-CH3 | m-CH3 | H |
| **58** | trans | 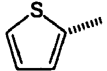 | 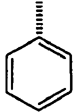 | H | p-OCH3 | H |
| **59** | trans | 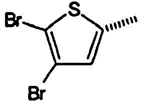 | 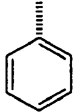 | H | p-OCH3 | H |
| **60** | trans | 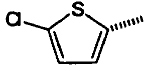 | 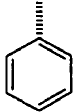 | H | p-OCH3 | H |
| **61** | trans | 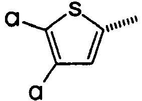 | 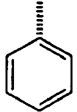 | H | p-OCH3 | H |
| **62** | trans | 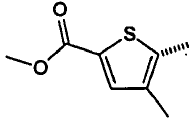 | 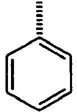 | H | p-OCH3 | H |
| **63** | trans | 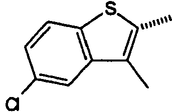 | 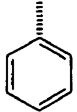 | H | p-OCH3 | H |
| **64** | trans | 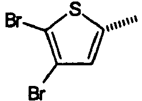 | 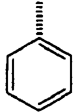 | H | H | H |
| **65** | trans | 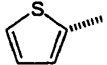 | 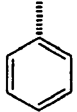 | o-CO2Me | m'CO2Me | H |
| **66** | trans | 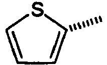 | 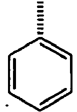 | o-Cl | m'-Cl | H |
| **67** | trans | 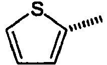 | 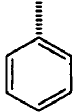 | o-CH3 | m'-CH3 | H |
| **68** | trans | 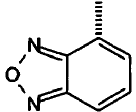 | 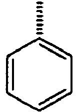 | o-CH3 | m'-CH3 | H |
| **69** | trans | 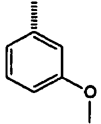 | 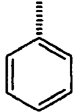 | o-CH3 | m'-CH3 | H |
| **70** | trans | 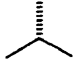 | 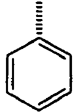 | o-CH3 | m'-CH3 | H |
| **71** | trans | 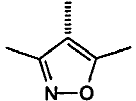 | 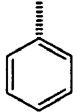 | o-CH3 | m'-CH3 | H |
| **72** | trans | 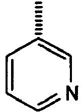 | 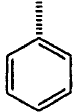 | o-CH3 | m'-CH3 | H |
| **73** | trans | 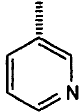 | 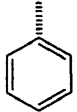 | o-Cl | m'-Cl | H |
| **74** | trans | 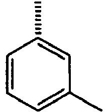 | 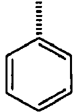 | o-Cl | m'-Cl | H |
| **75** | trans | 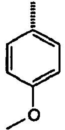 | 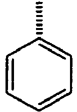 | o-Cl | m'-Cl | H |
| **76** | trans | 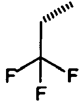 | 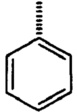 | o-OCH3 | m'-OCH3 | H |
| **77** | trans | 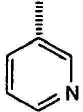 | 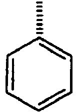 | o-OCH3 | m'-OCH3 | H |
| **78** | trans | 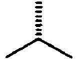 | 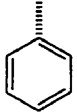 | o-OCH3 | m'-OCH3 | H |
| **79** | trans | 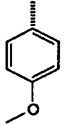 | 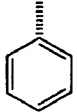 | o-OCH3 | m'-OCH3 | H |
| **80** | trans | 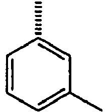 | 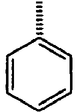 | o-OCH3 | m'-OCH3 | H |
| **81** | trans | 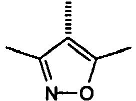 | 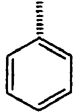 | o-OCH3 | m'-OCH3 | H |
| **82** | trans | 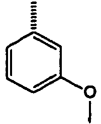 | 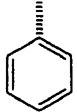 | o-OCH3 | m'-OCH3 | H |
| **83** | trans | 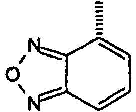 | 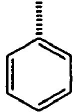 | o-OCH3 | m'-OCH3 | H |
| **84** | trans | 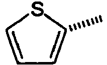 | 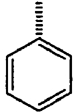 | o-OCH3 | m'-OCH3 | H |
| **85** | trans | 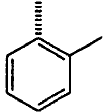 | 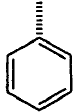 | o-Cl | m'-Cl | H |
| **86** | trans | 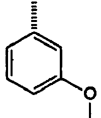 | 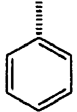 | o-CH3 | o'-CH3 | H |
| **87** | trans | 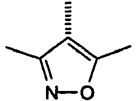 | 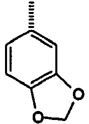 | o-CH3 | m'-CH3 | H |
| **88** | trans | 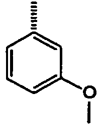 | 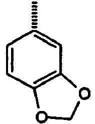 | o-CH3 | m'-CH3 | H |
| **89** | trans | 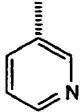 | 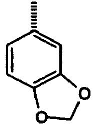 | o-CH3 | m'-CH3 | H |
| **90** | trans | 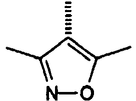 | 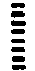 | o-CH3 | m'-CH3 | H |
| **91** | trans | 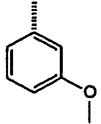 | 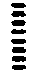 | o-CH3 | m'-CH3 | H |
| **92** | trans | 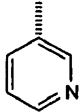 | 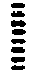 | o-CH3 | m'-CH3 | H |
| **93** | trans | 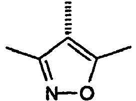 | 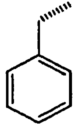 | o-CH3 | m'-CH3 | H |
| **94** | trans | 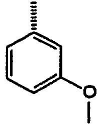 | 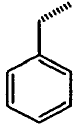 | o-CH3 | m'-CH3 | H |
| **95** | trans | 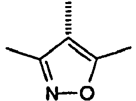 | 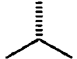 | o-CH3 | m'-CH3 | H |
| **96** | trans | 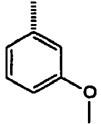 | 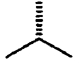 | o-CH3 | m'-CH3 | H |
| **97** | trans | 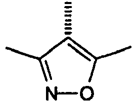 | 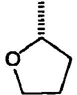 | o-CH3 | m'-CH3 | H |
| **98** | trans | 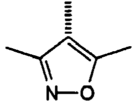 | 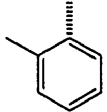 | o-CH3 | m'-CH3 | H |
| **99** | trans | 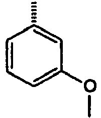 | 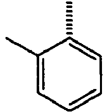 | o-CH3 | m'-CH3 | H |
| **100** | trans | 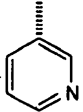 | 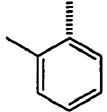 | o-CH3 | m'-CH3 | H |
| **101** | trans | 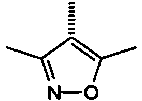 | 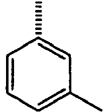 | o-CH3 | m'-CH3 | H |
| **102** | trans | 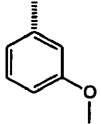 | 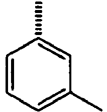 | o-CH3 | m'-CH3 | H |
| **103** | trans | 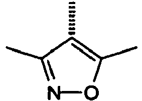 | 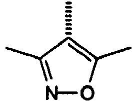 | o-CH3 | m'-CH3 | H |
| **104** | trans | 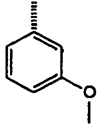 | 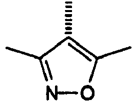 | o-CH3 | m'-CH3 | H |
| **105** | trans | 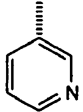 | 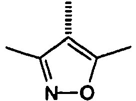 | o-CH3 | m'-CH3 | H |
| **106** | trans | 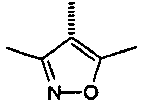 | 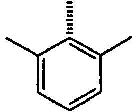 | o-CH3 | m'-CH3 | H |
| **107** | trans | 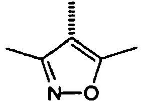 | 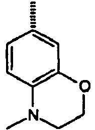 | o-CH3 | m'-CH3 | H |
| **108** | trans | 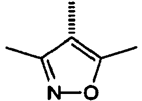 | 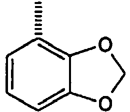 | o-CH3 | m'-CH3 | H |
| **109** | trans | 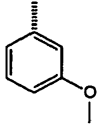 | 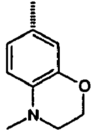 | o-CH3 | m'-CH3 | H |
| **110** | trans | 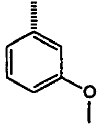 | 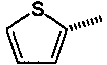 | o-CH3 | m'-CH3 | H |
| **111** | trans | 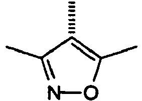 | 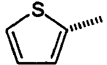 | o-CH3 | m'-CH3 | H |
| **112** | trans | 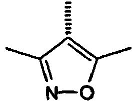 | 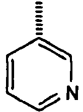 | o-CH3 | m'-CH3 | H |
| **113** | trans | 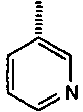 | 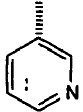 | o-CH3 | m'-CH3 | H |
| **114** | trans | 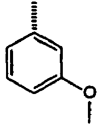 | 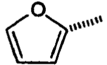 | o-CH3 | m'-CH3 | H |
| **115** | trans | 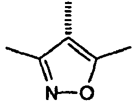 | 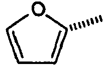 | o-CH3 | m'-CH3 | H |
| **116** | trans | 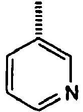 | 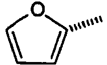 | o-CH3 | m'-CH3 | H |
| **117** | cis | 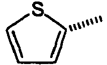 | 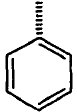 | o-CH3 | m'-CH3 | H |
| **118** | cis | 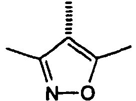 | 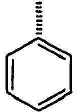 | o-CH3 | m'-CH3 | H |
| **119** | cis | 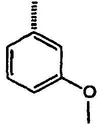 | 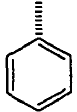 | o-CH3 | m'-CH3 | H |
| **120** | cis | 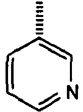 | 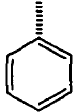 | o-CH3 | m'-CH3 | H |

Die Wirksamkeit der Verbindungen wurde wie folgt getestet:

ABCA-1 ist ein membrangebundenes Transporterprotein, welches eine Schlüsselrolle beim Efflux von Cholesterol aus extrahepatischen Zellen auf naszierende HDL-Partikel besitzt (Singaraja et al, Arterioscler Thromb Vasc Biol., 1322-1332, 2003). HDL-Partikel werden von der Leber über den SRB1-Rezeptor aufgenommen. Den Rücktransport von Cholesterol aus peripheren Geweben zur Leber, an dem ABCA1 maßgeblich beteiligt ist, bezeichnet man als reversen Cholesterintransport.

Es sind zahlreiche genetische Mutationen des ABCA-1 Gens bekannt. So hat die genetische Erkrankung "Tangier's Disease" ihre molekularen Ursprung in Mutationen von ABCA1. Diese äußern sich im homozygoten Krankheitszustand in einem sehr niedrigen HDL-Cholesterol Spiegel und einem stark erhöhten Risiko an kardiovaskulären Erkrankungen. (Brooks-Wilson et al., Nat. Genet. 22,336-345, 1999; Bodzioch et al. , Nat. Genet., 22, 347-351, 1999; Rust et al. , Nat. Genet., 22, 352-355,1999).

Analog zur Situation im Menschen haben homozygote ABCA-1 Knockout-Mäuse praktisch keine messbaren HDL Plasma Spiegel, während heterozygote ABCA-1 Knockout-Mäuse ein um etwa 50% reduziertes HDL-Niveau besitzen (Orso et al. Nat. Genet. 24, 192-196, 2000; McNeish et al., Proc. Natl. Acad. Sci. USA, 97, 4245 - 4250, 2000). ABCA-1 Knockout-Mäuse weisen zudem eine erhöhte Cholesterol-Absorbtion auf (McNeish et al., Proc. Natl. Acad. Sci. USA, 97, 4245 - 4250, 2000).

Eine Erhöhung der ABCA-1 Genexpression führt potenziell zu einer Zunahme an HDL Cholesterol, einer Abnahme der intestinalen Cholesterol-Absorption und zu einem vermehrten Ausscheiden von überschüssigem Cholesterol aus extrahepatischen Gewebe, eingeschlossen Makrophagen. Weiterhin wurde gezeigt, daß die Überexpression von ABCA1 in atherosklerotischen Mausmodellen zu einer signifikanten Reduktion der Atherosklerose führt (Brewer et al., Am J Cardiol., 10K-16K, 2003; Singaraja et al, J Clin Invest, 110, 35-42, 2002). ABCA1-Promotor-Test

### Prinzip

Für die Analyse der Wirkstärke von Substanzen, welche die Expression des humanen ABCA1-Gens über dessen Promotor aktivieren, wird eine stabil transfizierte THP1-Zellinie (humane Monozytenzellinie) benutzt, die hier als ABCA1-Reporterzellinie bezeichnet wird. Sie enthält ein stabil ins Genom integriertes 3,2 kb großes Fragment des humanen ABCA1-Promotors vor einem Luziferase-Reporterelement. Ohne Zugabe eines ABCA1-Expressionsaktivators ist die Expression des Luziferase-Reportergens nur gering. Die ABCA1-Expression stimulierenden Substanzen bewirken über eine Aktivierung des ABCA1-Promotors eine verstärkte Expression des Luciferase-Reportergens. Die gebildete Luziferase läßt sich über ein entsprechendes Substrat mittels Chemilumineszenz nachweisen.

### Konstruktion der Zellinie

Die ABCA1-Reporterzellinie wurde wie folgt hergestellt: zuerst wurde in das Luziferase-Reporterplasmid pGL3basic (#E1751, Promega) das Gen für Neomycinresistenz (Acc.No.: AJ000156) 3'-unterhalb des Luziferase-Reporterelements eingebracht. Anschließend wurde ein aus humaner genomischer DNA (#6550-1, Clontech) mittels Polymerasekettenreaktion vervielfältigtes 3,2 kb großes ABCA1-Promotorfragment 5'-oberhalb des Luziferasereporterelements einkloniert. Das so entstandene Reporterplasmid wurde als pGL3BN-ABCA1P bezeichnet. In diesem Plasmid wird die Expression der Luziferase vom ABCA1-Promotor kontrolliert. Die Klonierung und Sequenzierung des Konstruktes erfolgte analog wie bei Sambrook J. et. al. beschrieben (Molecular cloning, Cold Spring Harbor Laboratory Press, 1989). Für die stabile Transfektion in THP1-Zellen wurde pGL3BN-ABCA1P zuerst mit einer Restriktionsendonuklease linearisiert und dann nach den Angaben in Ausubel, F.M. et al. (Current protocols in molecular biology, Vol. 1-3, John Wiley & Sons, Inc., 1995) in die THP1-Zellen eingebracht. Unter Verwendung von geneticinhaltigem Medium (0,5 mg/ml) wurde ein geeigneter stabiler Zellklon selektioniert, der eine möglichst starke Aktivierung des Luziferasegens nach Behandlung mit einem Standard ABCA1-Expressionsaktivator zeigte.

### Durchführung des Tests

Die Aktivität von ABCA1-Expressionsaktivatoren wird in einem 3-Tagestest bestimmt, der nachfolgend beschrieben ist:

### Tag 1

Bei der ABCA1-Reporterzellinie handelt es sich um Suspensionszellen. Diese werden bis zu einer Konzentration von 0,5x10⁶ Zellen/ml in RPMI-Medium (# 52400-025, Invitrogen) kultiviert, das mit folgenden Zusätzen versetzt ist: 10% FKS (fötales Kälberserum; #16000-044, Invitrogen), 0,5 mg/ml Geneticin (#10131-019, Invitrogen) und 1% Penicillin-Streptomycin-Lösung (#15140-122, Invitrogen). Die Kultivierung erfolgt in Standard-Zellkulturflaschen (# 353112, Becton Dickinson) in einem Zellkulturbrutschrank bei 37°C in Anwesenheit von 5% CO₂.

Für den ABCA1-Promotor-Test werden die Suspensionszellen in 50 ml Röhrchen bei 1200 U/min abzentrifugiert. Der Überstand wird abgesaugt, das Pellet in 15 ml RPM1-Medium resuspendiert und in einem Zellzählgerät gezählt. Nach der Verdünnung auf 175.000 Zellen/ml wird 0,1 µg/ml PMA (Phorbol 12-Myristat 13-Acetat, #8139-5MG, Sigma) zugesetzt, um die Differenzierung der monozytären THP1-Suspensionszellen zu adhärenten Makrophagen zu induzieren. Jeweils 35.000 Zellen pro well werden in einer 96 well-Mikrotiterplatte mit klarem Plastikboden (#3610, Coming Costar) ausgesät. Die Platten werden für 24 h in einem Zellkulturbrutschrank bei 37°C und 5% CO2 inkubiert.

### Tag 2

Zu testende ABCA1-Expressionsaktivatoren werden in einer Konzentration von 10 mM in DMSO gelöst. Diese Stocklösung wird in oben beschriebenem RPMI-Medium (versetzt mit 0,1 µg/ml PMA) verdünnt. Testsubstanzen werden in 11 verschiedenen Konzentrationen im Bereich von 33 µM bis 330 pM getestet.

Das Medium der am Tag 1 ausgesäten ABCA1-Reporterzellinie wird vollständig abgesaugt und die verdünnten Testsubstanzen sofort zu den Zellen zugegeben: Die Verdünnung und Zugabe der Substanzen erfolgt mit einem Pipettierroboter (Beckman FX). Das Endvolumen der in Medium verdünnten Testsubstanzen beträgt 100 µl pro well einer 96 well-Mikrotiterplatte. Die DMSO-Konzentration in dem Test beträgt unter 0.1 % v/v, um zelltoxische Effekte des Lösungsmittels zu vermeiden.

Jede Platte wurde mit einem Standard ABCA1-Expressionsaktivator belegt, der ebenfalls in 11 verschiedenen Konzentrationen verdünnt wird, um die Funktionsfähigkeit des Tests in jeder Einzelplatte nachzuweisen. Die Testplatten werden für 24 h in einem Brutschrank bei 37 °C und 5 % CO₂ inkubiert.

### Tag 3

Die mit den Testsubstanzen behandelten ABCA1-Reporterzellen werden aus dem Brutschrank entnommen und das Medium abgesaugt. Zur Lyse der Zellen werden 50 µl Bright Glo Reagenz (#E2650, Promega) pro well einer 96-well Mikrotiterplatte zupipettiert. Nach einer 10 minütigen Inkubation im Dunkeln bei Raumtemperatur werden die Mikrotiterplatten im Lumineszenzmeßgerät (Trilux der Firma Wallac) gemessen. Die Meßzeit pro well einer Mikrotiterplatte beträgt 1 sec.

### Auswertung

Die Rohdaten des Lumineszenzmeßgerätes werden in ein Microsoft Excel-File transferiert. Dosis-Wirkungskurven und EC50-Werte von ABCA1-Expressionsaktivatoren werden mit dem Program XL.Fit nach Vorgabe des Herstellers (Firma IDBS) berechnet.

**Tabelle 2: Biologische Aktivität**

| **Beispiel Nr** | **EC50 ABCA1 [µM]** |
|---|---|
| **1** | 2,83705 |
| **7** | 13,18232 |
| **21** | 4,62253 |
| **24** | 14,98243 |
| **25** | 3,81272 |
| **26** | 2,93693 |
| **27** | 6,99135 |
| **29** | 5,60744 |
| **30** | 3,21622 |
| **31** | 1,77475 |
| **32** | 16,16255 |
| **33** | 6,5901 |
| **36** | 3,60377 |
| **37** | 2,56764 |
| **38** | 2,65662 |
| **39** | 3,72624 |
| **42** | 18,81371 |
| **43** | 4,07876 |
| **44** | 15,14785 |
| **45** | 4,61792 |
| **48** | 0,7057 |
| **49** | 1,9526 |
| **50** | 7,34391 |
| **51** | 2,76602 |
| **52** | 3,27045 |
| **53** | 5,75795 |
| **54** | 4,63021 |
| **55** | 0,82611 |
| **59** | 1,15728 |
| **60** | 6,67634 |
| **61** | 2,6119 |
| **64** | 3,15637 |
| **66** | 0,43197 |
| **67** | 2,19183 |
| **68** | 0,86925 |
| **69** | 0,33012 |
| **70** | 0,91525 |
| **71** | 0,38061 |
| **72** | 0,24141 |
| **73** | 0,40652 |
| **74** | 0,34049 |
| **75** | 0,97963 |
| **76** | 3,11583 |
| **79** | 1,5805 |
| **80** | 3,1521 |
| **81** | 1,81796 |
| **82** | 1,49147 |
| **83** | 3,00327 |
| **84** | 2,66994 |
| **85** | 0,25968 |
| **86** | 0,45878 |
| **87** | 1,22638 |
| **88** | 2,71771 |
| **90** | 16,76062 |
| **91** | 2,34185 |
| **93** | 2,47707 |
| **94** | 2,92544 |
| **95** | 4,59268 |
| **96** | 3,604 |
| **98** | 0,40095 |
| **99** | 0,70676 |
| **100** | 1,34805 |
| **101** | 1,85365 |
| **102** | 1,04924 |
| **104** | 6,27323 |
| **106** | 0,37205 |
| **108** | 0,58486 |
| **110** | 0,9142 |
| **111** | 0,86686 |
| **112** | 14,29582 |
| **114** | 2,13601 |
| **115** | 2,63737 |
| **116** | 10,82924 |
| **117** | 0,29118 |
| **118** | 0,34646 |
| **119** | 0,17737 |
| **120** | 0,39477 |

Aus der Tabelle ist abzulesen, dass die Verbindungen der Formel I die Genexpression von ABCA-1 erhöhen, und so eine Zunahme des HDL-Cholesterols bewirken.

### Verfahren

Die erfindungsgemäßen Verbindungen der Formel I können entsprechend den folgenden Reaktionsschemata hergestellt werden:

### Verfahren A:

Dieses Verfahren dient zur Synthese des Bausteins B, wobei R3, R4 und R5 die oben genannten Bedeutungen haben.

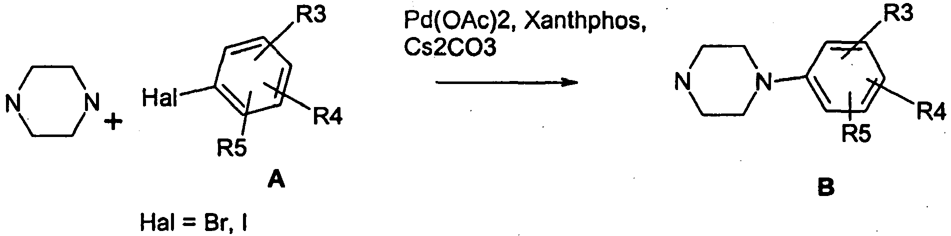

Ein aromatisches oder heteroaromatisches Halogenid der allgemeinen Formel A, wobei R3, R4 und R5 die oben genannten Bedeutungen haben, wird mit Piperazin unter Palladiumkatalyse in Gegenwart einer Base wie zum Beispiel Cäsiumcarbonat und eines Liganden wie zum Beispiel 9,9-Diemethyl-4,5-Bis(diphenylphosphino)xanthene und einer Palladiumquelle wie zum Beispiel Palladiumacetat in einem Lösungsmittel wie zum Beispiel 1,4-Dioxan zum substituierten Piperazin der allgemeinen Formel B umgesetzt.

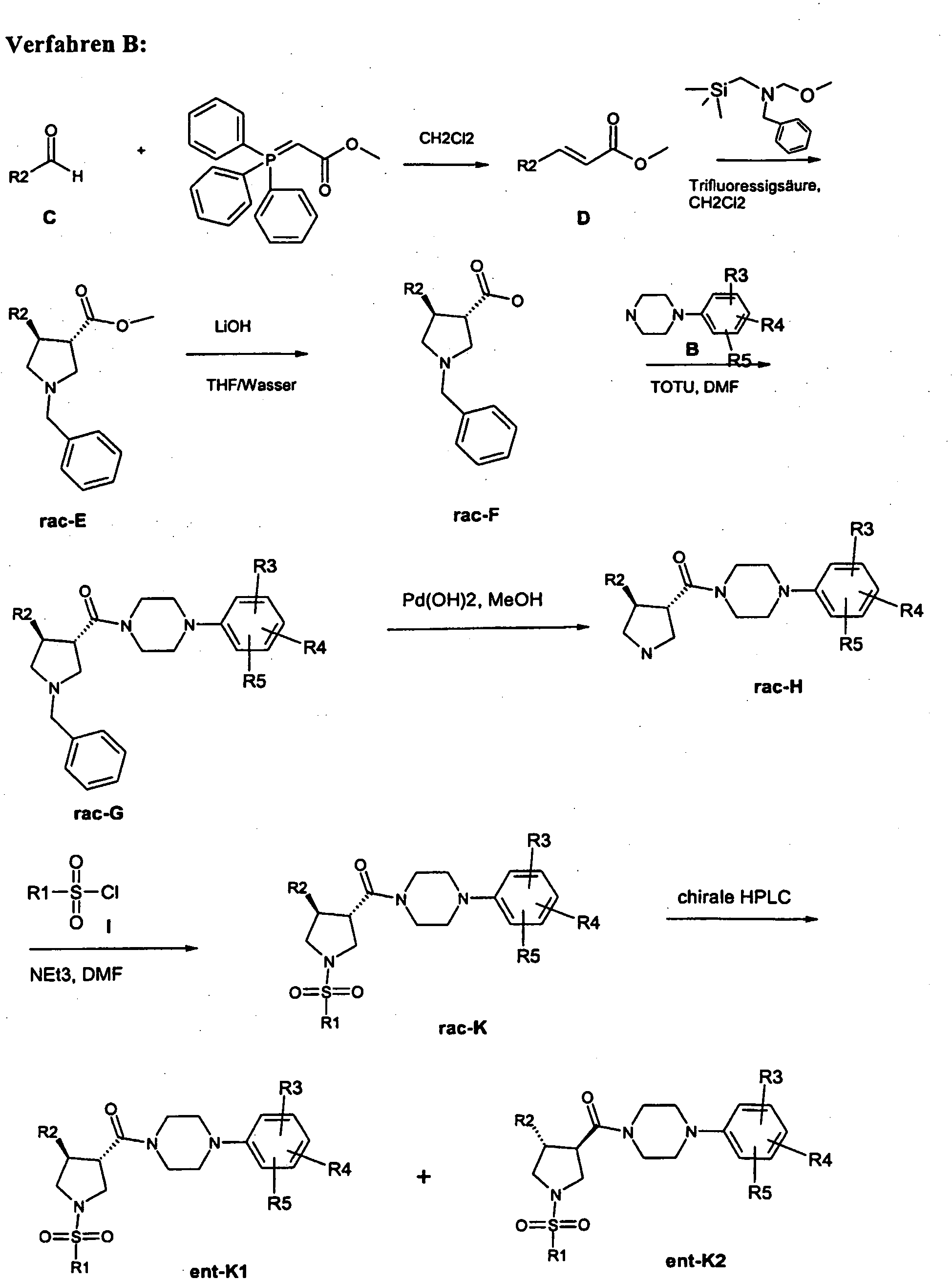

Ein Aldehyd der allgemeinen Formel C, worin R2 die oben beschriebene Bedeutung hat, wird mit Methyl(triphenylphoshoranylide)acetat in einem unpolar aprotischen Lösungsmittel wie zum Beispiel Dichlormethan zum trans konfigurierten α,β-ungesättigten Ester der allgemeinen Formel D umgesetzt. Der Ester wird mit N-(Methoxymethyl)-N-(trimethylsilylmethyl)benzylamin in einem unpolar aprotischen Lösungsmittel wie zum Beispiel Dichlormethan in Gegenwart einer Säure wie zum Beispiel Trifluoressigsäure zum trans konfigurierten Pyrrolidin-3-carbonsäuremethylester der allgemeinen Formel E umgesetzt. Der Ester wird mit einer Base wie Lithiumhydroxid in einem polaren Lösungsmittelgemisch wie Tetrahydrofuran und Wasser zur freien Carbonsäure der allgemeinen Formel F gespalten. Unter Einwirkung eines Kupplungsreagenzes wie zum Beispiel O-[Cyan(ethoxycarbonyl)methylenamino]-1,1,3,3-tetramethyluronium-tetrafluoroborat in Gegenwart einer Base wie zum Beispiel Triethylamin in einem polar aprotischen Lösungsmittel wie N,N.Diemethylformamid wird die Carbonsäure der allgemeinen Formel F mit dem Piperazin der allgemeinen Formel B, worin R3 , R4 und R5 die oben beschriebenen Bedeutungen haben, zum Amid der allgemeinen Formel G umgesetzt. Durch Einwirkung von Wasserstoff in Gegenwart eines Katalysators wie zum Beispiel Palladiumhydroxid in einem polaren Lösungsmittel wie Methanol oder, falls R2 ein hydrogenolyse empfindlicher Substituent beinhaltet, durch Umsetzung mit 2,2,2-Trichlorethoxycarbonylchlorid in einem Lösungsmittel wie beispielsweise Acetonitril und anschließender Spaltung des entstandenen Carbamats durch Einwirkung von Zink in Essigsäure , erhält man aus dem Benzyl-geschützten Pyrrolidin der allgemeinen Formel G das ungeschützte Pyrrolidin der allgemeinen Formel H. Durch Umsetzung mit einem Sulfonylchlorid der allgemeinen Formel I, worin R1 die oben beschriebene Bedeutung hat, erhält man daraus das Sulfonylpyrrolidin der allgemeinen Formel K. Erfolgt die Reinigung von K mittels RP-HPLC mit dem Eluent Acetonitril/Wasser+0.1 % Trifluoressigsäure so erhält man das Trifluoracetat-Salz von K. Das racemische Gemisch kann durch chirale HPLC in die enantiomeren reinen Formen der allgemeinen Formeln K1 und K2 getrennt werden.

Nach diesem Verfahren wurden die Beispiele 1 bis 110 synthetisiert.

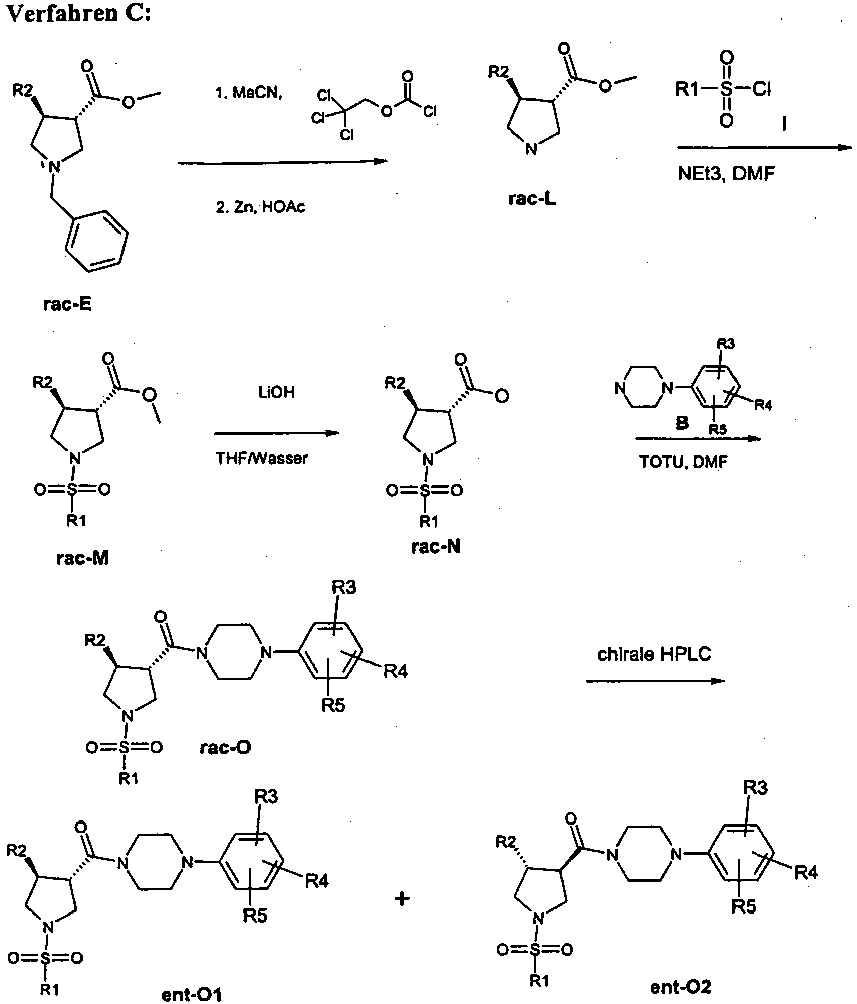

Der trans konfigurierte Pyrrolidin-3-carbonsäuremethylester der allgemeinen Formel E wird mit 2,2,2-Trichlorethoxycarbonylchlorid in einem Lösungsmittel wie beispielsweise Acetonitril umgesetzt. Das so entstandene Carbamat wird sofort durch Einwirkung von Zink in Essigsäure zum ungeschützten Pyrrolidin der allgemeinen Formel L umgesetzt. Durch Umsetzung mit einem Sulfonylchlorid der allgemeinen Formel I, worin R1 die oben beschriebene Bedeutung hat, erhält man daraus das Sulfonylpyrrolidin der allgemeinen Formel M. Der Ester wird durch Einwirkung einer Base wie Lithiumhydroxid in einem polaren Lösungsmittelgemisch wie Tetrahydrofuran und Wasser zur freien Carbonsäure der allgemeinen Formel N gespalten. Unter Einwirkung eines Kupplungsreagenzes wie zum Beispiel O-[Cyan(ethoxycarbonyl)methylenamino]-1,1,3,3-tetramethyluronium-tetrafluoroborat in Gegenwart einer Base wie zum Beispiel Triethylamin in einem polar aprotischen Lösungsmittel wie N,N.Diemethylformamid wird die Carbonsäure der allgemeinen Formel N mit dem Piperazin der allgemeinen Formel B, worin R3, R4 und R5 die oben beschriebenen Bedeutungen haben, zum Amid der allgemeinen Formel O umgesetzt. Erfolgt die Reinigung von O mittels RP-HPLC mit dem Eluent Acetonitril/Wasser+0.1 % Trifluoressigsäure so erhält man das Trifluoracetat-Salz von O. Das racemische Gemisch kann durch chirale HPLC in die enantiomeren reinen Formen der allgemeinen Formeln O1 und O2 getrennt werden.

Nach diesem Verfahren wurden die Beispiele 110 bis 117 synthetisiert.

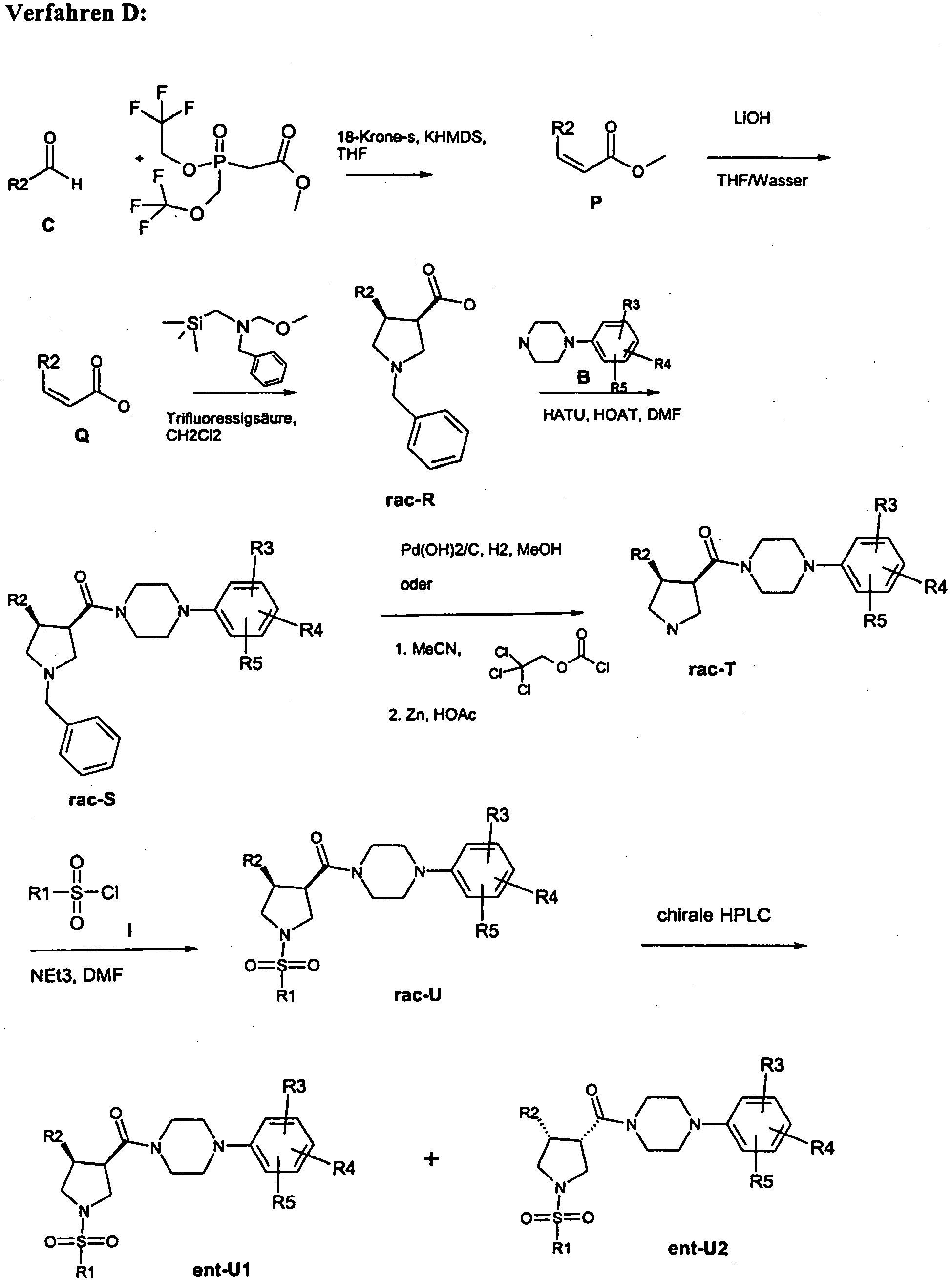

Ein Aldehyd der allgemeinen Formel C, worin R2 die oben beschriebene Bedeutung hat, wird mit [Bis-(2,2,2-trifluoro-ethoxy)-phosphoryl]-essigsäuremethylester in einem polar aprotischen Lösungsmittel wie zum Beispiel Tetrahydrofuran in Gegenwart einer Base wie Kalium-bis(trimethylsilyl)amid zum cis-konfigurierten α.β-ungesättigten Ester der allgemeinen Formel P umgesetzt. Der Ester wird mit einer Base wie Lithiumhydroxid in einem polaren Lösungsmittelgemisch wie Tetrahydrofuran und Wasser zur freien Carbonsäure der allgemeinen Formel Q gespalten. Die Carbonsäure wird mit N-(Methoxymethyl)-N-(trimethylsilylmethyl)benzylamin in einem unpolar aprotischen Lösungsmittel wie zum Beispiel Dichlormethan in Gegenwart einer Säure wie zum Beispiel Trifluoressigsäure zur cis-konfigurierten Pyrrolidin-3-carbonsäure der allgemeinen Formel R umgesetzt. Unter Einwirkung eines Kupplungsreagenzes wie zum Beispiel O-(7-Azabenzotriazol-1-yl)-N,N,N',N'-tetramethyluronium-hexafluorophoaphat in Gegenwart einer Base wie zum Beispiel Triethylamin in einem polar aprotischen Lösungsmittel wie N,N.Diemethylformamid wird die Carbonsäure der allgemeinen Formel R mit dem Piperazin der allgemeinen Formel B, worin R3, R4 und R5 die oben beschriebenen Bedeutungen haben, zum Amid der allgemeinen Formel S umgesetzt. Durch Einwirkung von Wasserstoff in Gegenwart eines Katalysators wie zum Beispiel Palladiumhydroxid in einem polaren Lösungsmittel wie Methanol oder, falls R2 ein hydrogenolyse empfindlicher Substituent beinhaltet, durch Umsetzung mit 2,2,2-Trichlorethoxycarbonylchlorid in einem Lösungsmittel wie beispielsweise Acetonitril und anschließender Spaltung des entstandenen Carbamats durch Einwirkung von Zink in Essigsäure , erhält man aus dem Benzyl-geschützten Pyrrolidin der allgemeinen Formel S das ungeschützte Pyrrolidin der allgemeinen Formel T. Durch Umsetzung mit einem Sulfonylchlorid der allgemeinen Formel I, worin R1 die oben beschriebene Bedeutung hat, erhält man daraus das Sulfonylpyrrolidin der allgemeinen Formel U. Erfolgt die Reinigung von U mittels RP-HPLC mit dem Eluent Acetonitril/Wasser+0.1 % Trifluoressigsäure so erhält man das Trifluoracetat-Salz von U. Das racemische Gemisch U kann durch chirale HPLC in die enantiomeren reinen Formen der allgemeinen Formeln U1 und U2 getrennt werden.

Nach diesem Verfahren wurden die Beispiele 117 bis 120 synthetisiert.

Die verwendeten Abkürzungen stehen für:

| | |
|---|---|
| Ac | Acetyl |
| Bn | Benzyl |
| iBu | Isobutyl |
| tBu | tert-Butyl |
| BuLi | n-Butyllithium |
| DC | Dünnschichtchromatographie |
| DCI | direkte chemische Ionisation (bei MS) |
| DCM | Dichlormethan |
| DMAP | 4-N,N-Dimethylaminopyridin |
| DMF | N,N-Dimethylformamid |
| DMSO | Dimethylsulfoxid |
| EE | Essigsäureethylester |
| ent | Enantiomer / enantiomerenrein |
| EI | Elektronenstoß-Ionisation (bei MS) |
| eq | Äquivalent |
| ESI | Elektronenspray-Ionisation (bei MS) |
| HPLC | Hochdruck-, Hochleistungsflüssigchromatographie |
| LC-MS | Flüssigchromatographie-gekoppelte Massenspektroskopie |
| m | meta |
| Me | Methyl |
| MS | Massenspektroskopie |
| NMR | Kernresonanzspektroskopie |
| o | ortho |
| p | para |
| Pd/C | Palladium auf Kohle |
| iPr | Isopropyl |
| nPr | n-Propyl |
| rac | Racemisch / racemisches Gemisch |
| Rf | Retentionszeit (bei DC) |

### Bausteinsynthesen nach Verfahren A:

### 1-(2,5-Dimethoxy-phenyl)-piperazin

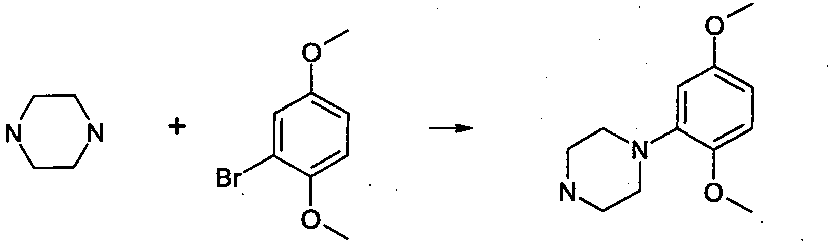

1.98 g Piperazin, 1.0 g 1-Bromo-2,5-dimethoxybenzol, 800 mg 9,9-Diemthyl-4,5-bis(diphenylphosphino)xanthene , 310 mg Palladium(II)acetat und 2.25 g Cäsiumcarbonat werden in 30 ml 1,4-Dioxan gelöst und unter einer Argonatmosphäre 24 Stunden bei 100 °C gerührt. Das Reaktionsgemisch wird mit 50 ml gesättigter Natriumhydrogencarbonatlösung und 50 ml gesättigter Natriumchloridlösung extrahiert, die vereinigten wässrigen Phasen werden mit 2N Natriumhydroxidlösung basisch gestellt und fünfmal mit je 50 ml Dichlormethan extrahiert. Die vereinigten organischen Phasen werden über MgS04 getrocknet und anschließend das Lösungsmittel im Vakuum entfernt. Man erhält 500 mg 1-(2,5-Dimethoxy-phenyl)-piperazin als amorphen Feststoff.
C12H18N2O2 (222.29), LCMS(ESI): 223.3 (M+H⁺).

### 2-Piperazin-1-yl-terephthalsäuredimethylester

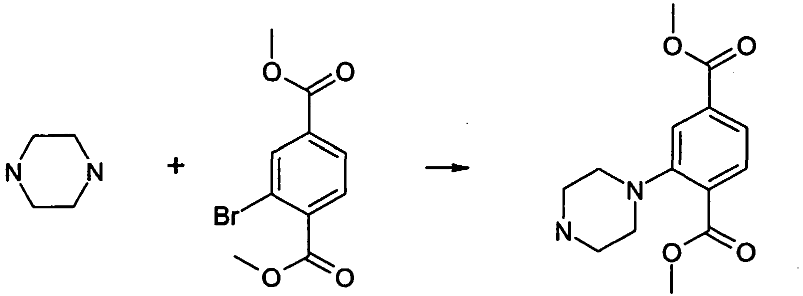

1.80 g Piperazin, 1.0 g 2-Brom- terephthalsäuredimethylester, 720 mg 9,9-Diemthyl-4,5-bis(diphenylphosphino)xanthene, 280 mg Palladium(II)acetat und 2.20 g Cäsiumcarbonat werden in 40 ml 1,4-Dioxan gelöst und unter einer Argonatmosphäre 24 Stunden bei 100 °C gerührt. Das Reaktionsgemisch wird mit 100 ml Dichlormethan verdünnt und mit 50 ml gesättigter Natriumhydrogencarbonatlösung und 50 ml gesättigter Natriumchloridlösung extrahiert. Die organischenPhasen wird über MgS04 getrocknet und anschließend das Lösungsmittel im Vakuum entfernt. Man erhält 330 mg 2-Piperazin-1-yl-terephthalsäuredimethylester als Öl.
C14H18N2O4 (278.31), LCMS(ESI): 279.3 (M+H⁺).

### Beispielsynthesen nach Verfahren B:

### Beispiel 1

### [1-(3,5-Dimethyl-isoxazol-4-sulfonyl)3,4-trans-4-p-tolyl-pyrrolidin-3-yl]-[4-(2,5-dimethylphenyl)-piperazin-1-yl]-methanontrifluoracetat

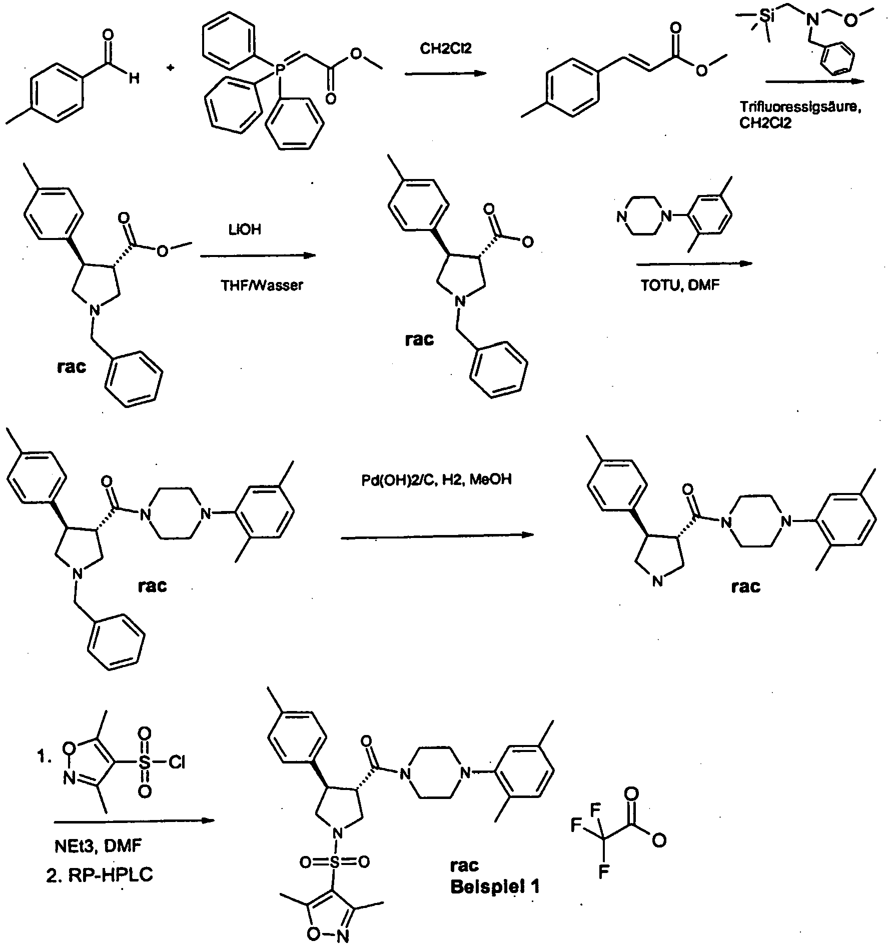

### trans-3-p-Tolyl-acrylsäuremethylester

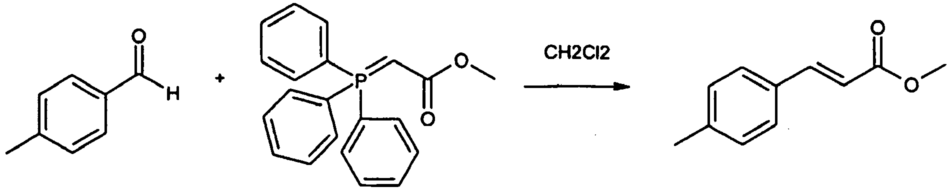

2.0 g Methyl(triphenylphosphoranyliden)acetat und 720 mg p-Toluolaldehyd werden in 10 ml Dichlormethan gelöst und zwölf Stunden bei Raumtemperatur gerührt. Danach werden 4g Kieselgur hinzugegeben und das Lösungsmittel im Vakuum entfernt. Der resultierende Rückstand wird an Kieselgel mit dem Eluens n-Heptan:Ethylacetat = 4:1 gereinigt. Man erhält 850 mg trans-3-p-Tolyl-acrylsäuremethylester als Öl.
C11H1202 (176.22), LCMS(ESI): 177.2 (M+H⁺), Rf(n-Heptan:Ethylacetat = 2:1) = 0.71.

### 1-Benzyl-3,4-trans-4-p-tolyl-pyrrolidin-3-carbonsäuremethylester

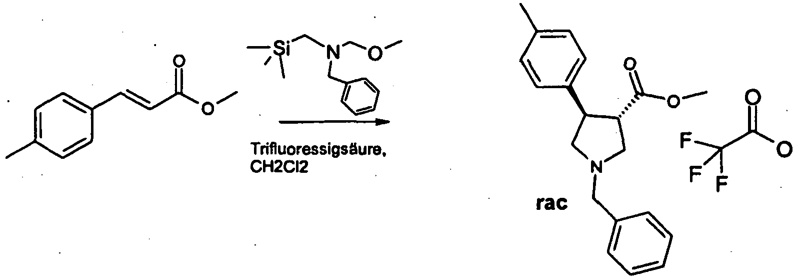

1 ml einer einmolaren Lösung von Trifluoressigsäure in Dichlormethan wird bei 0°C zu einer Lösung von 1.36 ml N-(Methoxymethyl)-N-(trimethylsilylmethyl)-benzylamin und 850 mg trans-3-p-Tolyl-acrylsäuremethylester in 10 ml Dichlormethan zugetropft. Nach zwölfstündigem Rühren bei Raumtemperatur wird das Lösungsmittel im Vakuum entfernt und der Rückstand durch RP-HPLC gereinigt. Man erhält 1.18 g 1-Benzyl-3,4-trans-4-p-tolyl-pyrrolidin-3-carbonsäuremethylesterdrifluoracetat als farbloses Öl.
C20H23N02.C2HF3O2 (423.43), LCMS(ESI): 310.4 (M+H⁺).

### 1-Benzyl-3,4-trans-4-p-tolyl-pyrrolidin-3-carbonsäure

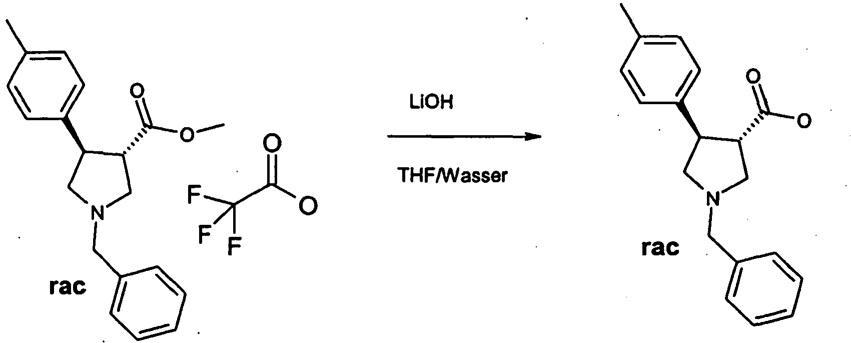

1.18 g 1-Benzyl-3,4-trans-4-p-tolyl-pyrrolidin-3-carbonsäuremethylestertrifluoracetat werden in 8 ml eines Gemischs aus Tetrahydrofuran und Wasser im Verhältnis Fünf zu Drei gelöst und mit 632 mg Lithiumhydroxid versetzt. Das Reaktionsgemisch wird zwei Stunden bei 80°C gerührt. Durch Zugabe verdünnter Essigsäure wird das Reaktionsgemisch neutralisiert, das THF im Vakuum entfernt und der Rückstand gefriergetrocknet. Der resultierende Rückstand wird an Kieselgel mit dem Eluens Dichlormethan:Methanol = 10:1=> 5:1 gereinigt. Man erhält 1.3 g 1-Benzyl-3,4-trans-4-p-tolyl-pyrrolidin-3-carbonsäure als farbloses Öl.
C19H21NO2 (295.38), LCMS(ESI): 296.4 (M+H⁺).

### (1-Benzyl-3,4-trans-4-p-tolyl-pyrrolidin-3-yl)-[4-(2,5-dimethyl-phenyl)-piperazin-1-yl]-methanon

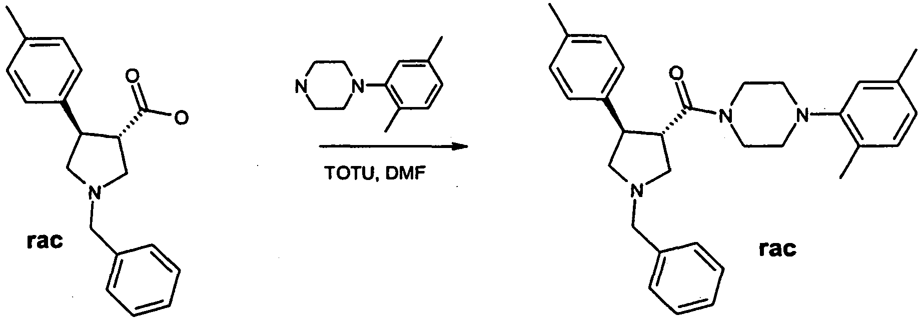

500 mg 1-Benzyl-3,4-trans-4-p-tolyl-pyrrolidin-3-carbonsäure, 322 mg kommerziell erhältliches 1-(2,5-Dimethylphenyl)piperazin und 0.97 ml Triethylamin werden in 30 ml Dimethylformamid gelöst. Man gibt 555 mg O-[Cyan(ethoxycarbonyl)methylenamino]-1,1,3,3-tetramethyluronium-tetrafluoroborat hinzu und rührt bei Raumtemperatur nach. Nach einer Stunde wird das Reaktionsgenmisch durch Zugabe von 100 ml Ethylacetat verdünnt und fünfmal mit je 30 ml gesättigter Natriumhydrogencarbonatlösung gewaschen. Die organische Phase wird über MgS04 getrocknet und anschließend das Lösungsmittel im Vakuum entfernt. Man erhält 520 mg (1-Benzyl-3,4-trans-4-p-tolyl-pyrrolidin-3-yl)-[4-(2,5-dimethyl-phenyl)-piperazin-1-yl]- methanon als Öl.
C31H37N30 (467.66), LCMS(ESI): 468.7(M+H⁺).

### [4-(2,5-Dimethyl-phenyl)-piperazin-1-yl]-(3,4-trans-4-p-tolyl-pyrrolidin-3-yl)-methanon

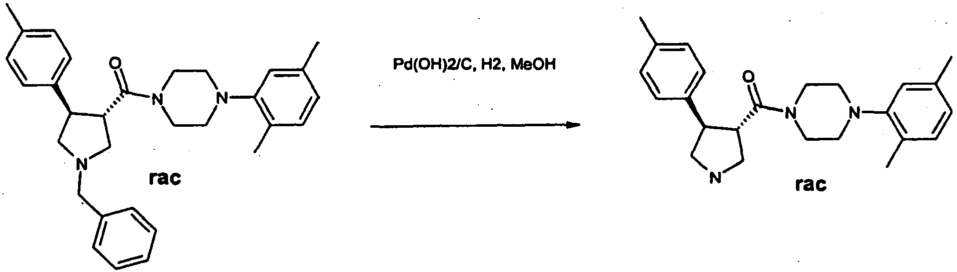

520 mg (1-Benzyl-3,4-trans-4-p-tolyl-pyrrolidin-3-yl)-[4-(2,5-dimethyl-phenyl)-piperazin-1-yl]- methanon werden in 15 ml Methanol gelöst und mit 30 mg Palladiumhydroxid auf Kohle versetzt. Es wird vier Stunden unter einer wasserstoffatmosphäre von 5 bar gerührt. Anschließend wird das Reaktionsgemisch über Celite filtriert und das Filtrat im Vakuum eingeengt. Man erhält 360 mg [4-(2,5-Dimethyl-phenyl)-piperazin-1-yl]-(3,4-trans-4-p-tolyl-pyrrolidin-3-yl)-methanon als Öl.
C24H31N3O (377.53), LCMS(ESI): 378.5 (M+H⁺).

### [1-(3,5-Dimethyl-isoxazol-4-sulfonyl)3,4-trans-4-p-tolyl-pyrrolidin-3-yl]-[4-(2,5-dimethylphenyl)-piperazin-1-yl]-methanontrifluoracetat

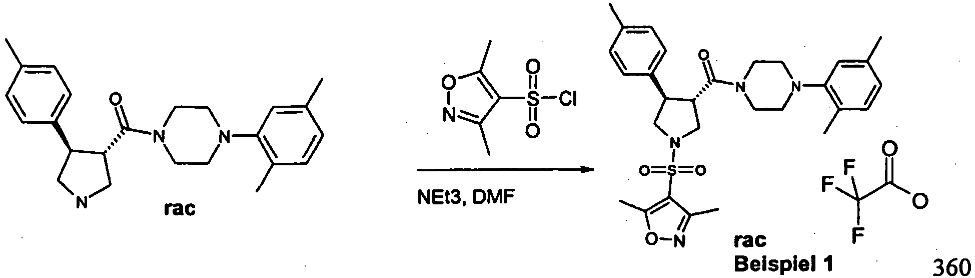

mg [4-(2,5-Dimethyl-phenyl)-piperazin-1-yl]-(3,4-trans-4-p-tolyl-pyrrolidin-3-yl)-methanon und 0.56 ml N,N-Diisopropyl-N-Ethyl-amin werden in 10 ml Dimethylformamid gelöst und mit 186 mg 3,5-Dimethylisoxazol-4-sulfonylchlorid versetzt. Nach 15 Minuten wird das Reaktionsgemisch durch Zugabe von 50 ml Ethylacetat verdünnt und mit 20 ml gesättigter Natriumhydrogencarbonatlösung und dreimal mit je 20 ml Wasser gewaschen. Die organische Phase wird über MgS04 getrocknet und anschließend das Lösungsmittel im Vakuum entfernt. Der Rückstand wird mittels RP-HPLC gereinigt. Man erhält 212 mg [1-(3,5-Dimethyl-isoxazol-4-sulfonyl)3,4-trans-4-p-tolyl-pyrrolidin-3-yl]-[4-(2,5-dimethyl-phenyl)-piperazin-1-yl]-methanontrifluoracetat als farbloses Lyophilisat.
C29H36N4O4S.C2HF3O2 (650.72), LCMS(ESI): 537.3 (M+H⁺).

### Beispiel 2

### [1-(Biphenyl-4-sulfonyl)-3,4-trans-4-phenyl-pyrrolidin-3-yl]-(4-o-tolyl-piperazin-1-yl)-methanon

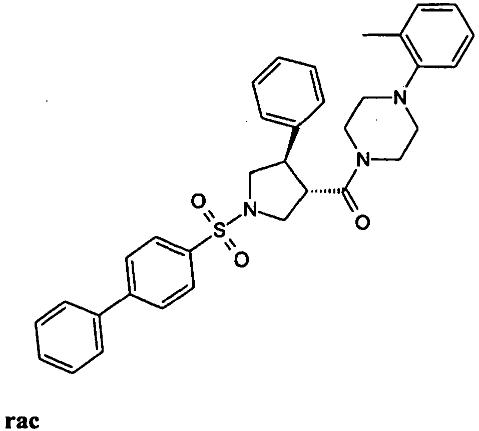

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-o-Tolylpiperazin und Biphenyl-4-sulfonylchlorid {[1-(Biphenyl-4-sulfonyl)-3,4-trans-4-phenyl-pyrrolidin-3-yl]-(4-o-tolyl-piperazin-1-yl)- methanon.
C34H35N3O3S (565.74), LCMS(ESI): 566.8 (M+H⁺).

### Beispiel 3

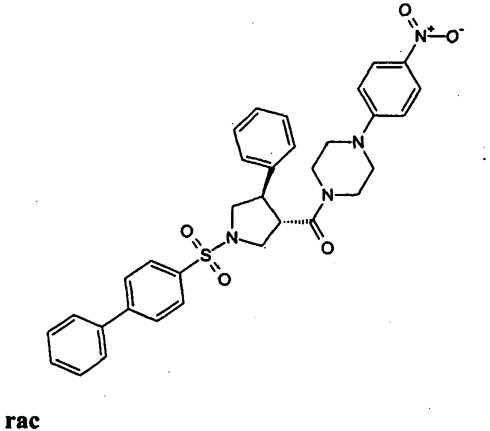

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(1-Nitropyridin-4-yl)-piperazin und Biphenyl-4-sulfonylchlorid [1-(Biphenyl-4-sulfonyl)-3,4-trans-4-phenyl-pyrrolidin-3-yl]-[4-(4-nitro-phenyl)-piperazin-1-yl]- methanon.
C33H32N4O5S (596.71), LCMS(ESI): 597.7 (M+H⁺).

### Beispiel 4

### [1-(2,5-Dichloro-benzolsulfonyl)-3,4-trans-4-phenyl-pyrrolidin-3-yl]-[4-(3-trifluoromethylphenyl)- piperazin-1-yl]-methanon

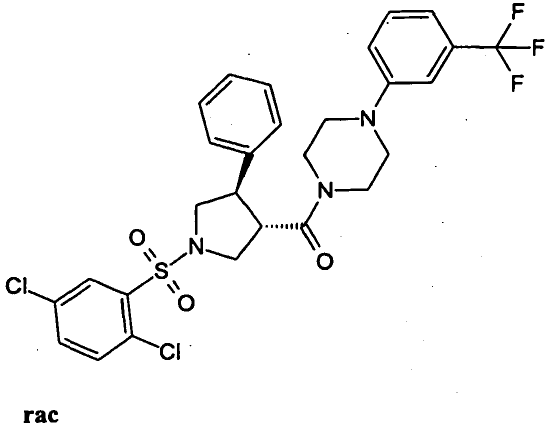

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(3-Trifluoromethyl-phenyl)-piperazin und 2,5-Dichloro-benzolsulfonylchlorid [1-(2,5-Dichlorobenzolsulfonyl)-3,4-trans-4-Phenyl-pyrrolidin-3-yl]-[4-(3-trifluoromethyl-phenyl)-piperazin-1-yl]-methanon.
C28H26Cl2F3N3O3S (612.50), LCMS(ESI): 612.1, 614.1 (M+H⁺).

### Beispiel 5

### [1-(2,5-Dimethoxy-benzolsulfonyl)-3,4-trans-4-phenyl-pyrrolidin-3-yl]-(4-phenyl-piperazin-1-yl)- methanon

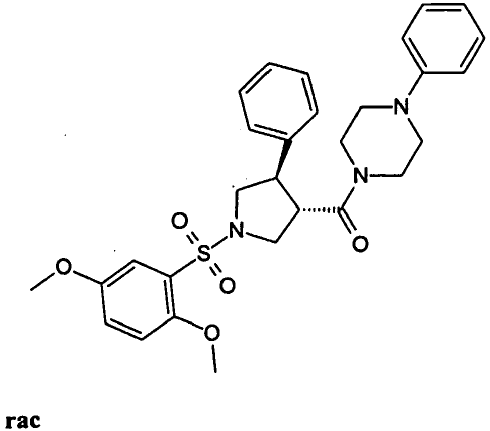

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-Phenylpiperazin und 2,5-Dimethoxy-benzolsulfonylchlorid [1-(2,5-Dimethoxy-benzolsulfonyl)-3,4-trans-4-phenyl-pyrrolidin-3-yl]-(4-phenyl-piperazin-1-yl)-methanon.
C29H33N3O5S (535.67), LCMS(ESI): 536.7 (M+H⁺).

### Beispiel 6

### (4-Phenyl-piperazin-1-yl)-[3,4-trans-4-phenyl-1-(2,4,6-triisopropyl-benzolsulfonyl)-pyrrolidin-3-yl]-methanon

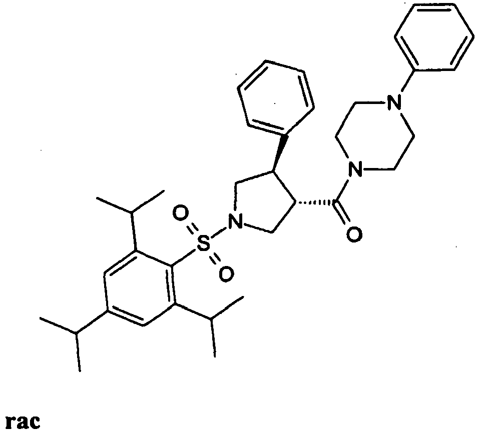

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-Phenylpiperazin und 2,4,6-Triisopropyl-benzolsulfonylchlorid (4-Phenyl-piperazin-1-yl)-[3,4-trans-4-phenyl-1-(2,4,6-triisopropyl-benzolsulfonyl)-pyrrolidin-3-yl]-methanon.
C36H47N3O3S (601.86), LCMS(ESI): 602-9 (M+H⁺).

### Beispiel 7

### 1-(4-{4-[3,4-trans-4-Phenyl-1-(thiophene-2-sulfonyl)-pyrrolidine-3-carbonyl]-piperazin-1-yl}-phenyl)-ethanon

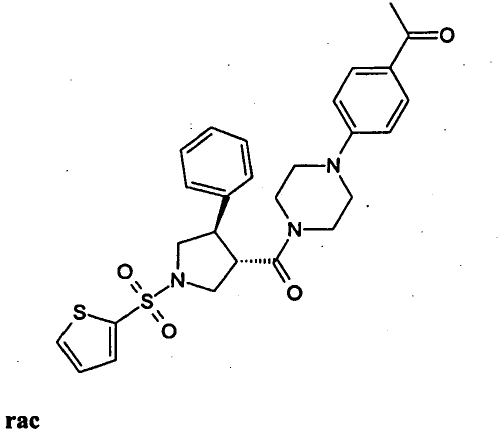

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(4-Piperazin-1-yl-phenyl)-ethanon und Thiophen-2-sulfonylchlorid 1-(4-{4-[3,4-trans-4-Phenyl-1-(thiophene-2-sulfonyl)-pyrrolidine-3-carbonyl]-piperazin-1-yl}-phenyl)-ethanon.
C27H29N3O4S2 (523.68), LCMS(ESI): 524.7 (M+H⁺).

### Beispiel 8

### [1-(4-Chloro-benzolsulfonyl)-3,4-trans-4-phenyl-pyrrolidin-3-yl]-[4-(2,5-dimethyl-phenyl)-piperazin-1-yl]-methanon

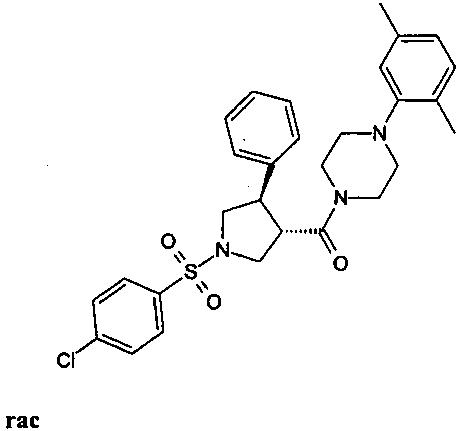

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethyl-phenyl)-piperazin und 5-Chlor-thiophen-2-sulfonylchlorid [1-(4-Chlorobenzolsulfonyl)-3,4-trans-4-phenyl-pyrrolidin-3-yl]-[4-(2,5-dimethyl-phenyl)-piperazin-1-yl]-methanon.
C29H32ClN3O3S (538.11), LCMS(ESI): 538.1 (M+H⁺).

### Beispiel 9

### [4-(2,3-Dimethyl-phenyl)-piperazin-1-yl]-[3,4-trans-4-phenyl-1-(4-propyl-benzolsulfonyl)-pyrrolidin-3-yl]-methanon

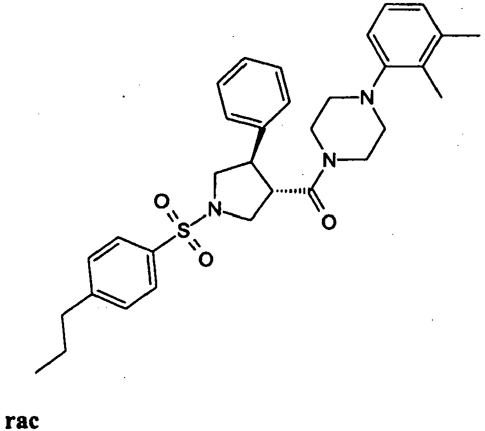

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,3-Dimethyl-phenyl)-piperazin und 4-Propyl-benzolsulfonylchlorid [4-(2,3-Dimethyl-phenyl)-piperazin-1-yl]-[3,4-trans-4-phenyl-1-(4-propyl-benzolsulfonyl)-pyrrolidin-3-yl]-methanon.
C32H39N3O3S (545.75), LCMS(ESI): 546.8 (M+H⁺).

### Beispiel 10

### [1-(3-Chlor-4-fluor-benzolsulfonyl)-3,4-trans-4-phenyl-pyrrolidin-3-yl]-[4-(2,5-dimethylphenyl)-piperazin-1-yl]-methanon

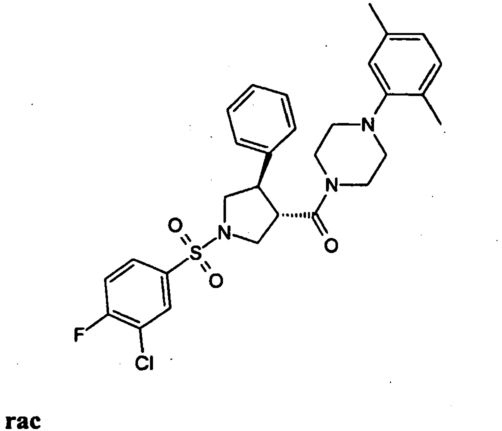

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethyl-phenyl)-piperazin und 3-Chlor-4-fluor-benzolsulfonylchlorid [1-(3-Chlor-4-fluorbenzolsulfonyl)-3,4-trans-4-phenyl-pyrrolidin-3-yl]-[4-(2,5-dimethyl-phenyl)-piperazin-1-yl]-methanon.
C29H31ClFN3O3S (556.10), LCMS(ESI): 556.3 (M+H⁺).

### Beispiel 11

### [4-(2-Chlor-phenyl)-piperazin-1-yl]-[3,4-trans-4-phenyl-1-(quinolin-8-sulfonyl)-pyrrolidin-3-yl]- methanon

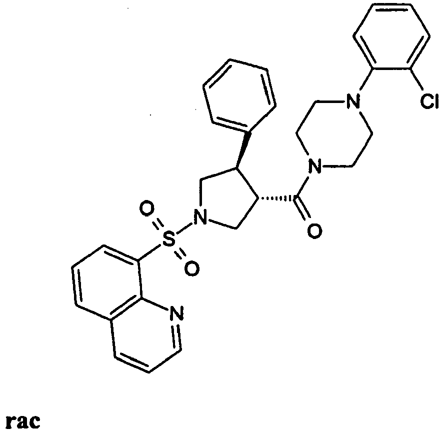

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2-Chlorphenyl)-piperazin und Quinolin-8-sulfonylchlorid [4-(2-Chlor-phenyl)-piperazin-1-yl]-[3,4-trans-4-phenyl-1-(quinolin-8-sulfonyl)-pyrrolidin-3-yl]-methanon.
C30H29ClN4O3S (561.11), LCMS(ESI): 561.1 (M+H⁺).

### Beispiel 12

### [4-(4-Chlor-phenyl)-piperazin-1-yl]-(3,4-trans-4-phenyl-1-phenylmethansulfonyl-pyrrolidin-3-yl)- methanon

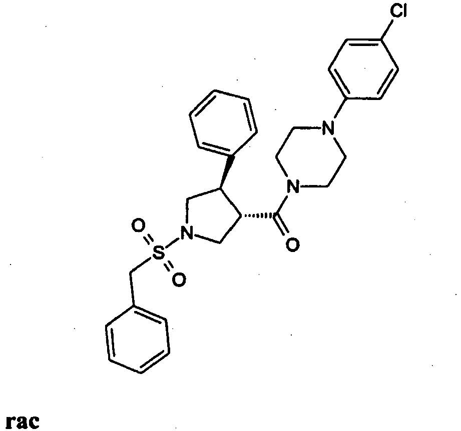

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(4-Chlorphenyl)-piperazin und Phenyl-methansulfonylchlorid [4-(4-Chlor-phenyl)-piperazin-1-yl]-(3,4-trans-4-phenyl-1-phenylmethansulfonyl-pyrrolidin-3-yl)-methanon.
C28H30ClN3O3S (524.09), LCMS(ESI): 524.1 (M+H⁺).

### Beispiel 13

### [4-(4-Methoxy-phenyl)-piperazin-1-yl]-[3,4-trans-4-phenyl-1-(2-phenyl-(E)-ethensulfonyl)-pyrrolidin-3- yl]-methanon

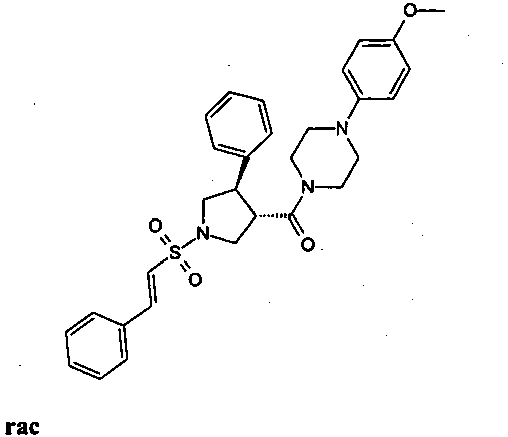

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(4-Methoxy-phenyl)-piperazin und (E)-3-Phenyl-prop-2-en-1-sulfonylchlorid [4-(4-Methoxyphenyl)-piperazin-1-yl]-[3,4-trans-4-phenyl-1-(2-phenyl-(E)-ethensulfonyl)-pyrrolidin-3-yl]-methanon.
C30H33N3O4S (531.68), LCMS(ESI): 532.7 (M+H⁺).

### Beispiel 14

### [4-(2-Chlor-phenyl)-piperazin-1-yl]-[3,4-trans-4-phenyl-1-(4-trifluoromethoxybenzolsulfonyl)-pyrrolidin-3-yl]-methanon

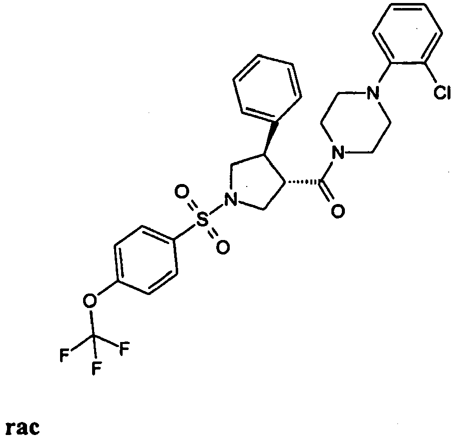

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2-Chlorphenyl)-piperazin und 4-Trifluoromethoxy-benzolsulfonylchlorid; [4-(2-Chlor-phenyl)-piperazin-1-yl]-[3,4-trans-4-phenyl-1-(4-trifluoromethoxy-benzolsulfonyl)-pyrrolidin-3-yl]-methanon.
C28H27ClF3N3O4S (594.06), LCMS(ESI): 594.1 (M+H⁺).

### Beispiel 15

### 1-(4-Chlor-phenyl)-piperazin und 4-Nitro-benzolsulfonylchlorid; [4-(4-Chlor-phenyl)-piperazin-1-yl]-[1-(4-nitro-benzolsulfonyl)-3,4-trans-4-phenyl-pyrrolidin-3-yl]-methanon

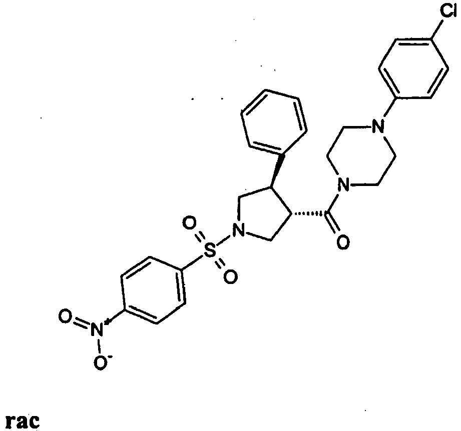

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(4-Chlorphenyl)-piperazin und 4-Nitro-benzolsulfonylchlorid; [4-(4-Chlor-phenyl)-piperazin-1-yl]-[1-(4-nitro-benzolsulfonyl)-3,4-trans-4-phenyl-pyrrolidin-3-yl]-methanon.
C27H27ClN4O5S (555.06), LCMS(ESI): 555.1 (M+H⁺).

### Beispiel 16

### [1-(4-tert.-Butyl-benzolsulfonyl)-3,4-trans-4-phenyl-pyrrolidin-3-yl]-[4-(4-methoxy-phenyl)-piperazin-1-yl]-methanon

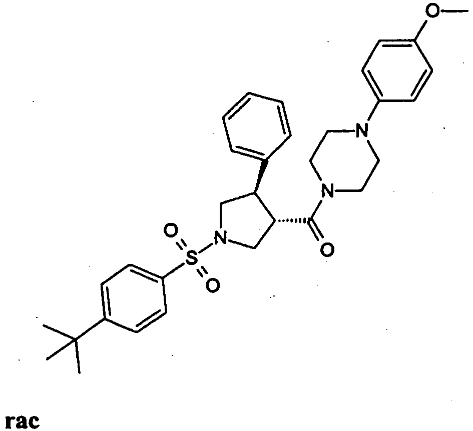

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(4-Methoxy-phenyl)-piperazin und 4-tert.-Butyl-benzol-sulfonylchlorid [1-(4-tert.-Butylbenzolsulfonyl)-3,4-trans-4-phenyl-pyrrolidin-3-yl]-[4-(4-methoxy-phenyl)-piperazin-1-yl]-methanon.
C32H39N3O4S (561.75), LCMS(ESI): 562.8 (M+H⁺).

### Beispiel 17

### [4-(4-Methoxy-phenyl)-piperazin-1-yl]-[3,4-trans-4-phenyl-1-(3-trifluoromethylbenzolsulfonyl)-pyrrolidin-3-yl]-methanon

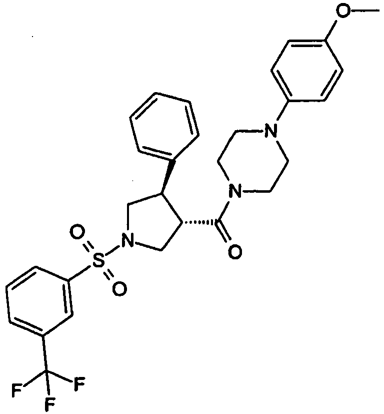

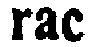

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(4-Methoxy-phenyl)-piperazin und 3-Trifluormethyl-benzol-sulfonylchlorid [4-(4-Methoxyphenyl)-piperazin-1-yl]-[3,4-trans-4-phenyl-1-(3-trifluoromethyl-benzolsulfonyl)-pyrrolidin-3-yl]-methanon.
C29H30F3N3O4S (573.64), LCMS(ESI): 574.6 (M+H⁺).

### Beispiel 18

### [4-(2-Ethoxy-phenyl)-piperazin-1-yl]-[1-(naphthalen-2-sulfonyl)-3,4-trans-4-phenyl-pyrrolidin-3-yl]-methanon

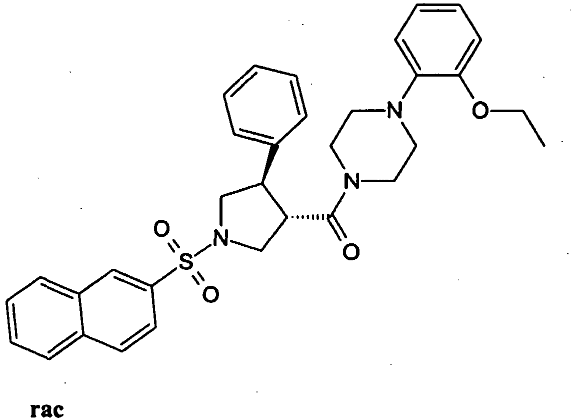

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2-Ethoxyphenyl)-piperazin und Naphthalen-2-sulfonylchlorid [4-(2-Ethoxy-phenyl)-piperazin-1-yl]-[1-(naphthalen-2-sulfonyl)-3,4-trans-4-phenyl-pyrrolidin-3-yl]-methanon.
C33H35N3O4S (569.73), LCMS(ESI): 570.7 (M+H⁺).

### Beispiel 19

### [1-(4,5-Dibrom-thiophen-2-sulfonyl)-3,4-trans-4-phenyl-pyrrolidin-3-yl]-[4-(2-fluor-phenyl)-piperazin-1-yl]-methanon

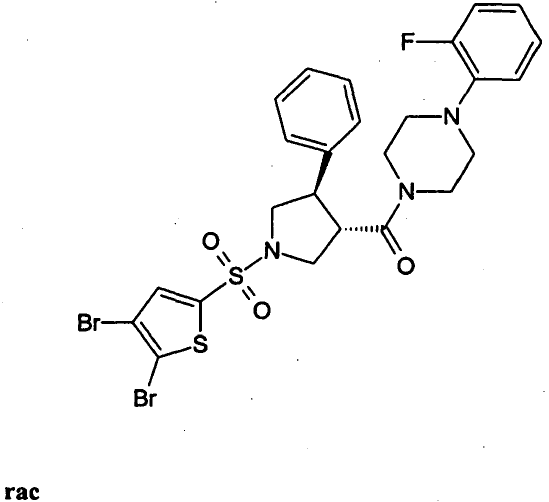

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2-Fluorphenyl)-piperazin und 4,5-Dibrom-thiophen-2-sulfonylchlorid [1-(4,5-Dibrom-thiophen-2-sulfonyl)-3,4-trans-4-phenyl-pyrrolidin-3-yl]-[4-(2-fluor-phenyl)-piperazin-1-yl]-methanon.
C25H24Br2FN3O3S2 (657.42), LCMS(ESI): 656.1, 660.1 (M+H⁺).

### Beispiel 20

### 1-(5-Chlor-thiophen-2-sulfonyl)-3,4-trans-4-phenyl-pyrrolidin-3-yl]-(4-phenyl-piperazin-1-yl)-methanon

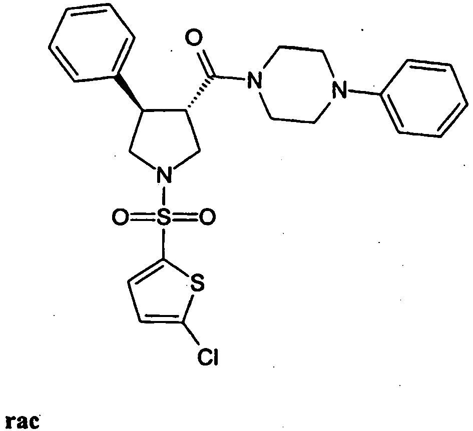

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-Phenylpiperazin und 5-Chlor-thiophen-2-sulfonylchlorid [1-(5-Chlor-thiophen-2-sulfonyl)-3,4-trans-4-phenyl-pyrrolidin-3-yl]-(4-phenyl-piperazin-1-yl)-methanon.
C25H26ClN3O3S2 (516.09), LCMS(ESI): 516.1 (M+H⁺).

### Beispiel 21

### [1-(4,5-Dichlor-thiophen-2-sulfonyl)-3,4-trans4-phenyl-pyrrolidin-3-yl]-(4-phenyl-piperazin-1-yl)-methanon

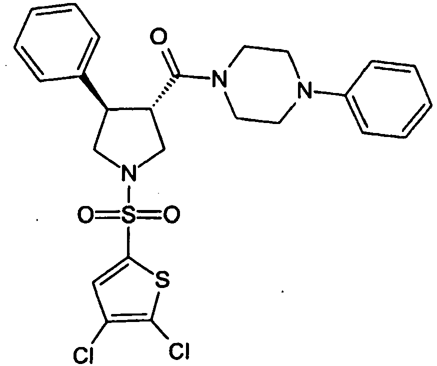

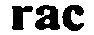

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-Phenylpiperazin und 4,5-Dichlor-thiophen-2-sulfonylchlorid [1-(4,5-Dichlor-thiophen-2-sulfonyl)-3,4-trans-4-phenyl-pyrrolidin-3-yl]-(4-phenyl-piperazin-1-yl)-methanon.
C25H25Cl2N3O3S2 (550.53), LCMS(ESI): 550.1 (M+H⁺).

### Beispiel 22

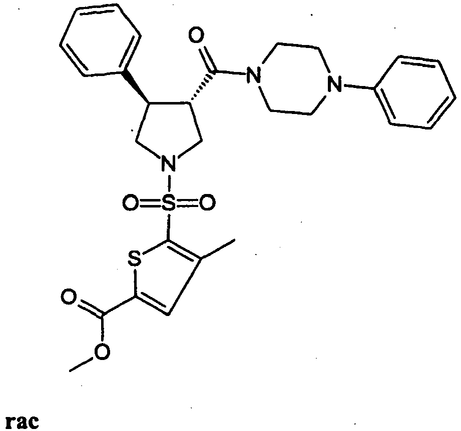

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-Phenylpiperazin und 5-Chlorsulfonyl-4-methyl-thiophen-2-carbonsäuremethylester 4-Methyl-5-[3,4-trans-3-phenyl-4-(4-phenyl-piperazine-1-carbonyl)-pyrrolidin-1-sulfonyl]-thiophen-2-carbonsäuremethylester.
C28H31N3O5S2 (553.70), LCMS(ESI): 554.1 (M+H⁺).

### Beispiel 23

### [1-(5-Chlor-3-methyl-benzo[b]thiophen-2-sulfonyl)-3,4-trans-4-phenyl-pyrrolidin-3-yl]-(4-phenyl- piperazin-1-yl)-methanon

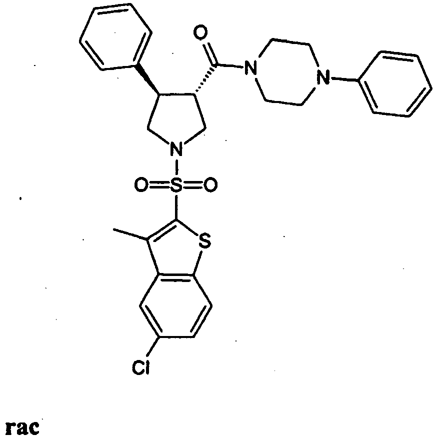

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-Phenylpiperazin und 5-Chlor-3-methyl-benzo[b]thiophen-2-sulfonylchlorid [1-(5-Chlor-3-methylbenzo[b]thiophen-2-sulfonyl)-3,4-trans-4-phenyl-pyrrolidin-3-yl]-(4-phenyl-piperazin-1-yl)-methanon.
C30H30ClN3O3S2 (580.17), LCMS(ESI): 580.2 (M+H⁺).

### Beispiel 24

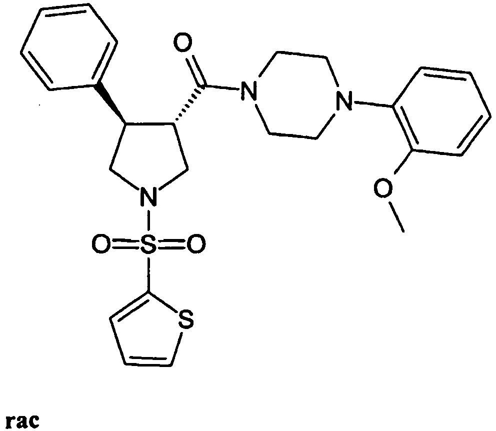

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2-Methoxy-phenyl)-piperazin und Thiophen-2-sulfonylchlorid [4-(2-Methoxy-phenyl)-piperazin-1-yl]-[3,4-trans-4-phenyl-1-(thiophen-2-sulfonyl)-pyrrolidin-3-yl]-methanon.
C26H29N3O4S2 (511.67), LCMS(ESI): 512.1 (M+H⁺).

### Beispiel 25

### [1-(4,5-Dibrom-thiophen-2-sulfonyl)-3,4-trans-4-phenyl-pyrrolidin-3-yl]-[4-(2-methoxyphenyl)-piperazin-1-yl]-methanon

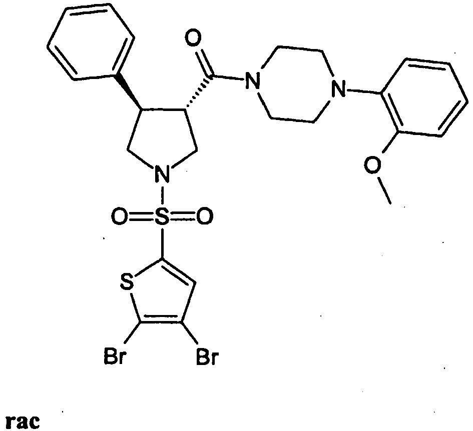

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2-Methoxy-phenyl)-piperazin und 4,5-Dibrom-thiophen-2-sulfonylchlorid [1-(4,5-Dibromthiophen-2-sulfonyl)-3,4-trans-4-phenyl-pyrrolidin-3-yl]-[4-(2-methoxy-phenyl)-piperazin-1-yl]-methanon.
C26H27Br2N3O4S2 (669.46), LCMS(ESI): 669.9, 671.9 (M+H⁺).

### Beispiel 26

### [1-(5-Chlor-thiophen-2-sulfonyl)-3,4-trans-4-phenyl-pyrrolidin-3-yl]-[4-(2-methoxy-phenyl)-piperazin-1-yl]-methanon

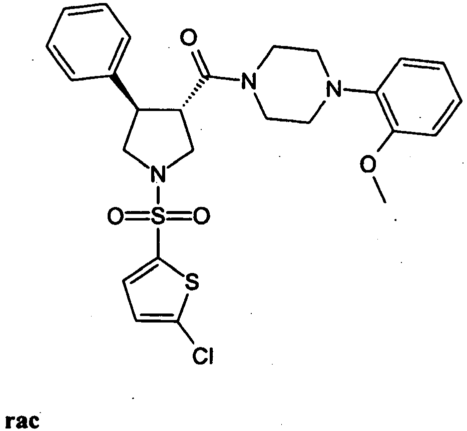

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2-Methoxy-phenyl)-piperazin und 5-Chlor-thiophen-2-sulfonylchlorid [1-(5-Chlor-thiophen-2-sulfonyl)-3,4-trans-4-phenyl-pyrrolidin-3-yl]-[4-(2-methoxy-phenyl)-piperazin-1-yl]-methanon.
C26H28ClN3O4S2 (546.11), LCMS(ESI): 546.0 (M+H⁺).

### Beispiel 27

### [1-(4,5-Dichlor-thiophen-2-sulfonyl)-3,4-trans-4-phenyl-pyrrolidin-3-yl]-[4-(2-methoxyphenyl)- piperazin-1-yl]-methanon

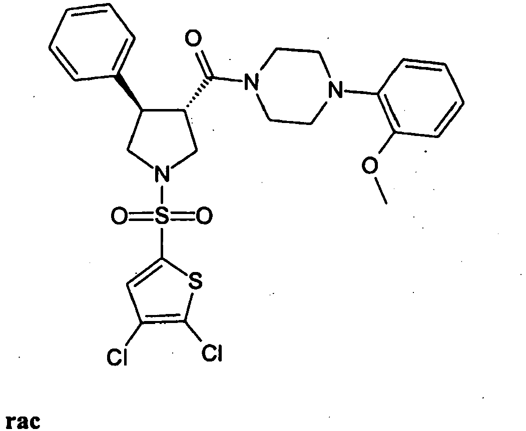

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2-Methoxy-phenyl)-piperazin und 4,5-Dichlor-thiophen-2-sulfonylchlorid [1-(4,5-Dichlorthiophen-2-sulfonyl)-3,4-trans-4-phenyl-pyrrolidin-3-yl]-[4-(2-methoxy-phenyl)- piperazin-1-yl]-methanon.
C26H27Cl2N3O4S2 (580.56), LCMS(ESI): 580.0 (M+H⁺).

### Beispiel 28

### [5-{3,4-trans-3-[4-(2-Methoxy-phenyl)-piperazin-1-carbonyl]-4-phenyl-pyrrolidin-1-sulfonyl}-4- methyl-thiophen-2-carbonsäuremethylester

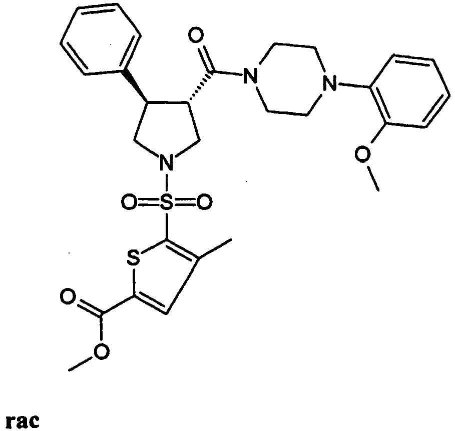

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2-Methoxy-phenyl)-piperazin und 5-Chlorsulfonyl-4-methyl-thiophen-2-carbonsäuremethylester [5-{3,4-trans-3-[4-(2-Methoxy-phenyl)-piperazin-1-carbonyl]-4-phenyl-pyrrolidin-1-sulfonyl}-4- methyl-thiophen-2-carbonsäuremethylester.
C29H33N3O6S2 (583.73), LCMS(ESI): 584.1 (M+H⁺).

### Beispiel 29

### [1-(5-Chlor-3-methyl-benzo[b]thiophen-2-sulfonyl)-3,4-trans-4-phenyl-pyrrolidin-3-yl]-[4-(2-methoxy-phenyl)-piperazin-1-yl]-methanon

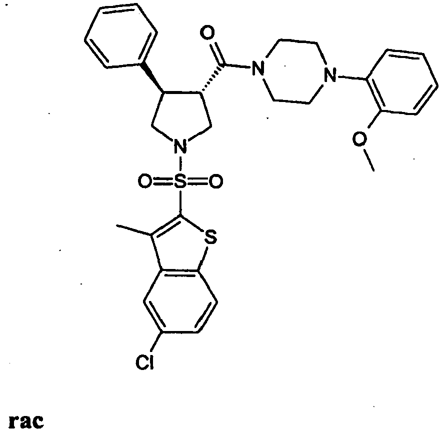

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2-Methoxy-phenyl)-piperazin und 5-Chlor-3-methyl-benzo[b]thiophen-2-sulfonylchlorid [1-(5-Chlor-3-methyl-benzo[b]thiophen-2-sulfonyl)-3,4-trans-4-phenyl-pyrrolidin-3-yl]-[4-(2-methoxy-phenyl)-piperazin-1-yl]-methanon.
C31H32ClN3O4S2 (610.20), LCMS(ESI): 610.2 (M+H⁺).

### Beispiel 30

### [3,4-trans-4-Phenyl-1-(thiophen-2-sulfonyl)-pyrrolidin-3-yl]-[4-(3-trifluoromethyl-phenyl)-piperazin-1-yl]-methanon

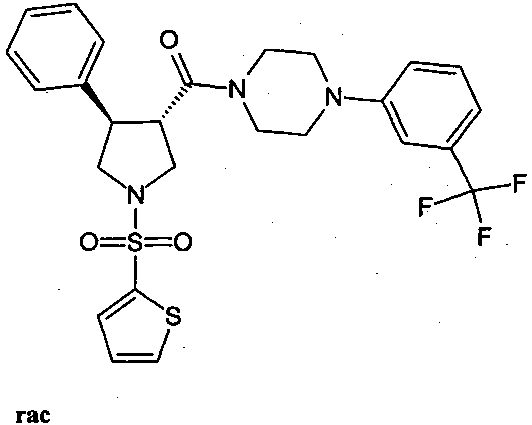

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(3-Trifluormethyl-phenyl)-piperazin und Thiophen-2-sulfonylchlorid [3,4-trans-4-Phenyl-1-(thiophen-2-sulfonyl)-pyrrolidin-3-yl]-[4-(3-trifluoromethyl-phenyl)-piperazin-1-yl]-methanon. C26H26F3N3O3S2 (549.64), LCMS(ESI): 550.1 (M+H⁺).

### Beispiel 31

### [1-(4,5-Dibrom-thiophen-2-sulfonyl)-3,4-trans-4-phenyl-pyrrolidin-3-yl]-[4-(3-trifluoromethylphenyl)-piperazin-1-yl]-methanon

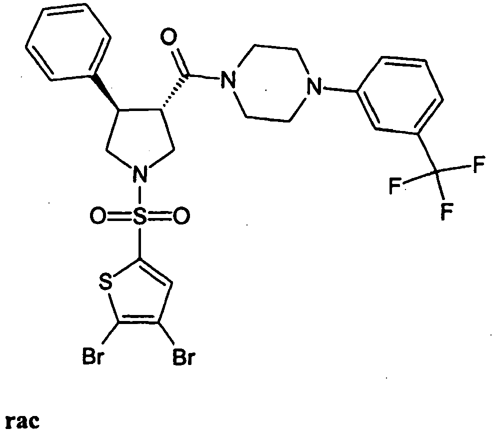

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(3-Trifluormethyl-phenyl)-piperazin und 4,5-Dibrom-thiophen-2-sulfonylchlorid [1-(4,5-Dibromthiophen-2-sulfonyl)-3,4-trans-4-phenyl-pyrrolidin-3-yl]-[4-(3-trifluoromethyl- phenyl)-piperazin-1-yl]-methanon.
C26H24Br2F3N3O3S2 (707.43), LCMS(ESI): 708.0 (M+H⁺).

### Beispiel 32

### [1-(5-Chlor-thiophen-2-sulfonyl)-3,4-trans-4-phenyl-pyrrolidin-3-yl]-[4-(3-trifluoromethylphenyl)-piperazin-1-yl]-methanon

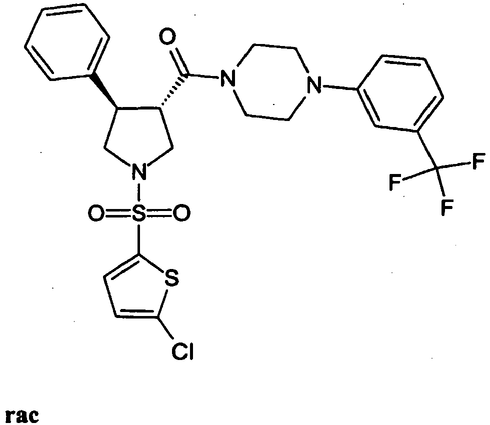

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(3-Trifluormethyl-phenyl)-piperazin und 5-Chlor-thiophen-2-sulfonylchlorid [1-(5-Chlorthiophen-2-sulfonyl)-3,4-trans-4-phenyl-pyrrolidin-3-yl]-[4-(3-trifluoromethyl-phenyl)-piperazin-1-yl]-methanon.
C26H25ClF3N3O3S2 (584.08), LCMS(ESI): 584.1 (M+H⁺).

### Beispiel 33

### [1-(4,5-Dichlor-thiophen-2-sulfonyl)-3,4-trans-4-phenyl-pyrrolidin-3-yl]-[4-(3-trifluormethylphenyl)-piperazin-1-yl]-methanon

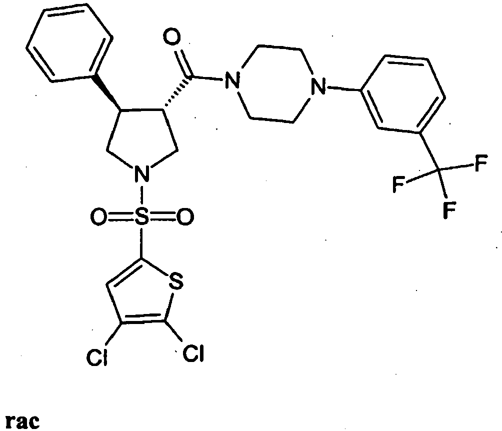

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(3-Trifluormethyl-phenyl)-piperazin und 4,5-Dichlor-thiophen-2-sulfonylchlorid [1-(4,5-Dichlorthiophen-2-sulfonyl)-3,4-trans-4-phenyl-pyrrolidin-3-yl]-[4-(3-trifluormethyl-phenyl)-piperazin-1-yl]-methanon.
C26H24Cl2F3N3O3S2 (618.53), LCMS(ESI): 618.1 (M+H⁺).

### Beispiel 34

### 4-Methyl-5-{3,4-trans-3-phenyl-4-[4-(3-trifluoromethyl-phenyl)-piperazin-1-carbonyl]-pyrrolidin-1- sulfonyl}-thiophen-2-carbonsäuremethylester

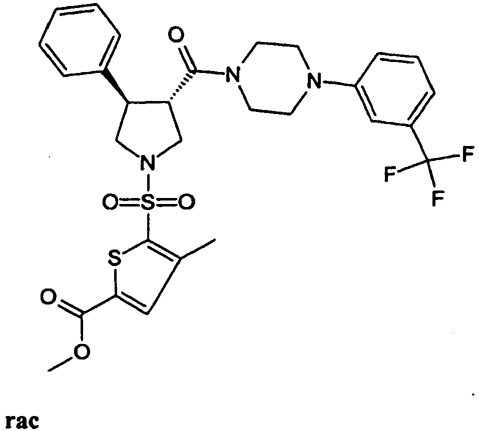

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(3-Trifluormethyl-phenyl)-piperazin und 5-Chlorsulfonyl-4-methyl-thiophen-2-carbonsäuremethylester 4-Methyl-5-{3,4-trans-3-phenyl-4-[4-(3-trifluoromethyl-phenyl)-piperazin-1-carbonyl]-pyrrolidin-1-sulfonyl}-thiophen-2-carbonsäuremethylester.
C29H30F3N3O5S2 (621.70), LCMS(ESI): 622.2 (M+H⁺).

### Beispiel 35

### [1-(5-Chlor-3-methyl-benzo[b]thiophen-2-sulfonyl)-3,4-trans-4-phenyl-pyrrolidin-3-yl]-[4-(3-trifluoromethyl-phenyl)-piperazin-1-yl]-methanon

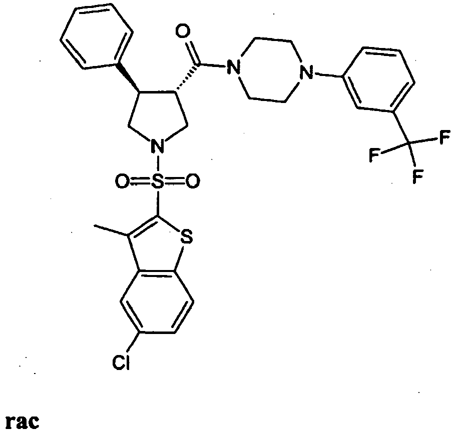

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(3-Trifluormethyl-phenyl)-piperazin und 5-Chlor-3-methyl-benzo[b]thiophen-2-sulfonylchlorid [1-(5-Chlor-3-methyl-benzo[b]thiophen-2-sulfonyl)-3,4-trans-4-phenyl-pyrrolidin-3-yl]-[4-(3-trifluoromethyl-phenyl)-piperazin-1-yl]-methanon.
C31H29ClF3N3O3S2 (648.17), LCMS(ESI): 648.1 (M+H⁺).

### Beispiel 36

### [4-(2,4-Dimethyl-phenyl)-piperazin-1-yl]-[3,4-trans-4-phenyl-1-(thiophen-2-sulfonyl)-pyrrolidin-3- yl]-methanon

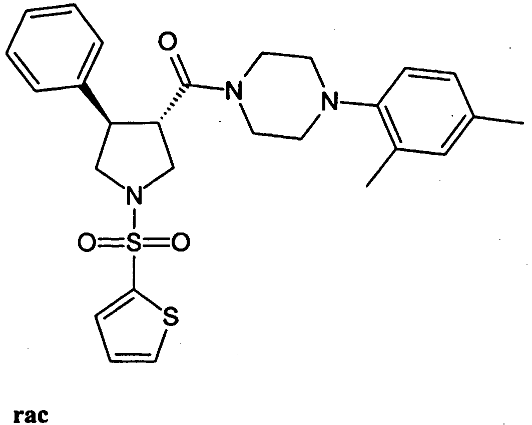

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,4-Dimethyl-phenyl)-piperazin und Thiophen-2-sulfonylchlorid [4-(2,4-Dimethyl-phenyl)-piperazin-1-yl]-[3,4-trans-4-phenyl-1-(thiophen-2-sulfonyl)-pyrrolidin-3-yl]-methanon.
C27H31N3O3S2 (509.69), LCMS(ESI): 510.2 (M+H⁺).

### Beispiel 37

### [1-(4,5-Dibrom-thiophen-2-sulfonyl)-3,4-trans-4-phenyl-pyrrolidin-3-yl]-[4-(2,4-dimethylphenyl)- piperazin-1-yl]-methanon

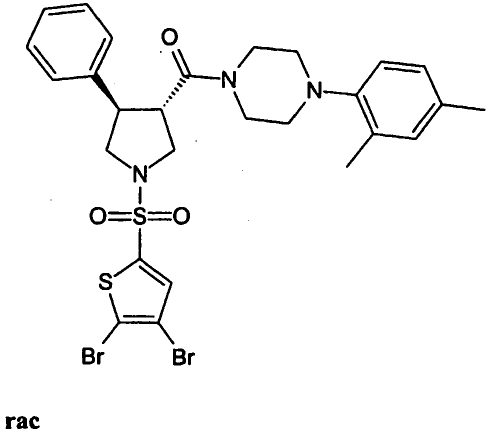

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,4-Dimethyl-phenyl)-piperazin und 4,5-Dibrom-thiophen-2-sulfonylchlorid [1-(4,5-Dibromthiophen-2-sulfonyl)-3,4-trans-4-phenyl-pyrrolidin-3-yl]-[4-(2,4-dimethyl-phenyl)- piperazin-1-yl]-methanon.
C27H29Br2N3O3S2 (667.49), LCMS(ESI): 668.0 (M+H⁺).

### Beispiel 38

### [1-(5-Chlor-thiophen-2-sulfonyl)-3,4-trans-4-phenyl-pyrrolidin-3-yl]-[4-(2,4-dimethyl-phenyl)-piperazin-1-yl]-methanon

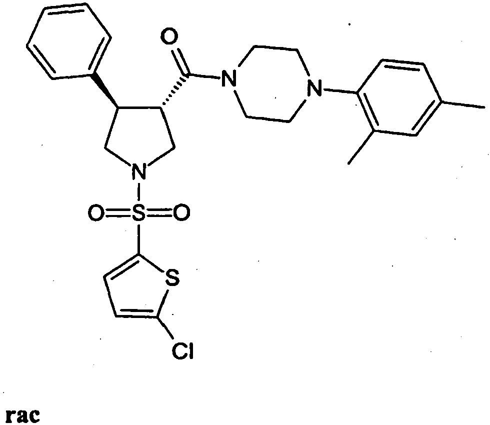

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,4-Dimethyl-phenyl)-piperazin und 5-Chlor-thiophen-2-sulfonylchlorid [1-(5-Chlor-thiophen-2-sulfonyl)-3,4-trans-4-phenyl-pyrrolidin-3-yl]-[4-(2,4-dimethyl-phenyl)- piperazin-1-yl]-methanon.
C27H30ClN3O3S2 (544.14), LCMS(ESI): 544.2 (M+H⁺).

### Beispiel 39

### [1-(4,5-Dichlor-thiophene-2-sulfonyl)-3,4-trans-4-phenyl-pyrrolidin-3-yl]-[4-(2,4-dimethylphenyl)- piperazin-1-yl]-methanon

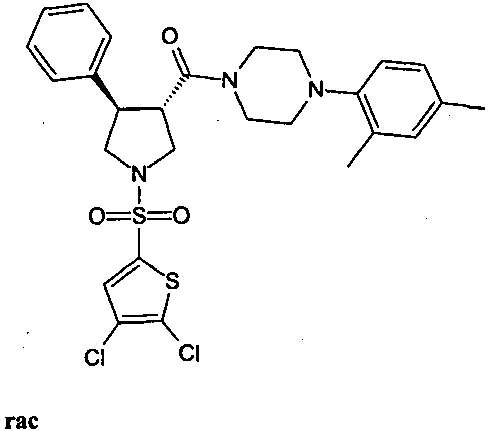

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,4-Dimethyl-phenyl)-piperazin und 4,5-Dichlor-thiophen-2-sulfonylchlorid [1-(4,5-Dichlorthiophene-2-sulfonyl)-3,4-trans-4-phenyl-pyrrolidin-3-yl]-[4-(2,4-dimethyl-phenyl)- piperazin-1-yl]-methanon.
C27H29Cl2N3O3S2 (578.58), LCMS(ESI): 578.1 (M+H⁺).

### Beispiel 40

### 5-{3,4-trans-3-[4-(2,4-Dimethyl-phenyl)-piperazin-1-carbonyl]-4-phenyl-pyrrolidine-1-sulfonyl}-4-methyl-thiophen-2-carbonsäuremethylester

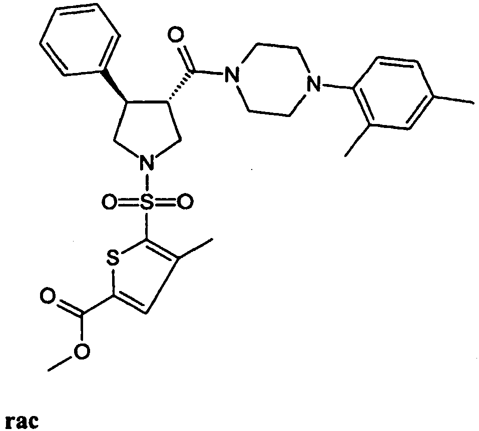

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,4-Dimethyl-phenyl)-piperazin und 5-Chlorsulfonyl-4-methyl-thiophen-2-carbonsäuremethylester 5-{3,4-trans-3-[4-(2,4-Dimethyl-phenyl)-piperazin-1-carbonyl]-4-phenyl-pyrrolidine-1-sulfonyl}-4-methyl-thiophen-2-carbonsäuremethylester.
C30H35N3O5S2 (581.76), LCMS(ESI): 582.2 (M+H⁺).

### Beispiel 41

### [1-(5-Chlor-3-methyl-benzo[b]thiophen-2-sulfonyl)-3,4-trans-4-phenyl-pyrrolidin-3-yl]-[4-(2,4-dimethyl-phenyl)-piperazin-1-yl]-methanon

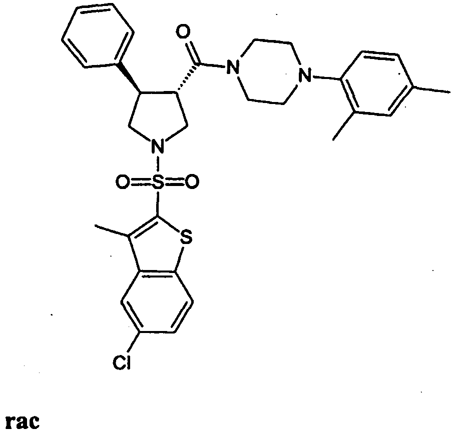

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,4-Dimethyl-phenyl)-piperazin und 5-Chlor-3-methyl-benzo[b]thiophen-2-sulfonylchlorid [1-(5-Chlor-3-methyl-benzo[b]thiophen-2-sulfonyl)-3,4-trans-4-phenyl-pyrrolidin-3-yl]-[4-(2,4-dimethyl-phenyl)-piperazin-1-yl]-methanon.
C32H34ClN3O3S2 (608.23), LCMS(ESI): 608.2 (M+H⁺).

### Beispiel 42

### [4-(3,4-Dimethyl-phenyl)-piperazin-1-yl]-[3,4-trans-4-phenyl-1-(thiophen-2-sulfonyl)-pyrrolidin-3- yl]-methanon

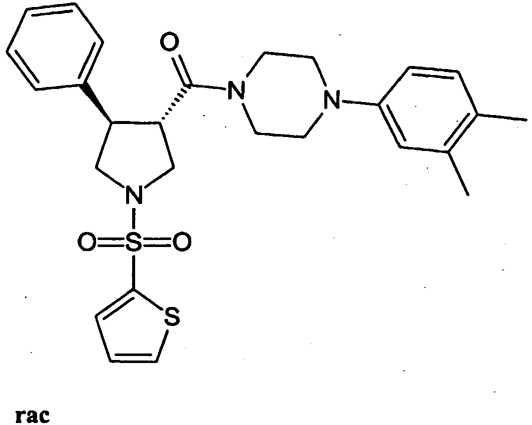

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(3,4-Dimethyl-phenyl)-piperazin und Thiophen-2-sulfonylchlorid [4-(3,4-Dimethyl-phenyl)-piperazin-1-yl]-[3,4-trans-4-phenyl-1-(thiophen-2-sulfonyl)-pyrrolidin-3-yl]-methanon.
C27H31N3O3S2 (509.69), LCMS(ESI): 510.1 (M+H⁺).

### Beispiel 43

### [1-(4,5-Dibrom-thiophen-2-sulfonyl)-3,4-trans-4-phenyl-pyrrolidin-3-yl]-[4-(3,4-dimethylphenyl)- piperazin-1-yl]-methanon

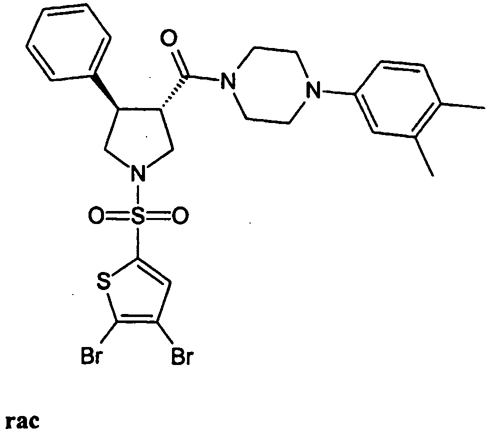

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(3,4-Dimethyl-phenyl)-piperazin und 4,5-Dibrom-thiophen-2-sulfonylchlorid [1-(4,5-Dibromthiophen-2-sulfonyl)-3,4-trans-4-phenyl-pyrrolidin-3-yl]-[4-(3,4-dimethyl-phenyl)- piperazin-1-yl]-methanon.
C27H29Br2N3O3S2 (667.49), LCMS(ESI): 668.0 (M+H⁺).

### Beispiel 44

### [1-(5-Chlor-thiophen-2-sulfonyl)-3,4-trans-4-phenyl-pyrrolidin-3-yl]-[4-(3,4-dimethyl-phenyl)-piperazin-1-yl]-methanon

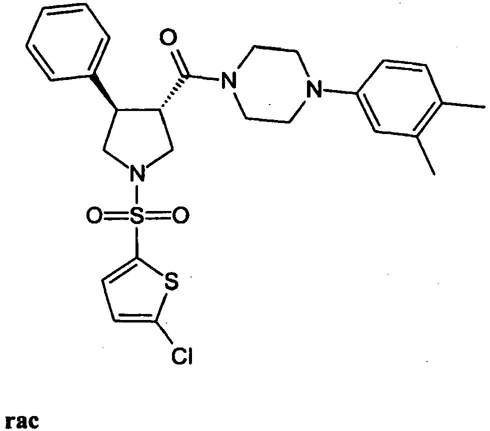

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(3,4-Dimethyl-phenyl)-piperazin und 5-Chlor-thiophen-2-sulfonylchlorid [1-(5-Chlor-thiophen-2-sulfonyl)-3,4-trans-4-phenyl-pyrrolidin-3-yl]-[4-(3,4-dimethyl-phenyl)- piperazin-1-yl]-methanon.
C27H30ClN3O3S2 (544.14), LCMS(ESI): 544.1 (M+H⁺).

### Beispiel 45

### [1-(4,5-Dichlor-thiophene-2-sulfonyl)-3,4-trans-4-phenyl-pyrrolidin-3-yl]-[4-(3,4-dimethylphenyl)- piperazin-1-yl]-methanon

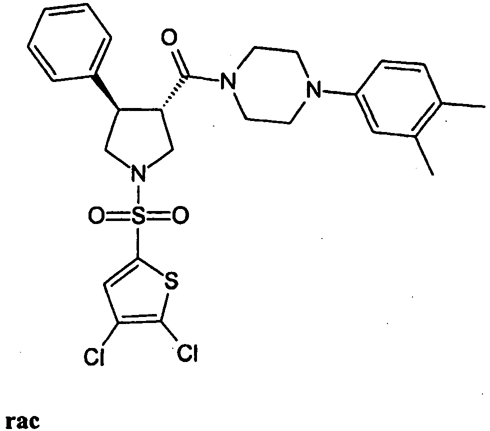

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(3,4-Dimethyl-phenyl)-piperazin und 4,5-Dichlor-thiophen-2-sulfonylchlorid [1-(4,5-Dichlorthiophene-2-sulfonyl)-3,4-trans-4-phenyl-pyrrolidin-3-yl]-[4-(3,4-dimethyl-phenyl)- piperazin-1-yl]-methanon.
C27H29Cl2N3O3S2 (578.58), LCMS(ESI): 578.1 (M+H⁺).

### Beispiel 46

### 5-{3,4-trans-3-[4-(3,4-Dimethyl-phenyl)-piperazin-1-carbonyl]-4-phenyl-pyrrolidine-1-sulfonyl}-4-methyl-thiophen-2-carbonsäuremethylester

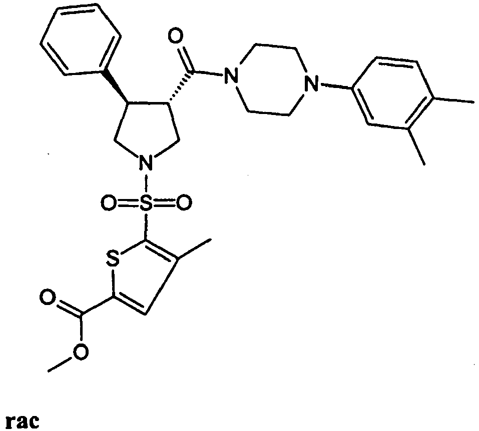

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(3,4-Dimethyl-phenyl)-piperazin und 5-Chlorsulfonyl-4-methyl-thiophen-2-carbonsäuremethylester 5-{3,4-trans-3-[4-(3,4-Dimethyl-phenyl)-piperazin-1-carbonyl]-4-phenyl-pyrrolidine-1-sulfonyl}-4-methyl-thiophen-2-carbonsäuremethylester.
C30H35N3O5S2 (581.76), LCMS(ESI): 582.2 (M+H⁺).

### Beispiel 47

### [1-(5-Chlor-3-methyl-benzo[b]thiophen-2-sulfonyl)-3,4-trans-4-phenyl-pyrrolidin-3-yl]-[4-(3,4-dimethyl-phenyl)-piperazin-1-yl]-methanon

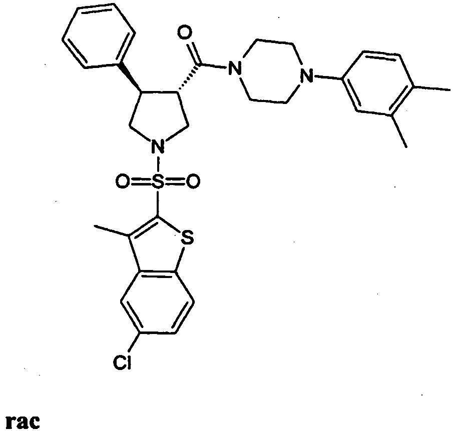

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(3,4-Dimethyl-phenyl)-piperazin und 5-Chlor-3-methyl-benzo[b]thiophen-2-sulfonylchlorid [1-(5-Chlor-3-methyl-benzo[b]thiophen-2-sulfonyl)-3,4-trans-4-phenyl-pyrrolidin-3-yl]-[4-(3,4-dimethyl-phenyl)-piperazin-1-yl]-methanon.
C32H34ClN3O3S2 (608.23), LCMS(ESI): 608.2 (M+H⁺).

### Beispiel 48

### [1-(5-Chlor-thiophen-2-sulfonyl)-3,4-trans-4-phenyl-pyrrolidin-3-yl]-[4-(2,5-dimethyl-phenyl)-piperazin-1-yl]-methanon

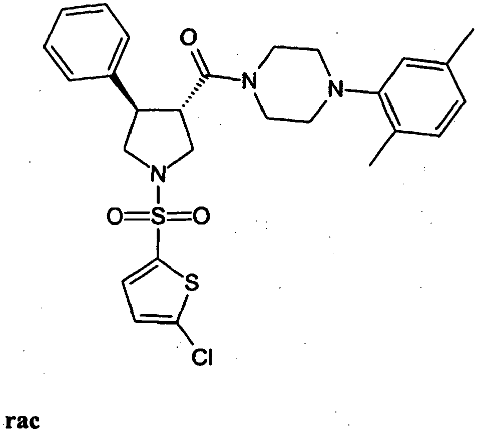

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethyl-phenyl)-piperazin und 5-Chlor-thiophen-2-sulfonylchlorid [1-(5-Chlor-thiophen-2-sulfonyl)-3,4-trans-4-phenyl-pyrrolidin-3-yl]-[4-(2,5-dimethyl-phenyl)- piperazin-1-yl]-methanon.
C27H30ClN3O3S2 (544.14), LCMS(ESI): 544.1 (M+H⁺).

### Beispiel 49

### [1-(4,5-Dichlor-thiophene-2-sulfonyl)-3,4-trans-4-phenyl-pyrrolidin-3-yl]-[4-(2,5-dimethylphenyl)- piperazin-1-yl]-methanon

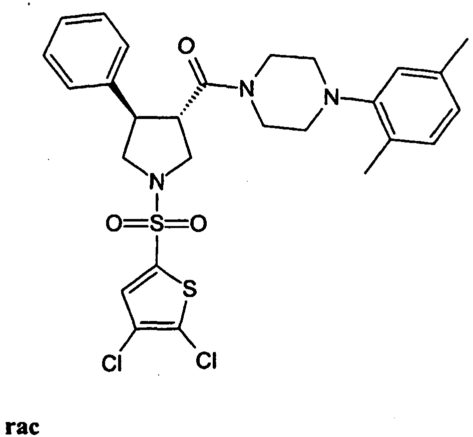

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethyl-phenyl)-piperazin und 4,5-Dichlor-thiophen-2-sulfonylchlorid [1-(4,5-Dichlorthiophene-2-sulfonyl)-3,4-trans-4-phenyl-pyrrolidin-3-yl]-[4-(2,5-dimethyl-phenyl)- piperazin-1-yl]-methanon.
C27H29Cl2N3O3S2 (578.58), LCMS(ESI): 578.1 (M+H⁺).

### Beispiel 50

### 5-{3,4-trans-3-[4-(2,5-Dimethyl-phenyl)-piperazin-1-carbonyl]-4-phenyl-pyrrolidine-1-sulfonyl}-4-methyl-thiophen-2-carbonsäuremethylester

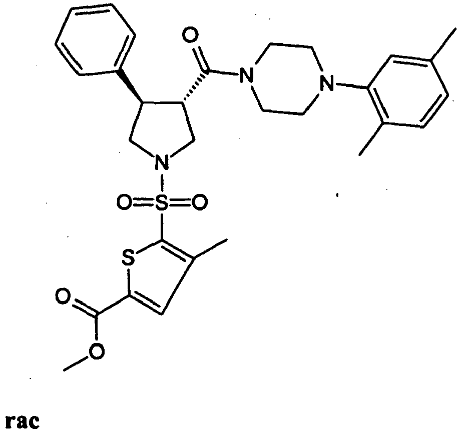

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethyl-phenyl)-piperazin und 5-Chlorsulfonyl-4-methyl-thiophen-2-carbonsäuremethylester 5-{3,4-trans-3-[4-(2,5-Dimethyl-phenyl)-piperazin-1-carbonyl]-4-phenyl-pyrrolidine-1-sulfonyl}-4-methyl-thiophen-2-carbonsäuremethylester.
C30H35N3O5S2 (581.76), LCMS(ESI): 582.2 (M+H⁺).

### Beispiel 51

### [1-(5-Chlor-3-methyl-benzo[b]thiophen-2-sulfonyl)-3,4-trans-4-phenyl-pyrrolidin-3-yl]-[4-(2,5-dimethyl-phenyl)-piperazin-1-yl]-methanon

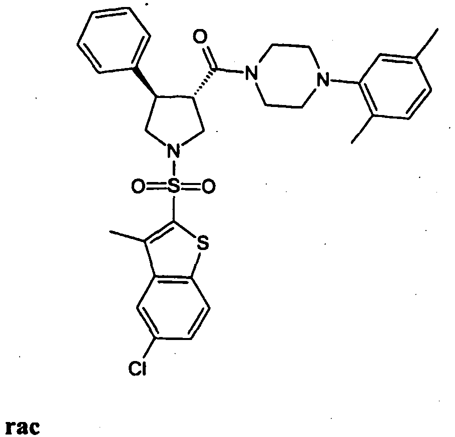

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethyl-phenyl)-piperazin und 5-Chlor-3-methyl-benzo[b]thiophen-2-sulfonylchlorid [1-(5-Chlor-3-methyl-benzo[b]thiophen-2-sulfonyl)-3,4-trans-4-phenyl-pyrrolidin-3-yl]-[4-(2,5-dimethyl-phenyl)-piperazin-1-yl]-methanon.
C32H34ClN3O3S2 (608.23), LCMS(ESI): 608.2 (M+H⁺).

### Beispiel 52

### [4-(2,3-Dimethyl-phenyl)-piperazin-1-yl]-[3,4-trans-4-phenyl-1-(thiophen-2-sulfonyl)-pyrrolidin-3- yl]-methanon

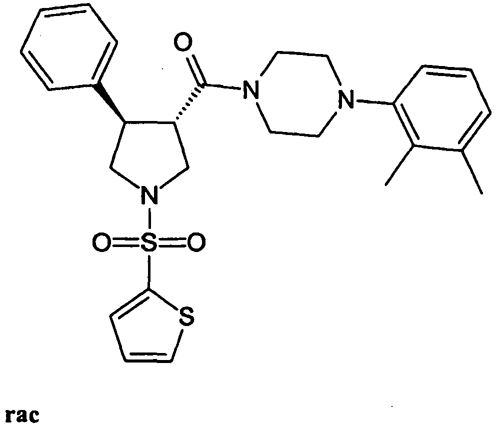

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,3-Dimethyl-phenyl)-piperazin und Thiophen-2-sulfonylchlorid [4-(2,3-Dimethyl-phenyl)-piperazin-1-yl]-[3,4-trans-4-phenyl-1-(thiophen-2-sulfonyl)-pyrrolidin-3-yl]-methanon.
C27H31N3O3S2 (509.69), LCMS(ESI): 510.2 (M+H⁺).

### Beispiel 53

### [1-(4,5-Dibrom-thiophen-2-sulfonyl)-3,4-tans-4-phenyl-pyrrolidin-3-yl]-[4-(2,3-dimethylphenyl)- piperazin-1-yl]-methanon

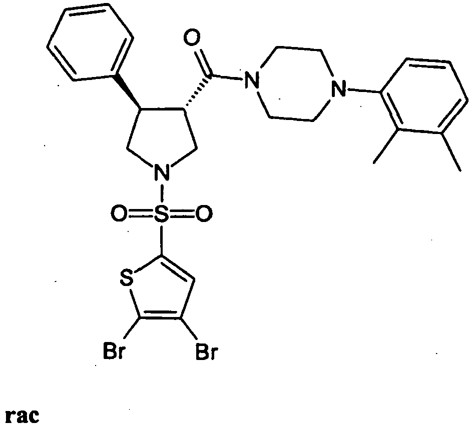

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,3-Dimethyl-phenyl)-piperazin und 4,5-Dibrom-thiophen-2-sulfonylchlorid [1-(4,5-Dibromthiophen-2-sulfonyl)-3,4-trans-4-phenyl-pyrrolidin-3-yl]-[4-(2,3-dimethyl-phenyl)-piperazin-1-yl]-methanon.
C27H29Br2N3O3S2 (667.49), LCMS(ESI): 668.1 (M+H⁺).

### Beispiel 54

### [1-(5-Chlor-thiophen-2-sulfonyl)-3,4-trans-4-phenyl-pyrrolidin-3-yl]-[4-(2,3-dimethyl-phenyl)-piperazin-1-yl]-methanon

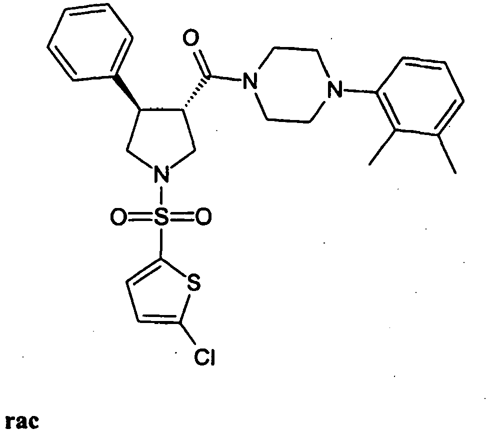

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,3-Dimethyl-phenyl)-piperazin und 5-Chlor-thiophen-2-sulfonylchlorid [1-(5-Chlor-thiophen-2-sulfonyl)-3,4-trans-4-phenyl-pyrrolidin-3-yl]-[4-(2,3-dimethyl-phenyl)- piperazin-1-yl]-methanon.
C27H30ClN3O3S2 (544.14), LCMS(ESI): 544.2 (M+H⁺).

### Beispiel 55

### [1-(4,5-Dichlor-thiophene-2-sulfonyl)-3,4-trans-4-phenyl-pyrrolidin-3-yl]-[4-(2,3-dimethylphenyl)- piperazin-1-yl]-methanon

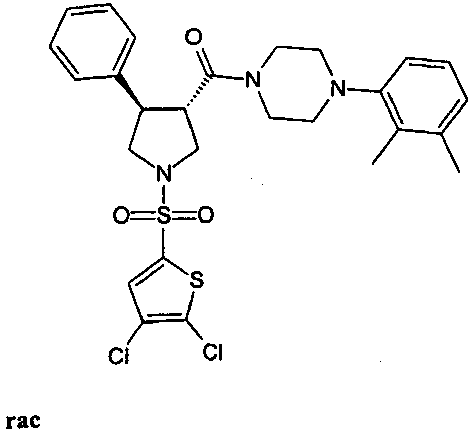

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,3-Dimethyl-phenyl)-piperazin und 4,5-Dichlor-thiophen-2-sulfonylchlorid [1-(4,5-Dichlorthiophene-2-sulfonyl)-3,4-trans-4-phenyl-pyrrolidin-3-yl]-[4-(2,3-dimethyl-phenyl)- piperazin-1-yl]-methanon.
C27H29Cl2N3O3S2 (578.58), LCMS(ESI): 578.1 (M+H⁺).

### Beispiel 56

### 5- {3,4-trans-3-[4-(2,3-Dimethyl-phenyl)-piperazin-1-carbonyl]-4-phenyl-pyrrolidine-1-sulfonyl}-4-methyl-thiophen-2-carbonsäuremethylester

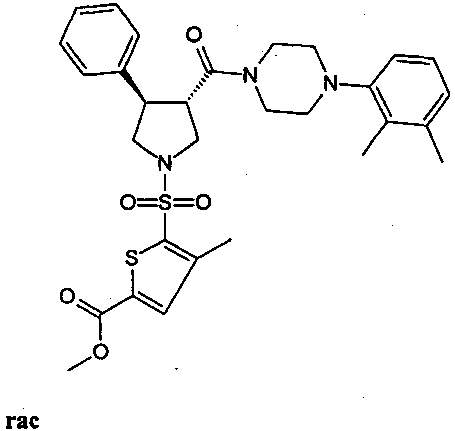

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,3-Dimethyl-phenyl)-piperazin und 5-Chlorsulfonyl-4-methyl-thiophen-2-carbonsäuremethylester 5-{3,4-trans-3-[4-(2,3-Dimethyl-phenyl)-piperazin-1-carbonyl]-4-phenyl-pyrrolidine-1-sulfonyl}-4-methyl-thiophen-2-carbonsäuremethylester.
C30H35N3O5S2 (581.76), LCMS(ESI): 582.2 (M+H⁺).

### Beispiel 57

### [1-(5-Chlor-3-methyl-benzo[b]thiophen-2-sulfonyl)-3,4-trans-4-phenyl-pyrrolidin-3-yl]-[4-(2,3-dimethyl-phenyl)-piperazin-1-yl]-methanon

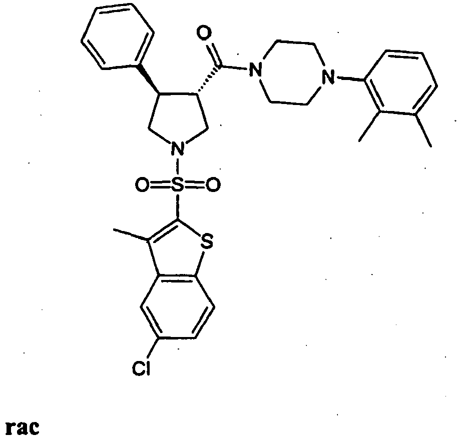

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,3-Dimethyl-phenyl)-piperazin und 5-Chlor-3-methyl-benzo[b]thiophen-2-sulfonylchlorid [1-(5-Chlor-3-methyl-benzo[b]thiophen-2-sulfonyl)-3,4-trans-4-phenyl-pyrrolidin-3-yl]-[4-(2,3-dimethyl-phenyl)-piperazin-1-yl]-methanon.
C32H34ClN3O3S2 (608.23), LCMS(ESI): 608.2 (M+H⁺).

### Beispiel 58

### [4-(4-Methoxy-phenyl)-piperazin-1-yl]-[3,4-trans-4-phenyl-1-(thiophene-2-sulfonyl)-pyrrolidin-3-yl]- methanon

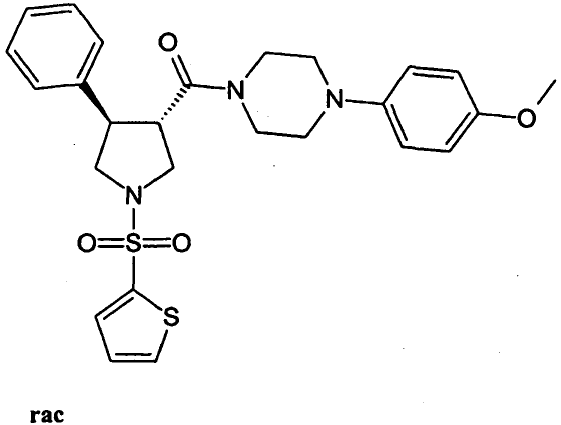

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(4-Methoxy-phenyl)-piperazin und Thiophen-2-sulfonylchlorid [4-(4-Methoxy-phenyl)-piperazin-1-yl]-[3,4-trans-4-phenyl-1-(thiophene-2-sulfonyl)-pyrrolidin-3-yl]- methanon.
C26H29N3O4S2 (511.67), LCMS(ESI): 512.1 (M+H⁺).

### Beispiel 59

### [1-(4,5-Dibrom-thiophen-2-sulfonyl)-3,4-trans-4-phenyl-pyrrolidin-3-yl]-[4-(4-methoxyphenyl)-piperazin-1-yl]-methanon

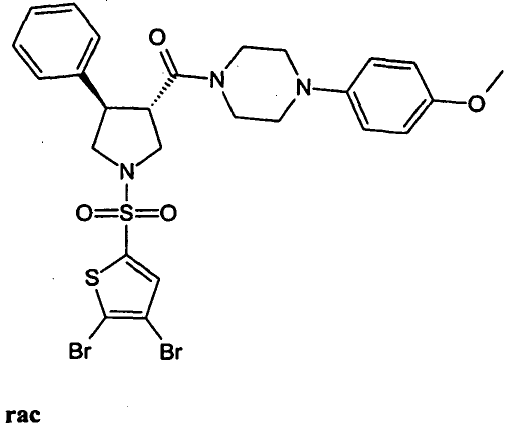

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(4-Methoxy-phenyl)-piperazin und 4,5-Dibrom-thiophen-2-sulfonylchlorid [1-(4,5-Dibromthiophen-2-sulfonyl)-3,4-trans-4-phenyl-pyrrolidin-3-yl]-[4-(4-methoxy-phenyl)-piperazin-1-yl]-methanon.
C26H27Br2N3O4S2 (669.46), LCMS(ESI): 669.9 (M+H⁺).

### Beispiel 60

### [1-(5-Chlor-thiophen-2-sulfonyl)-3,4-trans-4-phenyl-pyrrolidin-3-yl]-[4-(4-methoxy-phenyl)-piperazin-1-yl]-methanon

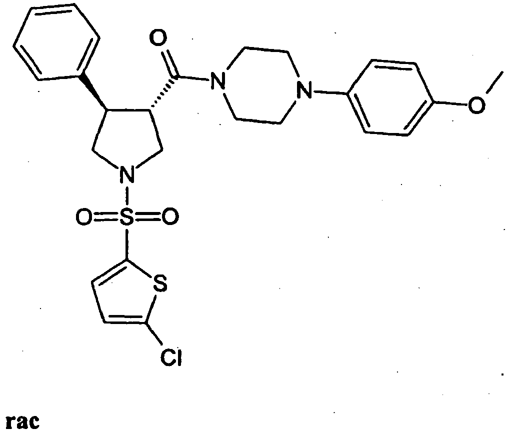

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(4-Methoxy-phenyl)-piperazin und 5-Chlor-thiophen-2-sulfonylchlorid [1-(5-Chlor-thiophen-2-sulfonyl)-3,4-trans-4-phenyl-pyrrolidin-3-yl]-[4-(4-methoxy-phenyl)- piperazin-1-yl]-methanon.
C26H28ClN3O4S2 (546.11), LCMS(ESI): 546.1 (M+H⁺).

### Beispiel 61

### [1-(4,5-Dichlor-thiophen-2-sulfonyl)-3,4-trans-4-phenyl-pyrrolidin-3-yl]-[4-(4-methoxyphenyl)- piperazin-1-yl]-methanon

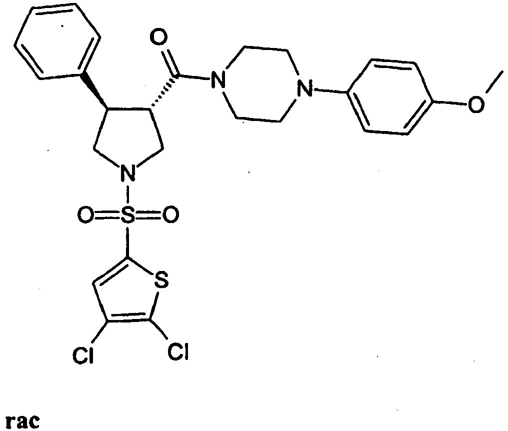

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(4-Methoxy-phenyl)-piperazin und 4,5-Dichlor-thiophen-2-sulfonylchlorid [1-(4,5-Dichlorthiophen-2-sulfonyl)-3,4-trans-4-phenyl-pyrrolidin-3-yl]-[4-(4-methoxy-phenyl)- piperazin-1-yl]-methanon.
C26H27Cl2N3O4S2 (580.56), LCMS(ESI): 580.0 (M+H⁺).

### Beispiel 62

### [5-{3,4-trans-3-[4-(4-Methoxy-phenyl)-piperazin-1-carbonyl]-4-phenyl-pyrrolidin-1-sulfonyl}-4- methyl-thiophen-2-carbonsäuremethylester

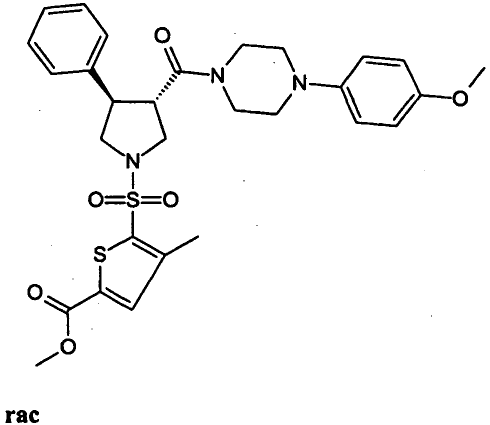

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(4-Methoxy-phenyl)-piperazin und 5-Chlorsulfonyl-4-methyl-thiophen-2-carbonsäuremethylester [5-{3,4-trans-3-[4-(4-Methoxy-phenyl)-piperazin-1-carbonyl]-4-phenyl-pyrrolidin-1-sulfonyl}-4- methyl-thiophen-2-carbonsäuremethylester.
C29H33N3O6S2 (583.73), LCMS(ESI): 584.1 (M+H⁺).

### Beispiel 63

### [1-(5-Chlor-3-methyl-benzo[b]thiophen-2-sulfonyl)-3,4-trans-4-phenyl-pyrrolidin-3-yl]-[4-(4-methoxy-phenyl)-piperazin-1-yl]-methanon

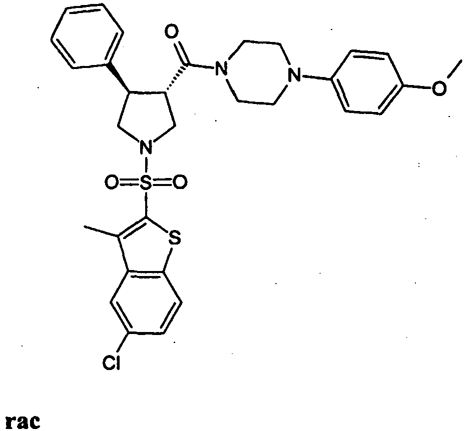

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(4-Methoxy-phenyl)-piperazin und 5-Chlor-3-methyl-benzo[b]thiophen-2-sulfonylchlorid [1-(5-Chlor-3-methyl-benzo[b]thiophen-2-sulfonyl)-3,4-trans-4-phenyl-pyrrolidin-3-yl]-[4-(4-methoxy-phenyl)-piperazin-1-yl]-methanon.
C31H32ClN3O4S2 (610.20), LCMS(ESI): 610.1 (M+H⁺).

### Beispiel 64

### [1-(4,5-Dibrom-thiophen-2-sulfonyl)-3,4-trans-4-phenyl-pyrrolidin-3-yl]-(4-phenyl-piperazin-1-yl)- methanon

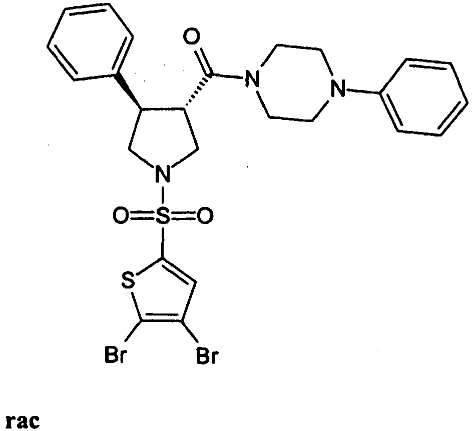

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-Phenylpiperazin und 4,5-Dibrom-thiophen-2-sulfonylchlorid [1-(4,5-Dibrom-thiophen-2-sulfonyl)-3,4-trans-4-phenyl-pyrrolidin-3-yl]-(4-phenyl-piperazin-1-yl)- methanon.
C25H25Br2N3O3S2 (639.43), LCMS(ESI): 640.0 (M+H⁺).

### Beispiel 65

### 2-{4-[3,4-trans-4-Phenyl-1-(thiophen-2-sulfonyl)-pyrrolidin-3-carbonyl]-piperazin-1-yl}-terephthalsäuredimethylester-trifluoracetat

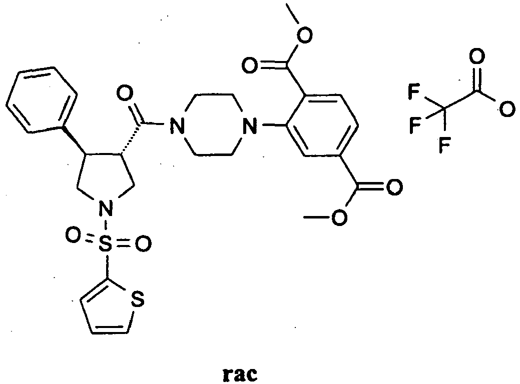

Analog zu Beispiel 1 erhält man aus 2-Piperazin-1-yl-terephthalsäuredimethylester und den kommerziell erhältlichen Reagenzien (E)-Zimtsäuremethylester, N-(Methoxymethyl)-N-(trimethylsilylmethyl)-benzylamin und Thiophen-2-sulfonylchlorid 2-{4-[3,4-trans-4-Phenyl-1-(thiophen-2-sulfonyl)-pyrrolidin-3-carbonyl]-piperazin-1-yl}- terephthalsäuredimethylestertrifluoracetat.
C29H31N3O7S2. C2HF302 (711.73), LCMS(ESI): 597.9 (M+H⁺).

### Beispiel 66

### [4-(2,5-Dichlor-phenyl)-piperazin-1-yl]-[3,4-trans-4-phenyl-1-(thiophen-2-sulfonyl)-pyrrolidin-3-yl]-methanon-trifluoracetat

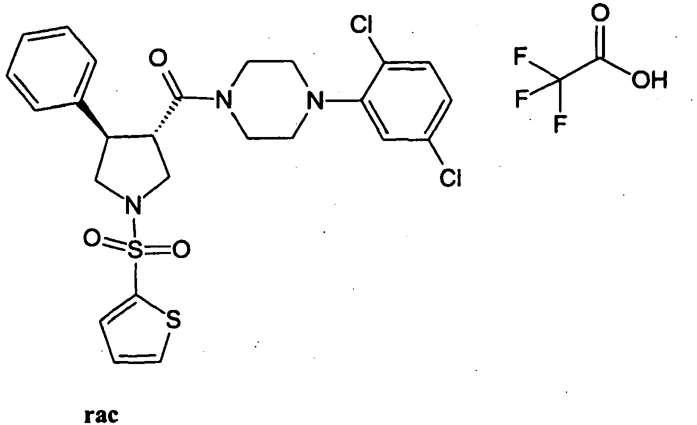

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dichlor-phenyl)-piperazin und Thiophen-2-sulfonylchlorid [4-(2,5-Dichlor-phenyl)-piperazin-1-yl]-[3,4-trans-4-phenyl-1-(thiophen-2-sulfonyl)-pyrrolidin-3-yl]-methanon-trifluoracetat.
C25H25Cl2N3O3S2. C2HF3O2 (664.55), LCMS(ESI): 550.0 (M+H⁺).

### Beispiel 67

### [4-(2,5-Dimethyl-phenyl)-piperazin-1-yl]-[3,4-trans-4-phenyl-1-(thiophen-2-sulfonyl)-pyrrolidin-3-yl]-methanon

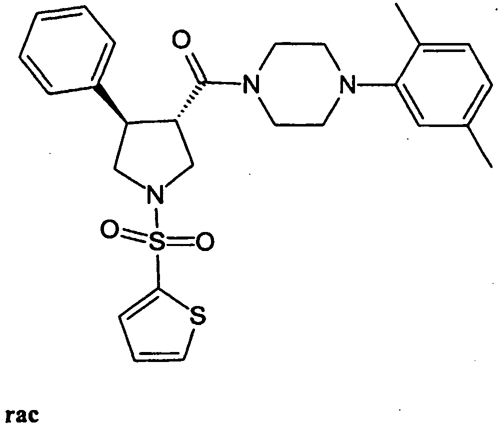

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethyl-phenyl)-piperazin und Thiophen-2-sulfonylchlorid [4-(2,5-Dimethyl-phenyl)-piperazin-1-yl]-[3,4-trans-4-phenyl-1-(thiophen-2-sulfonyl)-pyrrolidin-3-yl]-methanon.
C27H31N3O3S2 (509.69), LCMS(ESI): 510.3 (M+H⁺).

Das Racemat [4-(2,5-Dimethyl-phenyl)-piperazin-1-yl]-[3,4-trans-4-phenyl-1-(thiophen-2-sulfonyl)-pyrrolidin-3-yl]-methanon wurde durch Chromatographie an chiraler Phase in die Enantiomere aufgetrennt. Man erhält [4-(2,5-Dimethyl-phenyl)-piperazin-1-yl]-[(3S,4R)/(3R,4S)-4-phenyl-1-(thiophene-2-sulfonyl)-pyrrolidin-3-yl]-methanon Beispiel **67A** (Chiracel OJ/37 250x4.6 mm, Eluent Methanol, Rt = 8.4 min) und [4-(2,5-Dimethyl-phenyl)-piperazin-1-yl]-[(3R,4S)/(3S,4R)-4-phenyl-1-(thiophene-2-sulfonyl)-pyrrolidin-3-yl]-methanon Beispiel **67B** (Chiracel OJ/37 250x4.6 mm, Eluent Methanol, Rt =10.3 min).

### Beispiel 68

### [1-(Benzo[1,2,5]oxadiazol-4-sulfonyl)-3,4-trans-4-phenyl-pyrrolidin-3-yl]-[4-(2,5-dimethylphenyl)- piperazin-1-yl]-methanon-trifluoracetat

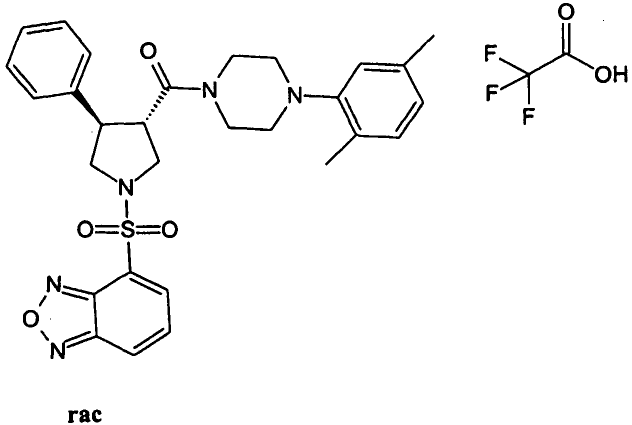

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethyl-phenyl)-piperazin und Benzo[1,2,5]oxadiazol-4-sulfonylchlorid [1-(Benzo[1,2,5]oxadiazol-4-sulfonyl)-3,4-trans-4-phenyl-pyrrolidin-3-yl]-[4-(2,5-dimethylphenyl)- piperazin-1-yl]-methanon-trifluoracetat.
C29H31N5O4S. C2HF302 (659.69), LCMS(ESI): 546.1 (M+H⁺).

### Beispiel 69

### [4-(2,5-Dimethyl-phenyl)-piperazin-1-yl]-[1-(3-methoxy-benzolsulfonyl)-3,4-trans-4-phenylpyrrolidin-3-yl]-methanon

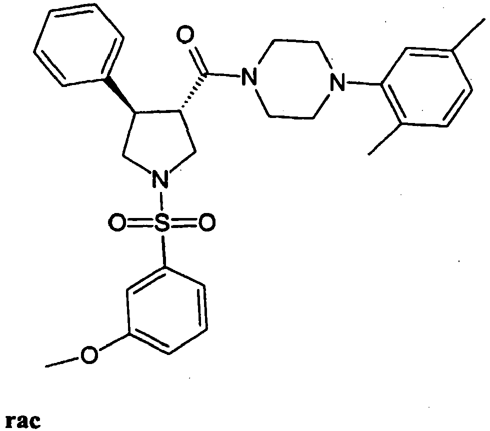

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethyl-phenyl)-piperazin und 3-Methoxy-benzolsulfonylchlorid [4-(2,5-Dimethyl-phenyl)-piperazin-1-yl]-[1-(3-methoxy-benzolsulfonyl)-3,4-trans-4-phenyl-pyrrolidin-3-yl]-methanon.
C30H35N3O4S (533.70), LCMS(ESI): 534.2 (M+H⁺).

Das Racemat [4-(2,5-Dimethyl-phenyl)-piperazin-1-yl]-[1-(3-methoxy-benzolsulfonyl)-3,4-trans-4-phenyl- pyrrolidin-3-yl]-methanon wurde durch Chromatographie an chiraler Phase in die Enantiomere aufgetrennt. Man erhält [4-(2,5-Dimethyl-phenyl)-piperazin-1-yl]-[1-(3-methoxy-benzolsulfonyl)- (3S,4R)/(3R,4S) -4-phenyl- pyrrolidin-3-yl]-methanon Beispiel **69A** (Chirapak AD-22 250x4.6 mm , Eluent Ethanol:Methanol =1:1, Rt = 7.6 min) und [4-(2,5-Dimethyl-phenyl)-piperazin-1-yl]-[1-(3-methoxy-benzolsulfonyl)- (3R,4S)/(3S,4R)-4-phenylpyrrolidin-3-yl]-methanon Beispiel **69B** (Chirapak AD-22 250x4.6 mm , Eluent Ethanol:Methanol = 1:1, Rt = 13.6 min).

### Beispiel 70

### [4-(2,5-Dimethyl-phenyl)-piperazin-1-yl]-[3,4-rans-4-phenyl-1-(propan-2-sulfonyl)-pyrrolidin-3-yl]-methanon-trifluoracetat

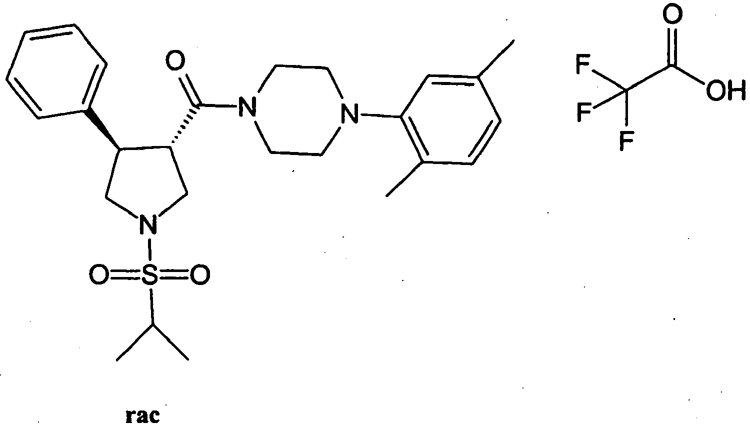

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethyl-phenyl)-piperazin und Isopropylsulfonylchlorid [4-(2,5-Dimethyl-phenyl)-piperazin-1-yl]-[3,4-rans-4-phenyl-1-(propan-2-sulfonyl)-pyrrolidin-3-yl]-methanon-trifluoracetat.
C26H35N3O3S. C2HF302 (583.68), LCMS(ESI): 470.2 (M+H⁺).

### Beispiel 71

### [1-(3,5-Dimethyl-isoxazol-4-sulfonyl)-3,4-trans-4-phenyl-pyrrolidin-3-yl]-[4-(2,5-dimethylphenyl)- piperazin-1-yl]-methanon

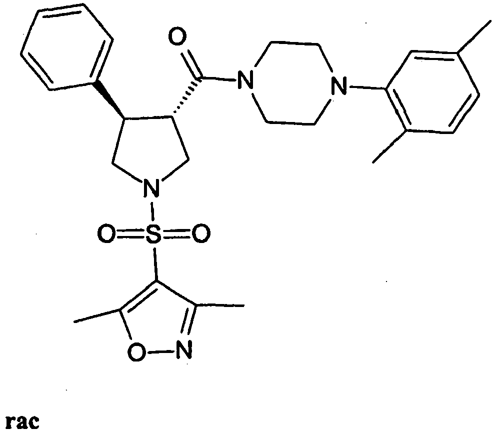

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethyl-phenyl)-piperazin und 3,5-Dimethyl-isoxazol-4-sulfonylchlorid [1-(3,5-Dimethylisoxazol-4-sulfonyl)-3,4-trans-4-phenyl-pyrrolidin-3-yl]-[4-(2,5-dimethyl-phenyl)- piperazin-1-yl]-methanon.
C28H34N4O4S (522.67), LCMS(ESI): 523.3 (M+H⁺).

Das Racemat [1-(3,5-Dimethyl-isoxazol-4-sulfonyl)-3,4-trans-4-phenyl-pyrrolidin-3-yl]-[4-(2,5-dimethyl-phenyl)- piperazin-1-yl]-methanon wurde durch Chromatographie an chiraler Phase in die Enantiomere aufgetrennt. Man erhält [1-(3,5-Dimethyl-isoxazol-4-sulfonyl)-(3S,4R)/(3R,4S)-4-phenyl-pyrrolidin-3-yl]-[4-(2,5-dimethyl-phenyl)- piperazin-1-yl]-methanon Beispiel **71A** (Chirapak AD-22 250x4.6 mm, Eluent Ethanol:Methanol = 1:1, Rt = 6.5 min) und [1-(3,5-Dimethyl-isoxazol-4-sulfonyl)- (3R,4S)/(3S,4R) -4-phenyl-pyrrolidin-3-yl]-[4-(2,5-dimethyl-phenyl)- piperazin-1-yl]-methanon Beispiel **71B** (Chirapak AD-22 250x4.6 mm, Eluent Ethanol:Methanol =1:1, Rt = 9.1 min).

### Beispiel 72

### [4-(2,5-Dimethyl-phenyl)-piperazin-1-yl]-[3,4-trans-4-phenyl-1-(pyridin-3-sulfonyl)-pyrrolidin-3-yl]-methanon-trifluoracetat

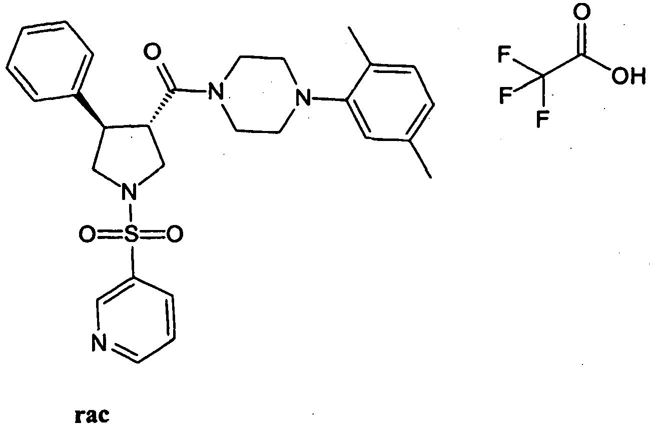

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethyl-phenyl)-piperazin und Pyridin-3-sulfonylchlorid [4-(2,5-Dimethyl-phenyl)-piperazin-1-yl]-[3,4-trans-4-phenyl-1-(pyridin-3-sulfonyl)-pyrrolidin-3-yl]-methanontrifluoracetat.
C28H32N4O3S.2C2HF3O2 (732.70), LCMS(ESI): 505.1 (M+H⁺).

### Beispiel 73

### [4-(2,5-Dichlor-phenyl)-piperazin-1-yl]-[3,4-trans-4-phenyl-1-(pyridin-3-sulfonyl)-pyrrolidin-3-yl]-methanon-trifluoracetat

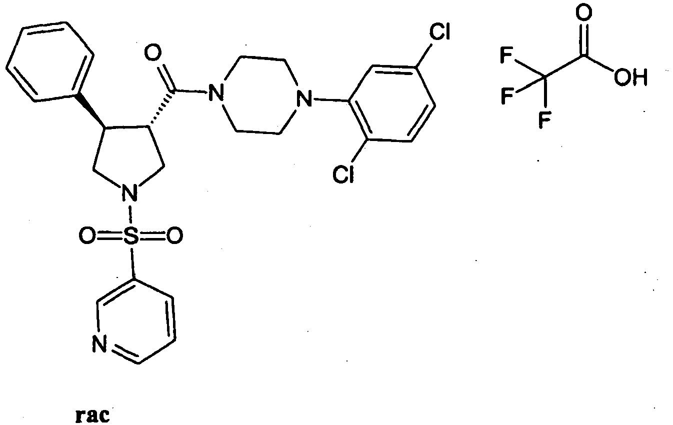

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dichlor-phenyl)-piperazin und Pyridin-3-sulfonylchlorid [4-(2,5-Dichlor-phenyl)-piperazin-1-yl]-[3,4-trans-4-phenyl-1-(pyridin-3-sulfonyl)-pyrrolidin-3-yl]-methanon-trifluoracetat.
C26H26Cl2N4O3S.2C2HF3O2 (773.54), LCMS(ESI): 545.0 (M+H⁺).

### Beispiel 74

### [4-(2,5-Dichlor-phenyl)-piperazin-1-yl]-[3,4-trans-4-phenyl-1-(toluene-3-sulfonyl)-pyrrolidin-3- yl]-methanon-trifluoracetat

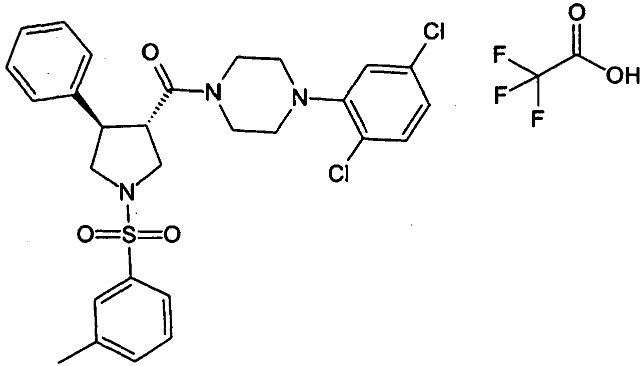

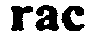

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dichlor-phenyl)-piperazin und 3-Methyl-benzolsulfonylchlorid [4-(2,5-Dichlor-phenyl)-piperazin-1-yl]-[3,4-trans-4-phenyl-1-(toluene-3-sulfonyl)-pyrrolidin-3- yl]-methanontrifluoracetat.
C28H29Cl2N3O3S.C2HF3O2 (672.56), LCMS(ESI): 558.2 (M+H⁺).

### Beispiel 75

### [4-(2,5-Dichlor-phenyl)-piperazin-1-yl]-[1-(4-methoxy-benzolsulfonyl)-3,4-trans-4-phenylpyrrolidin-3-yl]-methanon-trifluoracetat

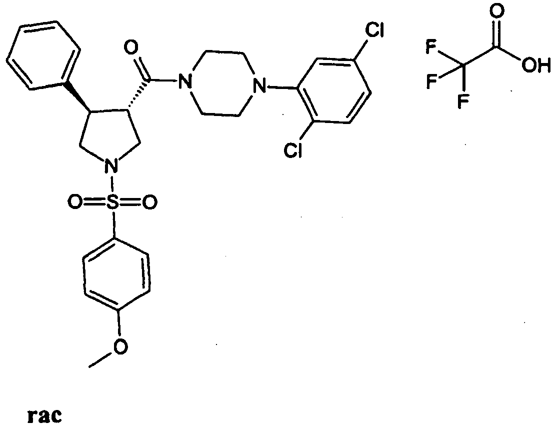

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dichlor-phenyl)-piperazin und 4-Methoxy-benzolsulfonylchlorid [4-(2,5-Dichlor-phenyl)-piperazin-1-yl]-[1-(4-methoxy-benzolsulfonyl)-3,4-trans-4-phenyl-pyrrolidin-3-yl]-methanontrifluoracetat.
C28H29Cl2N3O4S.C2HF3O2 (688.56), LCMS(ESI): 574.2 (M+H⁺).

### Beispiel 76

### [4-(2,5-Dimethoxy-phenyl)-piperazin-1-yl]-[3,4-trans4-phenyl-1-(2,2,2-trifluoroethansulfonyl)- pyrrolidin-3-yl]-methanon-trifluoracetat

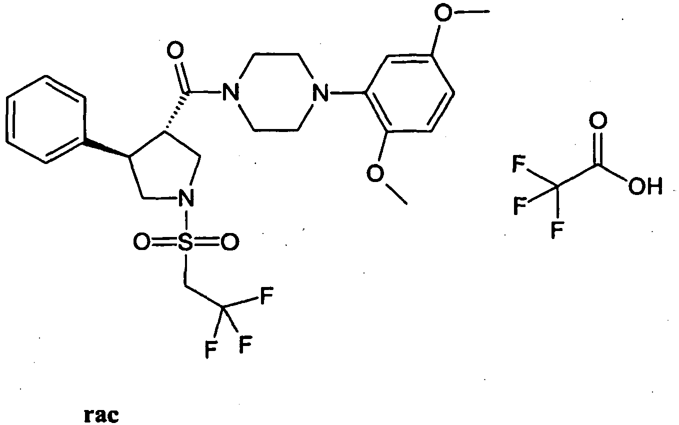

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethoxy-phenyl)-piperazin und 2,2,2-Trifluor-ethansulfonyl chlorid [4-(2,5-Dimethoxyphenyl)-piperazin-1-yl]-[3,4-trans4-phenyl-1-(2,2,2-trifluor-ethansulfonyl)- pyrrolidin-3-yl]-methanon-trifluoracetat.
C25H30F3N3O5S.C2HF302 (655.62), LCMS(ESI): 542.3 (M+H⁺).

### Beispiel 77

### [4-(2,5-Dimethoxy-phenyl)-piperazin-1-yl]-[3,4-trans-4-phenyl-1-(pyridin-3-sulfonyl)-pyrrolidin-3-yl]-methanon-trifluoracetat

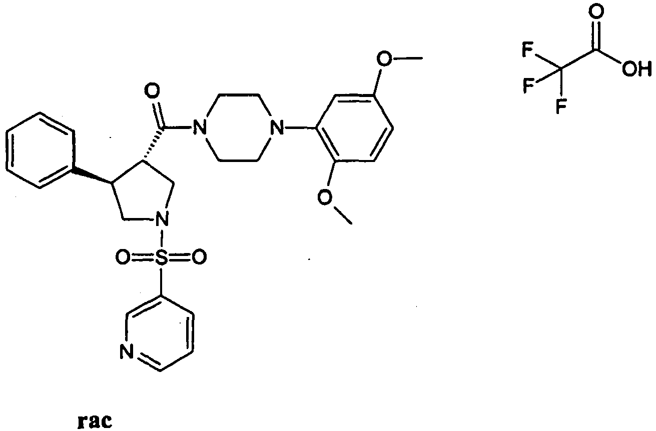

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethoxy-phenyl)-piperazin und Pyridin-3-sulfonylchlorid [4-(2,5-Dimethoxy-phenyl)-piperazin-1-yl]-[3,4-trans-4-phenyl-1-(pyridin-3-sulfonyl)-pyrrolidin-3-yl]-methanontrifluoracetat.
C28H32N4O5S.2C2HF3O2 (764.70), LCMS(ESI): 537.3 (M+H⁺).

### Beispiel 78

### [4-(2,5-Dimethoxy-phenyl)-piperazin-1-yl]-[3,4-trans-4-phenyl-1-(propan-2-sulfonyl)-pyrrolidin-3-yl]-methanon-trifluoracetat

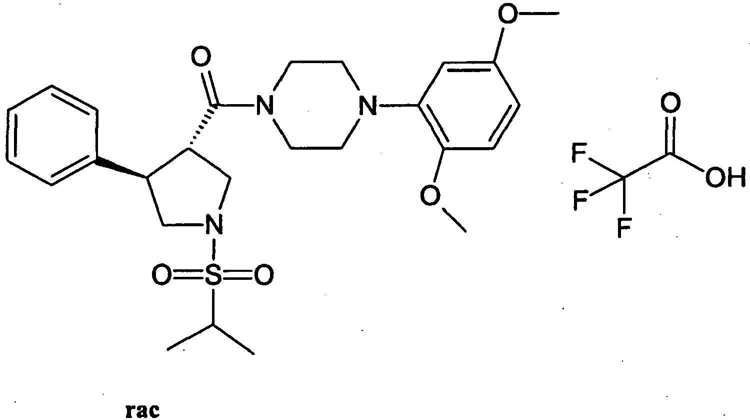

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethoxy-phenyl)-piperazin und Isopropylsulfonylchlorid [4-(2,5-Dimethoxy-phenyl)-piperazin-1-yl]-[3,4-trans-4-phenyl-1-(propan-2-sulfonyl)-pyrrolidin-3-yl]-methanontrifluoracetat.
C26H35N3O5S.C2HF302 (615.67), LCMS(ESI): 502.3 (M+H⁺).

### Beispiel 79

### [4-(2,5-Dimethoxy-phenyl)-piperazin-1-yl]-[1-(4-methoxy-benzolsulfonyl)-3,4-trans-4-phenylpyrrolidin-3-yl]-methanon-trifluoracetat

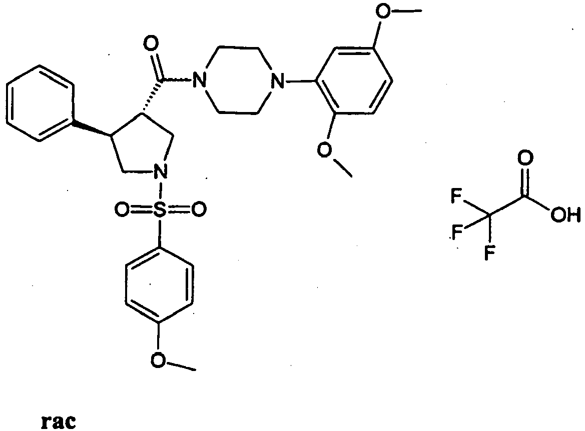

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethoxy-phenyl)-piperazin und 4-Methoxy-benzolsulfonylchlorid [4-(2,5-Dimethoxyphenyl)-piperazin-1-yl]-[1-(4-methoxy-benzolsulfonyl)-3,4-trans-4-phenyl- pyrrolidin-3-yl]-methanon-trifluoracetat.
C30H35N3O6S.C2HF302 (679.72), LCMS(ESI): 566.0 (M+H⁺).

### Beispiel 80

### 4-(2,5-Dimethoxy-phenyl)-piperazin-1-yl]-[3,4-rans-4-phenyl-1-(toluene-3-sulfonyl)-pyrrolidin-3- yl]-methanon-trifluoracetat

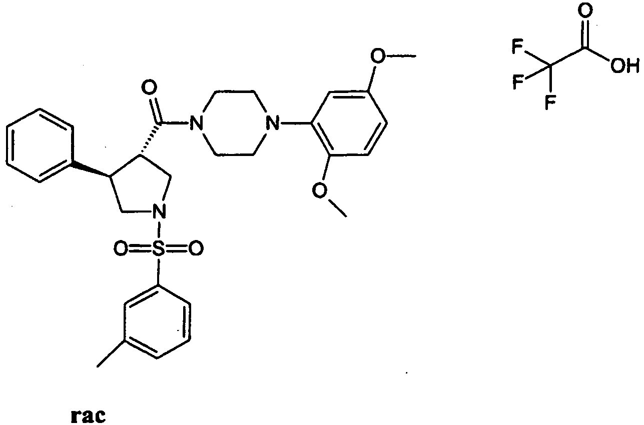

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethoxy-phenyl)-piperazin und 3-Methyl-benzolsulfonylchlorid [4-(2,5-Dimethoxyphenyl)-piperazin-1-yl]-[3,4-trans-4-phenyl-1-(toluene-3-sulfonyl)-pyrrolidin-3- yl]-methanontrifluoracetat.
C30H35N3O5S.C2HF3O2 (663.72), LCMS(ESI): 550.0 (M+H⁺).

### Beispiel 81

### [1-(3,5-Dimethoxy-isoxazol-4-sulfonyl)-3,4-trans-4-phenyl-pyrrolidin-3-yl]-[4-(2,5-dimethylphenyl)- piperazin-1-yl]-methanon-trifluoracetat

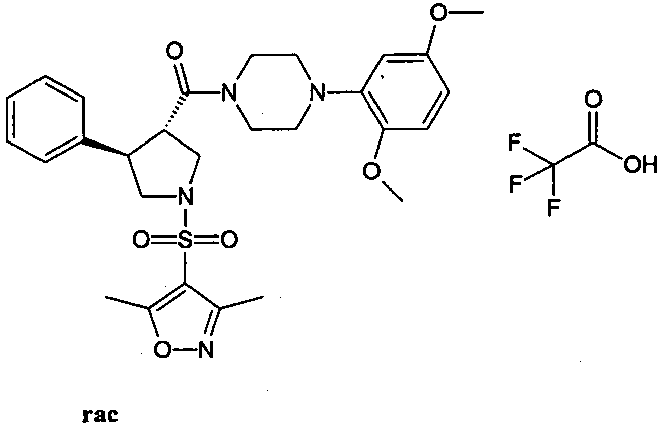

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethoxy-phenyl)-piperazin und 3,5-Dimethyl-isoxazol-4-sulfonylchlorid [1-(3,5-Dimethoxy-isoxazol-4-sulfonyl)-3,4-trans-4-phenyl-pyrrolidin-3-yl]-[4-(2,5-dimethyl-phenyl)-piperazin-1-yl]-methanon-trifluoracetat.
C28H34N4O6S.C2HF3O2 (668.69), LCMS(ESI): 555.0 (M+H⁺).

### Beispiel 82

### [4-(2,5- Dimethoxy -phenyl)-piperazin-1-yl]-[1-(3-methoxy-benzolsulfonyl)-3,4-trans-4-phenyl- pyrrolidin-3-yl]-methanon- trifluoracetat

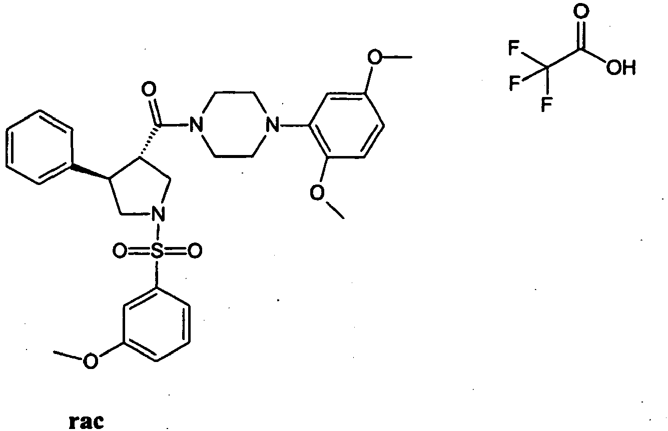

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethoxy-phenyl)-piperazin und 3-Methoxy-benzolsulfonylchlorid [4-(2,5- Dimethoxy - phenyl)-piperazin-1-yl]-[1-(3-methoxy-benzolsulfonyl)-3,4-trans-4-phenyl- pyrrolidin-3-yl]-methanon- trifluoracetat.
C30H35N3O6S.C2BF3O2 (679.72), LCMS(ESI): 566.0 (M+H⁺).

### Beispiel 83

### [1-(Benzo[1,2,5]oxadiazol-4-sulfonyl)-3,4-trans-4-phenyl-pyrrolidin-3-yl]-[4-(2,5- Dimethoxy - phenyl)-piperazin-1-yl]-methanon-trifluoracetat

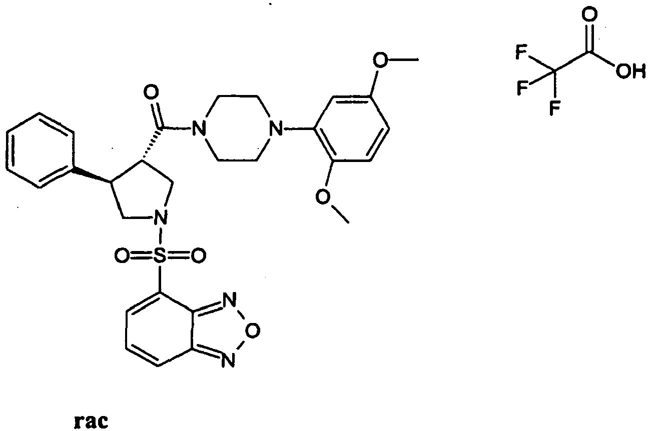

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethoxy -phenyl)-piperazin und Benzo[1,2,5]oxadiazol-4-sulfonylchlorid [1-(Benzo[1,2,5]oxadiazol-4-sulfonyl)-3,4-trans-4-phenyl-pyrrolidin-3-yl]-[4-(2,5- Dimethoxy - phenyl)- piperazin-1-yl]-methanon-trifluoracetat.
C29H31N5O6S.C2HF3O2 (691.69), LCMS(ESI): 578.0 (M+H⁺).

### Beispiel 84

### [4-(2,5- Dimethoxy -phenyl)-piperazin-1-yl]-[3,4-trans-4-phenyl-1-(thiophen-2-sulfonyl)-pyrrolidin-3-yl]-methanon- trifluoracetat

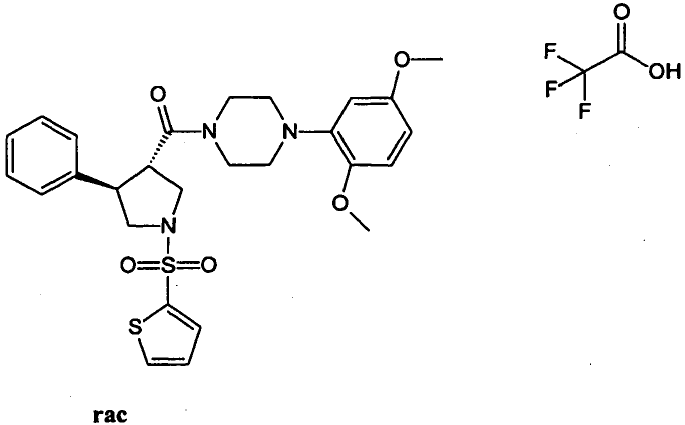

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dimethoxy -phenyl)-piperazin und Thiophen-2-sulfonylchlorid [4-(2,5- Dimethoxy -phenyl)-piperazin-1-yl]-[3,4-trans-4-phenyl-1-(thiophen-2-sulfonyl)-pyrrolidin-3-yl]-methanontrifluoracetat.
C27H31N3O5S2.C2HF3O2 (655.72), LCMS(ESI): 542.0 (M+H⁺).

### Beispiel 85

### [[4-(2,5-Dichlor-phenyl)-piperazin-1-yl]-[3,4-trans-4-phenyl-1-(toluen-2-sulfonyl)-pyrrolidin-3-yl]-methanon-trifluoracetat

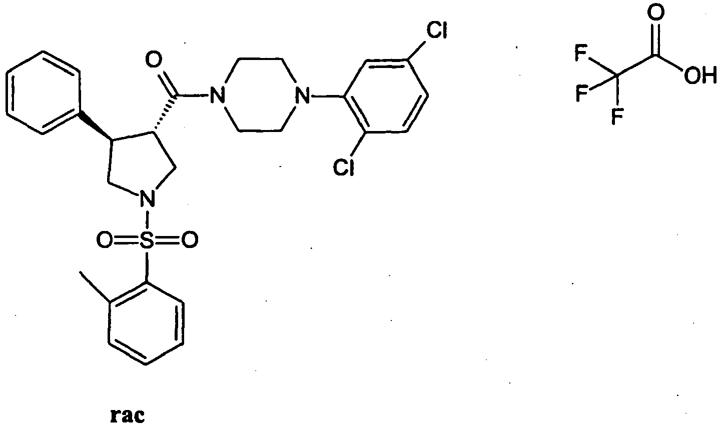

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,5-Dichlor-phenyl)-piperazin und 2-Methyl-benzolsulfonylchlorid [[4-(2,5-Dichlor-phenyl)-piperazin-1-yl]-[3,4-trans-4-phenyl-1-(toluen-2-sulfonyl)-pyrrolidin-3-yl]-methanontrifluoracetat.
28H29C12N3O3S.C2HF3O2 (672.56), LCMS(ESI): 558.1 (M+H⁺).

### Beispiel 86

### [4-(2,6- Dimethoxy -phenyl)-piperazin-1-yl]-[1-(3-methoxy-benzolsulfonyl)-3,4-trans-4-phenyl- pyrrolidin-3-yl]-methanon

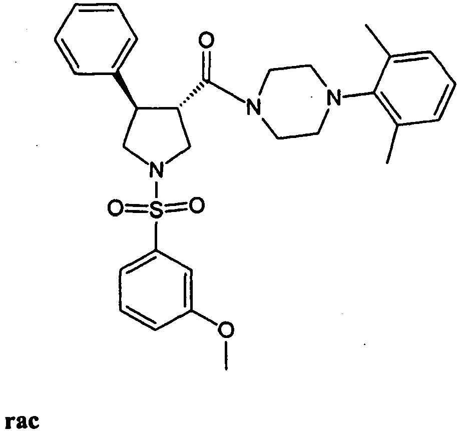

Analog zu Beispiel 1 erhält man aus den kommerziell erhältlichen Reagenzien (E)-Zimtsäuremethylester, N-(Methoxymethyl)-N-(trimethylsilylmethyl)-benzylamin, 1-(2,6-Dimethoxy-phenyl)-piperazin und 3-Methoxy-benzolsulfonylchlorid [4-(2,6- Dimethoxy - phenyl)-piperazin-1-yl]-[1-(3-methoxy-benzolsulfonyl)-3,4-trans-4-phenyl- pyrrolidin-3-yl]-methanon.
C30H35N3O4S (533.70), LCMS(ESI): 534.4 (M+H⁺).

### Beispiel 87

### [3,4-trans-4-Benzo[1,3]dioxol-5-yl-1-(3,5-dimethyl-isoxazol-4-sulfonyl)-pyrrolidin-3-yl]-[4-(2,5-dimethyl-phenyl)-piperazin-1-yl]-methanon-trifluoracetat

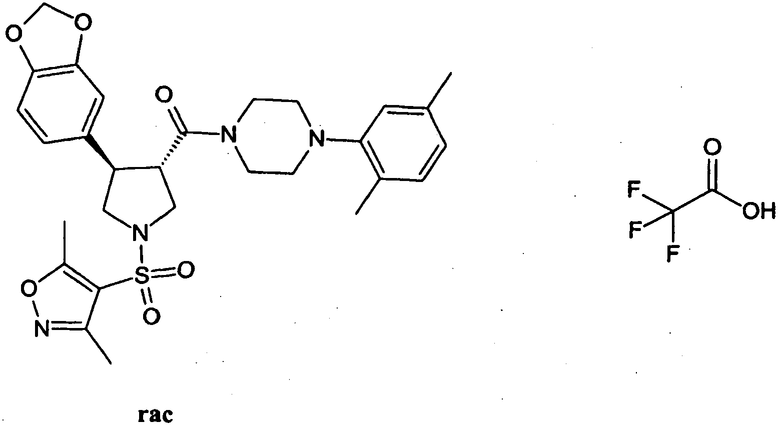

Analog zu Beispiel 1 erhält man aus den kommerziell erhältlichen Reagenzien Benzo[1,3]dioxol-5-carbaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3,5-Dimethyl-isoxazol-4-sulfonylchlorid [3,4-trans-4-Benzo[1,3]dioxol-5-yl-1-(3,5-dimethylisoxazol-4-sulfonyl)-pyrrolidin-3-yl]-[4-(2,5-dimethyl-phenyl)-piperazin-1-yl]-methanontrifluoracetat.
C29H34N4O6S.C2HF3O2 (680.71), LCMS(ESI): 567.3 (M+H⁺).

### Beispiel 88

### [3,4-trans-4-Benzo[1,3]dioxol-5-yl-1-(3-methoxy-benzolsulfonyl)-pyrrolidin-3-yl]-[4-(2,5-dimethyl-phenyl)-piperazin-1-yl]-methanon -trifluoracetat

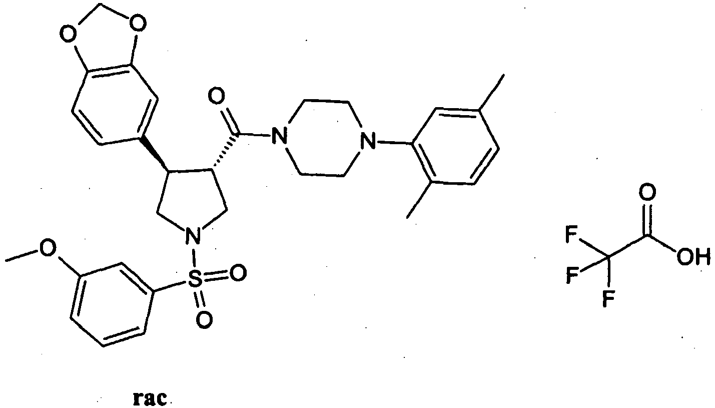

Analog zu Beispiel 1 erhält man aus den kommerziell erhältlichen Reagenzien Benzo[1,3]dioxol-5-carbaldehyd, Methyl(triphenylphosphoranyliden)acetat, N-(Methoxymethyl)-N-(trimethylsilyhnethyl)-benzylamin, 1-(2,5-Dimethyl-phenyl)-piperazin und 3-Methoxy-benzolsulfonylchlorid [3,4-trans-4-Benzo[1,3]dioxol-5-yl-1-(3-methoxybenzolsulfonyl)-pyrrolidin-3-yl]-[4-(2,5-dimethyl-phenyl)-piperazin-1-yl]-methanon - trifluoracetat.
C31H35N3O6S.C2HF3O2 (691.73), LCMS(ESI): 578.4 (M+H⁺).

### Beispiel 89

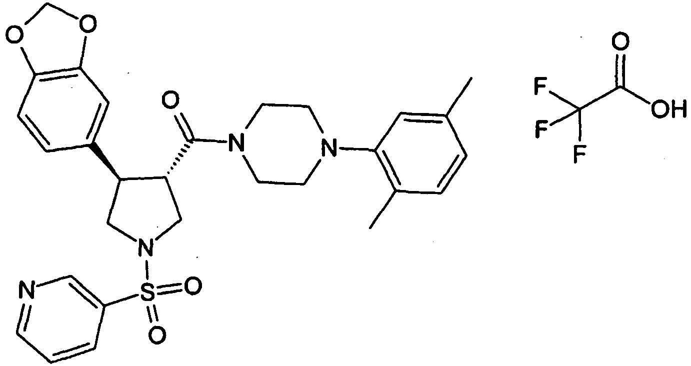

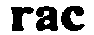

Analog zu Beispiel 1 erhält man aus den kommerziell erhältlichen Reagenzien Benzo[1,3]dioxol-5-carbaldehyd, Methyl(triphenylphosphoranyliden)acetat, N-(Methoxymethyl)-N-(trimethylsilylmethyl)-benzylamin, 1-(2,5-Dimethyl-phenyl)-piperazin und Pyridin-3-sulfonylchlorid [3,4-trans-4-Benzo[1,3]dioxol-5-yl-1-(pyridin-3-sulfonyl)-pyrrolidin-3-yl]-[4-(2,5-dimethyl-phenyl)-piperazin-1-yl]-methanon -trifluoracetat.
C29H32N4O5S.C2B13O2 (662.69), LCMS(ESI]: 549.3 (M+H⁺).

### Beispiel 90

### [[1-(3,5-Dimethyl-isoxazol-4-sulfonyl)-3,4-trans-4-methyl-pyrrolidin-3-yl]-[4-(2,5-dimethylphenyl)-piperazin-1-yl]-methanon-trifluoracetat

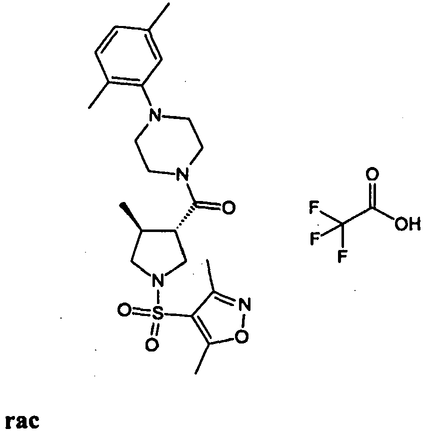

Analog zu Beispiel 1 erhält man aus den kommerziell erhältlichen Reagenzien Acetaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3,5-Dimethyl-isoxazol-4-sulfonylchlorid [[1-(3,5-Dimethyl-isoxazol-4-sulfonyl)-3,4-trans-4-methyl-pyrrolidin-3-yl]-[4-(2,5-dimethylphenyl)-piperazin-1-yl]-methanon-trifluoracetat.
C23H32N4O4S.C2HF3O2 (574.62), LCMS(ESI): 461.2 (M+H⁺).

### Beispiel 91

### [3,4-trans-4-(2,5-Dimethyl-phenyl)-piperazin-1-yl]-[1-(3-methoxy-benzolsulfonyl)-4-methylpyrrolidin-3-yl]-methanon-trifluoracetat

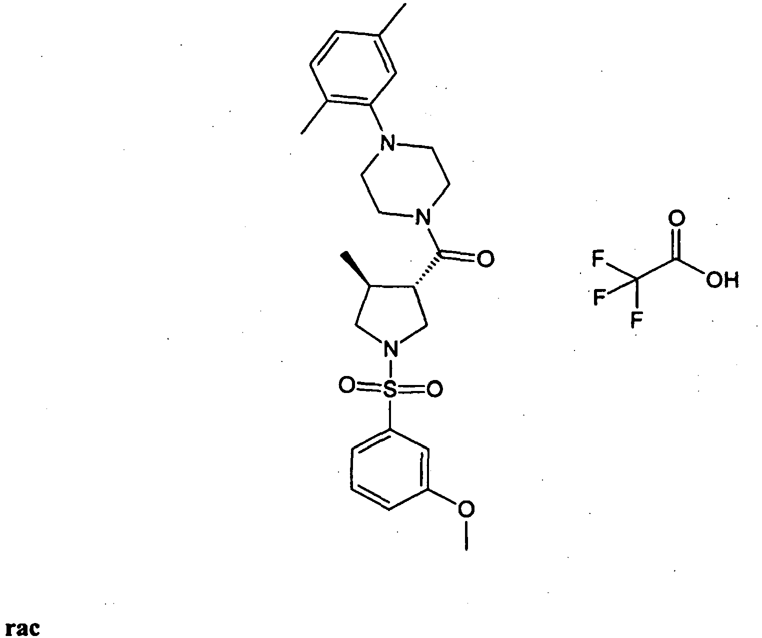

Analog zu Beispiel 1 erhält man aus den kommerziell erhältlichen Reagenzien Acetaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3-Methoxy-benzolsulfonylchlorid [3,4-trans-4-(2,5-Dimethyl-phenyl)-piperazin-1-yl]-[1-(3-methoxy-benzolsulfonyl)-4-methylpyrrolidin-3-yl]-methanon-trifluoracetat.
C25H33N3O4S.C2HF3O2 (585.65), LCMS(ESI): 472.2 (M+H⁺).

### Beispiel 92

### [3,4-trans-4-(2,5-Dimethyl-phenyl)-piperazin-1-yl]-[4-methyl-1-(pyridin-3-sulfonyl)-pyrrolidin-3- yl]-methanon-trifluoracetat

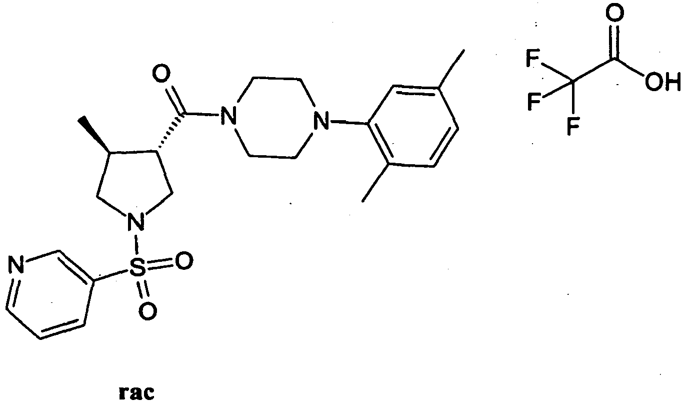

Analog zu Beispiel 1 erhält man aus den kommerziell erhältlichen Reagenzien Acetaldehyd, Methyl(triphenylphosphoranyliden)acetat, N-(Methoxymethyl)-N-(trimethylsilylmethyl)-benzylamin, 1-(2,5-Dimethyl-phenyl)-piperazin und Pyridin-3-sulfonylchlorid [3,4-trans-4-(2,5-Dimethyl-phenyl)-piperazin-1-yl]-[4-methyl-1-(pyridin-3-sulfonyl)-pyrrolidin-3- yl]-methanon-trifluoracetat.
C23H30N4O3S.C2HF3O2 (556.60), LCMS(ESI): 443.2 (M+H⁺).

### Beispiel 93

### [3,4-trans-4-Benzyl-1-(3,5-dimethyl-isoxazol-4-sulfonyl)-pyrrolidin-3-yl]-[4-(2,5-dimethylphenyl)- piperazin-1-yl]-methanon-trifluoracetat

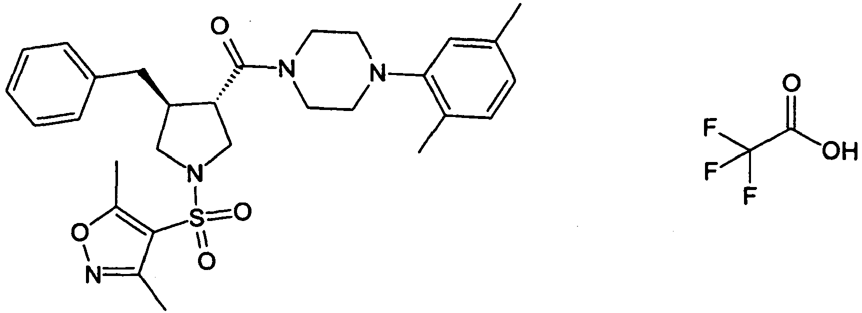

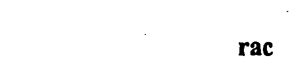

Analog zu Beispiel 1 erhält man aus den kommerziell erhältlichen Reagenzien Phenylacetaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3,5-Dimethylisoxazol-4-sulfonylchlorid [3,4-trans-4-Benzyl-1-(3,5-dimethyl-isoxazol-4-sulfonyl)-pyrrolidin-3-yl]-[4-(2,5-dimethyl-phenyl)- piperazin-1-yl]-methanon-trifluoracetat.
C29H36N4O4S.C2HF3O2 (650.72), LCMS(ESI): 537.3 (M+H⁺).

### Beispiel 94

### [3,4-trans-4-Benzyl-1-(3-methoxy-benzolsulfonyl)-pyrrolidin-3-yl]-[4-(2,5-dimethyl-phenyl)-piperazin-1-yl]-methanon-trifluoracetat

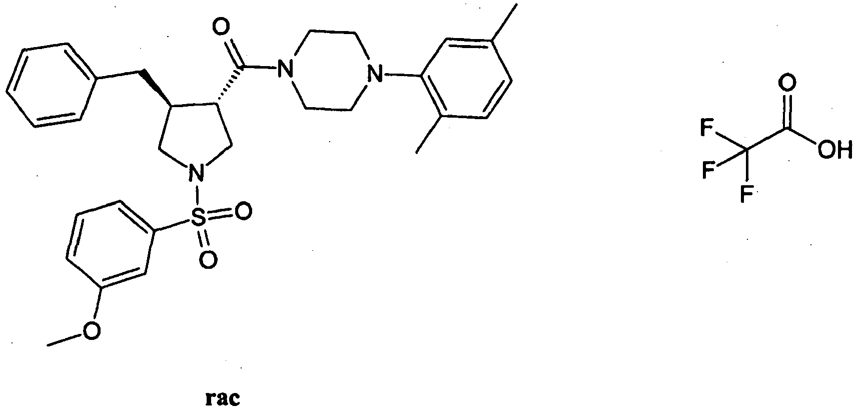

Analog zu Beispiel 1 erhält man aus den kommerziell erhältlichen Reagenzien Phenylacetaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3-Methoxy-benzolsulfonylchlorid [3,4-trans-4-Benzyl-1-(3-methoxy-benzolsulfonyl)-pyrrolidin-3-yl]-[4-(2,5-dimethyl-phenyl)-piperazin-1-yl]-methanon-trifluoracetat.
C31H37N3O4S.C2HF3O2 (661.75), LCMS(ESI): 548.3 (M+H⁺).

### Beispiel 95

### [1-(3,5-Dimethyl-isoxazol-4-sulfonyl)-3,4-trans-4-isopropyl-pyrrolidin-3-yl]-[4-(2,5-dimethylphenyl)-piperazin-1-yl]-methanon-trifluoracetat

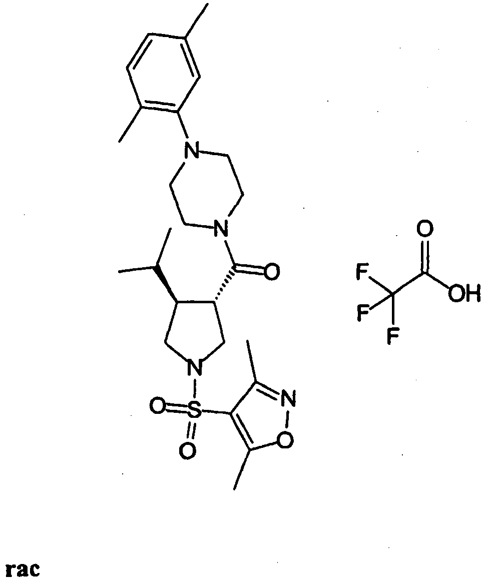

Analog zu Beispiel 1 erhält man aus den kommerziell erhältlichen Reagenzien 2-Methylpropionaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3,5-Dimethylisoxazol-4-sulfonylchlorid [1-(3,5-Dimethyl-isoxazol-4-sulfonyl)-3,4-trans-4-isopropylpyrrolidin-3-yl]-[4-(2,5-dimethyl-phenyl)-piperazin-1-yl]-methanon-trifluoracetat.
C25H36N4O4S.C2HF3O2 (602.78), LCMS(ESI): 489.3 (M+H⁺).

### Beispiel 96

### [4-(2,5-Dimethyl-phenyl)-piperazin-1-yl]-[3,4-trans-4-isopropyl-1-(3-methoxy-benzolsulfonyl)-pyrrolidin-3-yl]-methanon-trifluoracetat

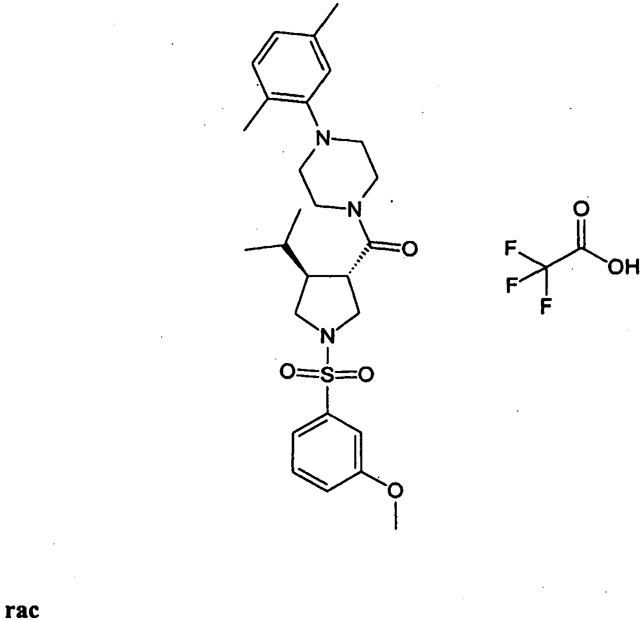

Analog zu Beispiel 1 erhält man aus den kommerziell erhältlichen Reagenzien Acetaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3-Methoxy-benzolsulfonylchlorid [4-(2,5-Dimethyl-phenyl)-piperazin-1-yl]-[3,4-trans-4-isopropyl-1-(3-methoxy-benzolsulfonyl)-pyrrolidin-3-yl]-methanon-trifluoracetat.
C27H37N3O4S.C2HF3O2 (613.70), LCMS(ESI): 500.3 (M+H⁺).

### Beispiel 97

### [1-(3,5-Dimethyl-isoxazol-4-sulfonyl)-3,4-trans-4-(tetrahydro-furan-3-yl)-pyrrolidin-3-yl]-[4-(2,5-dimethyl-phenyl)-piperazin-1-yl]-methanon-trifluoracetat

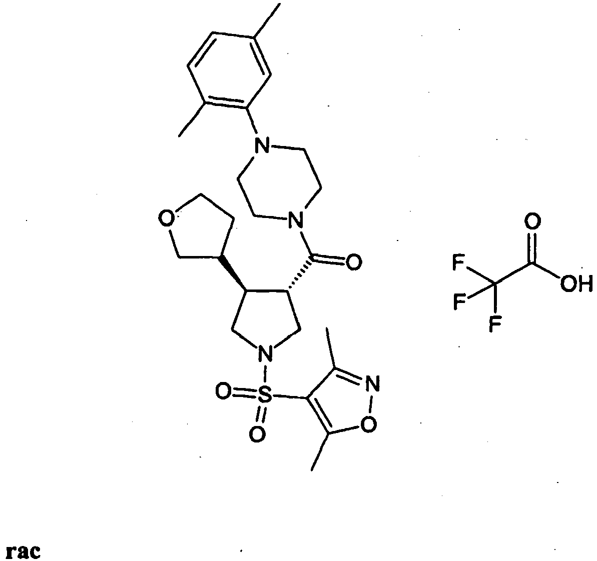

Analog zu Beispiel 1 erhält man aus den kommerziell erhältlichen Reagenzien Furan-2-carbaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3,5-Dimethylisoxazol-4-sulfonylchlorid [1-(3,5-Dimethyl-isoxazol-4-sulfonyl)-3,4-trans-4-(tetrahydrofuran-3-yl)-pyrrolidin-3-yl]-[4-(2,5-dimethyl-phenyl)-piperazin-1-yl]-methanon-trifluoracetat.
C26H36N4O5S.C2HF3O2 (630.69), LCMS(ESI): 517.3 (M+H⁺).

### Beispiel 98

### sulfonylchlorid [1-(3,5-Dimethyl-isoxazol-4-sulfonyl)-3,4-trans-4-o-tolyl-pyrrolidin-3-yl]-[4-(2,5-dimethyl-phenyl)-piperazin-1-yl]-methanon-trifluoracetat

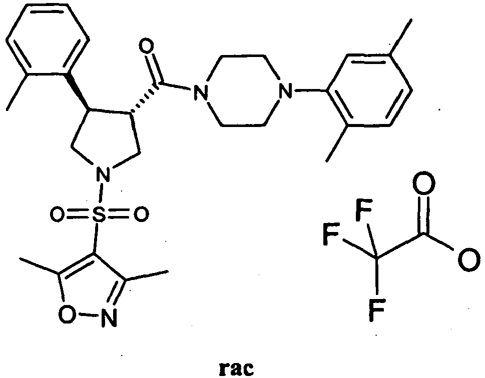

Analog zu Beispiel 1 erhält man aus den kommerziell erhältlichen Reagenzien 2-Methylbenzaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3,5-Dimethylisoxazol-4-sulfonylchlorid [1-(3,5-Dimethyl-isoxazol-4-sulfonyl)-3,4-rans-4-o-tolylpyrrolidin-3-yl]-[4-(2,5-dimethyl-phenyl)-piperazin-1-yl]-methanon-trifluoracetat.
C29H36N4O4S.C2HF3O2 (650.72), LCMS(ESI): 537.3 (M+H⁺).

### Beispiel 99

### [4-(2,5-Dimethyl-phenyl)-piperazin-1-yl]-[1-(3-methoxy-benzolsulfonyl)-3,4-trans-4-o-tolylpyrrolidin-3-yl]-methanon -trifluoracetat

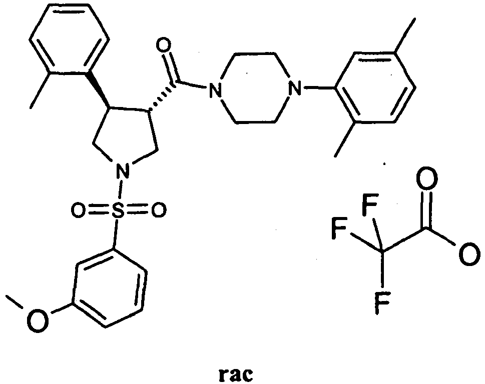

Analog zu Beispiel 1 erhält man aus den kommerziell erhältlichen Reagenzien 2-Methylbenzaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3-Methoxy-benzolsulfonylchlorid [4-(2,5-Dimethyl-phenyl)-piperazin-1-yl]-[1-(3-methoxybenzolsulfonyl)-3,4-trans-4-o-tolyl- pyrrolidin-3-yl]-methanon -trifluoracetat.
C31H37N304S.C2HF302 (661.75), LCMS(ESI): 548.3 (M+H⁺).

### Beispiel 100

### [4-(2,5-Dimethyl-phenyl)-piperazin-1-yl]-[1-(pyridin-3-sulfonyl)-3,4-trans-4-o-tolyl-pyrrolidin-3-yl]-methanon -trifluoracetat

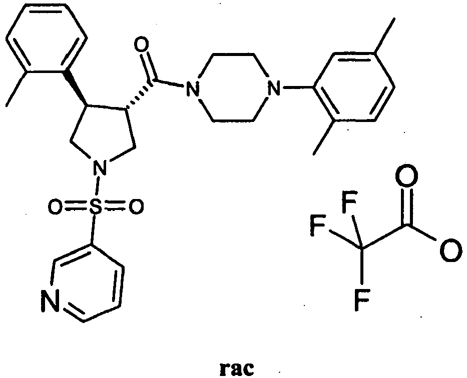

Analog zu Beispiel 1 erhält man aus den kommerziell erhältlichen Reagenzien 2-Methylbenzaldehyd, Methyl(triphenylphosphoranyliden)acetat, N-(Methoxymethyl)-N-(trimethylsilylmethyl)-benzylamin, 1-(2,5-Dimethyl-phenyl)-piperazin und Pyridin-3-sulfonylchlorid [4-(2,5-Dimethyl-phenyl)-piperazin-1-yl]-[1-(pyridin-3-sulfonyl)-3,4-trans-4-o-tolyl-pyrrolidin- 3-yl]-methanon -trifluoracetat.
C29H34N4O3S.C2HF3O2 (632.71), LCMS(ESI): 519.3 (M+H⁺).

### Beispiel 101

### [1-(3,5-Dimethyl-isoxazol-4-sulfonyl)-3,4-trans-4-m-tolyl-pyrrolidin-3-yl]-[4-(2,5-dimethylphenyl)-piperazin-1-yl]-methanon-trifluoracetat

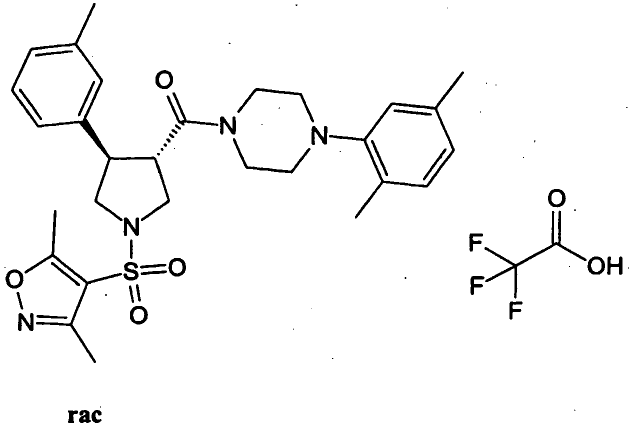

Analog zu Beispiel 1 erhält man aus den kommerziell erhältlichen Reagenzien 3-Methylbenzaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3,5-Dimethylisoxazol-4-sulfonylchlorid [1-(3,5-Dimethyl-isoxazol-4-sulfonyl)-3,4-trans-4-m-tolylpyrrolidin-3-yl]-[4-(2,5-dimethyl-phenyl)-piperazin-1-yl]-methanon-trifluoracetat.
C29H36N4O4S.C2HF3O2 (650.72), LCMS(ESI): 537.3 (M+H⁺).

### Beispiel 102

### [4-(2,5-Dimethyl-phenyl)-piperazin-1-yl]-[1-(3-methoxy-benzolsulfonyl)-3,4-trans-4-m-tolylpyrrolidin-3-yl]-methanon -trifluoracetat

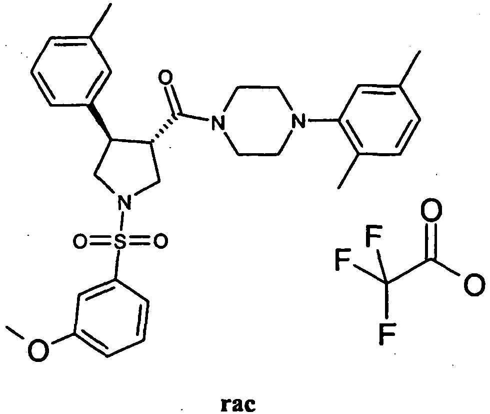

Analog zu Beispiel 1 erhält man aus den kommerziell erhältlichen Reagenzien 3-Methylbenzaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3-Methoxy-benzolsulfonylchlorid [4-(2,5-Dimethyl-phenyl)-piperazin-1-yl]-[1-(3-methoxybenzolsulfonyl)-3,4-trans-4-m-tolyl- pyrrolidin-3-yl]-methanon -trifluoracetat.
C31H37N3O4S.C2HF3O2 (661.75), LCMS(ESI): 548.3 (M+H⁺).

### Beispiel 103

### [1-(3,5-Dimethyl-isoxazol-4-sulfonyl)-3,4-trans-4-(3,5-dimethyl-isoxazol-4-yl)-pyrrolidin-3-yl]-[4- (2,5-dimethyl-phenyl)-piperazin-1-yl]-methanon-trifluoracetat.

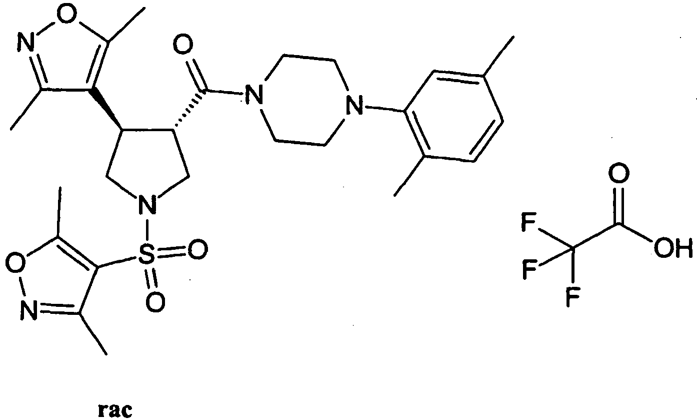

Analog zu Beispiel 1 erhält man aus den kommerziell erhältlichen Reagenzien 3,5-Dimethylisoxazol-4-carbaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3,5-Dimethylisoxazol-4-sulfonylchlorid [1-(3,5-Dimethyl-isoxazol-4-sulfonyl)-3,4-trans-4-(3,5-dimethylisoxazol-4-yl)-pyrrolidin-3-yl]-[4- (2,5-dimethyl-phenyl)-piperazin-1-yl]-methanontrifluoracetat.
C27H35N5O5S.C2HF3O2 (655.70), LCMS(ESI): 542.3 (M+H⁺).

### Beispiel 104

### [3,4-rans-4-(3,5-Dimethyl-isoxazol-4-yl)-1-(3-methoxy-benzolsulfonyl)-pyrrolidin-3-yl]-[4-(2,5- dimethyl-phenyl)-piperazin-1-yl]-methanon -trifluoracetat

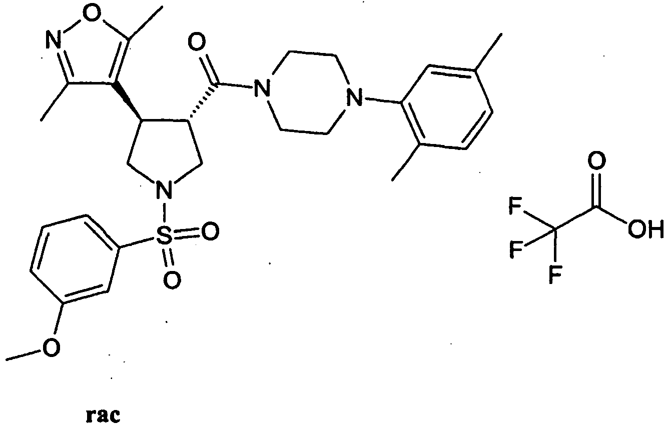

Analog zu Beispiel 1 erhält man aus den kommerziell erhältlichen Reagenzien 3,5-Dimethylisoxazol-4-carbaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3-Methoxy-benzolsulfonylchlorid [3,4-trans-4-(3,5-Dimethyl-isoxazol-4-yl)-1-(3-methoxy-benzolsulfonyl)-pyrrolidin-3-yl]-[4-(2,5-dimethyl-phenyl)-piperazin-1-yl]-methanon -trifluoracetat.
C29H36N4O5S.C2HF3O2.C2HF3O2 (666.72), LCMS(ESI): 553.3 (M+H⁺).

### Beispiel 105

### [3,4-trans-4-(3,5-Dimethyl-isoxazol-4-yl)-1-(pyridin-3-sulfonyl)-pyrrolidin-3-yl]-[4-(2,5-dimethyl-phenyl)-piperazin-1-yl]-methanon -trifluoracetat

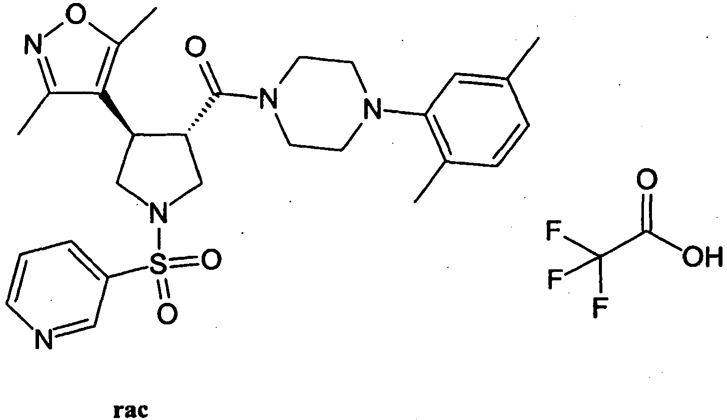

Analog zu Beispiel 1 erhält man aus den kommerziell erhältlichen Reagenzien 3,5-Dimethylisoxazol-4-carbaldehyd, Methyl(triphenylphosphoranyliden)acetat, N-(Methoxymethyl)-N-(trimethylsilylmethyl)-benzylamin, 1-(2,5-Dimethyl-phenyl)-piperazin und Pyridin-3-sulfonylchlorid [3,4-trans-4-(3,5-Dimethyl-isoxazol-4-yl)-1-(pyridin-3-sulfonyl)-pyrrolidin-3-yl]-[4-(2,5-dimethyl-phenyl)-piperazin-1-yl]-methanon-trifluoracetat.
C27H33N5O4S.C2HF3O2 (637.68), LCMS(ESI): 524.2 (M+H⁺).

### Beispiel 106

### [1-(3,5-Dimethyl-isoxazol-4-sulfonyl)-3,4-trans-4-(2,6-dimethyl-phenyl)-pyrrolidin-3-yl]-[4-(2,5-dimethyl-phenyl)-piperazin-1-yl]-methanon-trifluoracetat

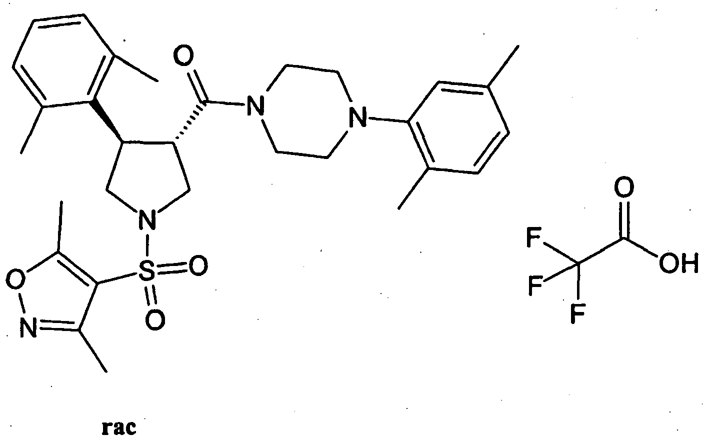

Analog zu Beispiel 1 erhält man aus den kommerziell erhältlichen Reagenzien 2,3-Dimethylbenzaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3,5-Dimethylisoxazol-4-sulfonylchlorid [1-(3,5-Dimethyl-isoxazol-4-sulfonyl)-3,4-trans-4-(2,6-dimethylphenyl)-pyrrolidin-3-yl]-[4-(2,5-dimethyl-phenyl)-piperazin-1-yl]-methanon-trifluoracetat.
C30H38N4O4S.C2HF3O2 (664.75), LCMS(ESI): 551.3 (M+H⁺).

### Beispiel 107

### [1-(3,5-Dimethyl-isoxazol-4-sulfonyl)-3,4-trans-4-(4-methyl-3,4-dihydro-2H-benzo[1,4]oxazin-7- yl)-pyrrolidin-3-yl]-[4-(2,5-dimethyl-phenyl)-piperazin-1-yl]-methanon-trifluoracetat

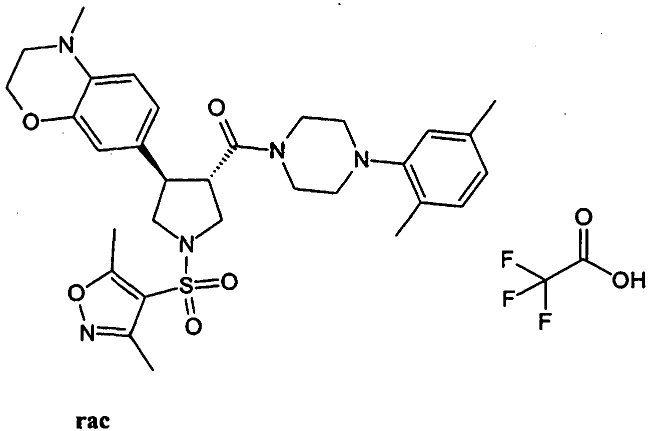

Analog zu Beispiel 1 erhält man aus den kommerziell erhältlichen Reagenzien 4-Methyl-3,4-dihydro-2H-benzo[1,4]oxazine-7-carbaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3,5-Dimethyl-isoxazol-4-sulfonylchlorid [1-(3,5-Dimethyl-isoxazol-4-sulfonyl)-3,4-trans-4-(4-methyl-3,4-dihydro-2H-benzo[1,4]oxazin-7- yl)-pyrrolidin-3-yl]-[4-(2,5-dimethyl-phenyl)-piperazin-1-yl]-methanon-trifluoracetat.
C31H39N5O5S.C2HF3O2 (707.76), LCMS(ESI): 594.2 (M+H⁺).

### Beispiel 108

### [3,4-trans-4-Benzo[1,3]dioxol-4-yl-1-(3,5-dimethyl-isoxazol-4-sulfonyl)-pyrrolidin-3-yl]-[4-(2,5-dimethyl-phenyl)-piperazin-1-yl]-methanon-trifluoracetat

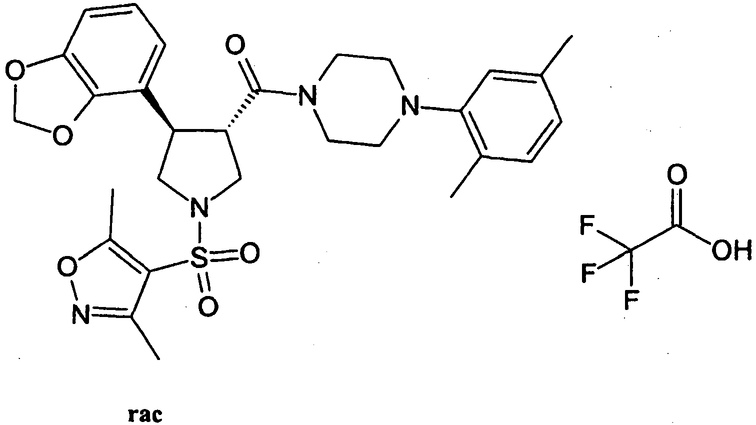

Analog zu Beispiel 1 erhält man aus den kommerziell erhältlichen Reagenzien 4-Methyl-3,4-dihydro-2H-benzo[1,4]oxazine-7-carbaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3,5-Dimethyl-isoxazol-4-sulfonylchlorid [3,4-trans-4-Benzo[1,3]dioxol-4-yl-1-(3,5-dimethylisoxazol-4-sulfonyl)-pyrrolidin-3-yl]-[4-(2,5-dimethyl-phenyl)-piperazin-1-yl]-methanontrifluoracetat.
C29H34N4O6S.C2HF3O2 (680.71), LCMS(ESI): 567.2 (M+H⁺).

### Beispiel 109

### [4-(2,5-Dimethyl-phenyl)-piperazin-1-yl]-[1-(3-methoxy-benzolsulfonyl)-3,4-trans-4-(4-methyl-3,4- dihydro-2H-benzo[1,4]oxazin-7-yl)-pyrrolidin-3-yl]-methanon-trifluoracetat

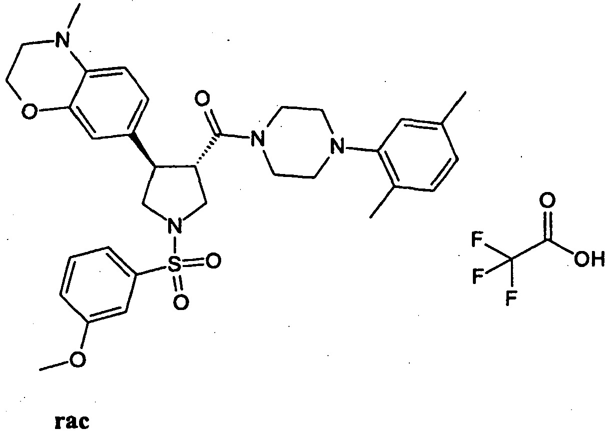

Analog zu Beispiel 1 erhält man aus den kommerziell erhältlichen Reagenzien 4-Methyl-3,4-dihydro-2H-benzo[1,4]oxazine-7-carbaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3,5-Dimethyl-isoxazol-4-sulfonylchlorid [4-(2,5-Dimethyl-phenyl)-piperazin-1-yl]-[1-(3-methoxy-benzolsulfonyl)-3,4-trans-4-(4-methyl-3,4- dihydro-2H-benzo[1,4]oxazin-7-yl)-pyrrolidin-3-yl]-methanon-trifluoracetat.
C33H40N405S.C2HF3O2 (718.80), LCMS(ESI): 605.2 (M+H⁺).

### Beispielsynthesen nach Verfahren B:

### Beispiel 110

### [4-(2,5-Dimethyl-phenyl)-piperazin-1-yl]-[1-(3-methoxy-benzolsulfonyl)-3,4-trans-4-thiophen-3-yl- pyrrolidin-3-yl]-methanon-trifluoracetat

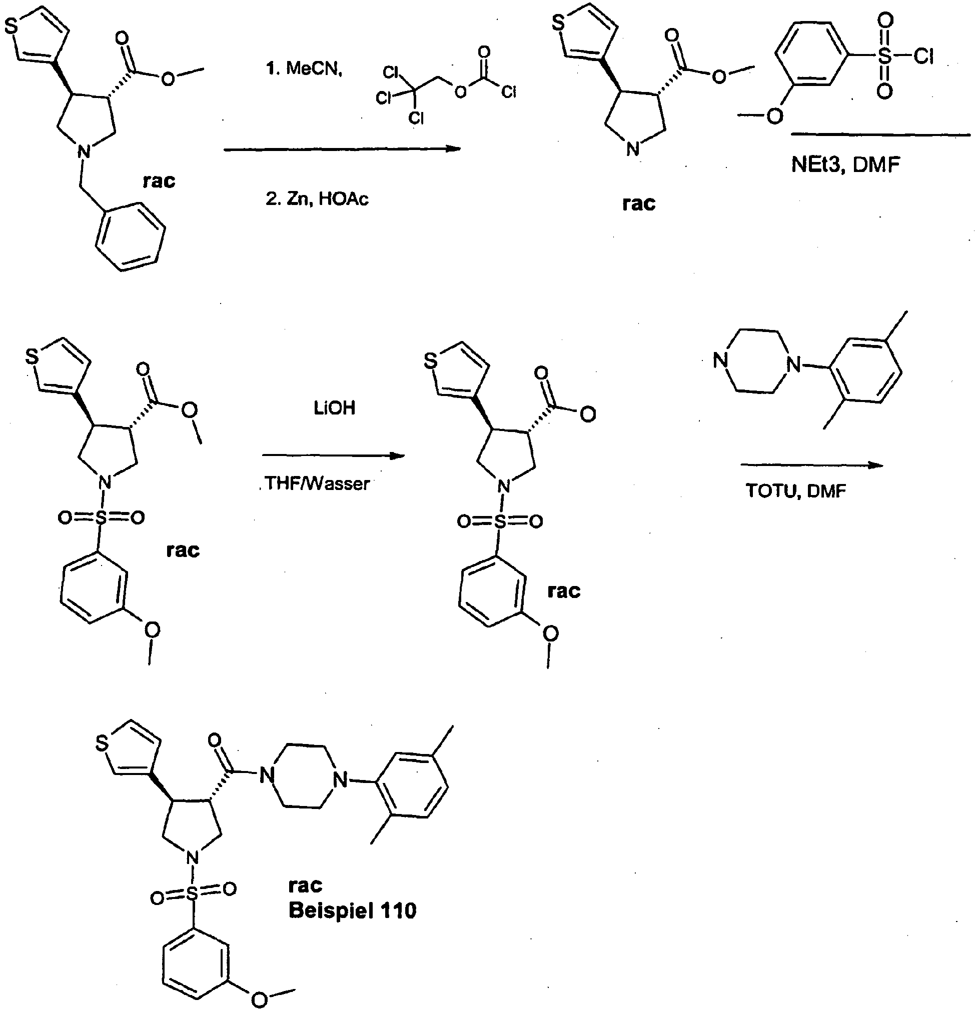

### 3,4-trans-4-Thiophen-3-yl-pyrrolidin-3-carbonsäuremethylester-trifluoracetat

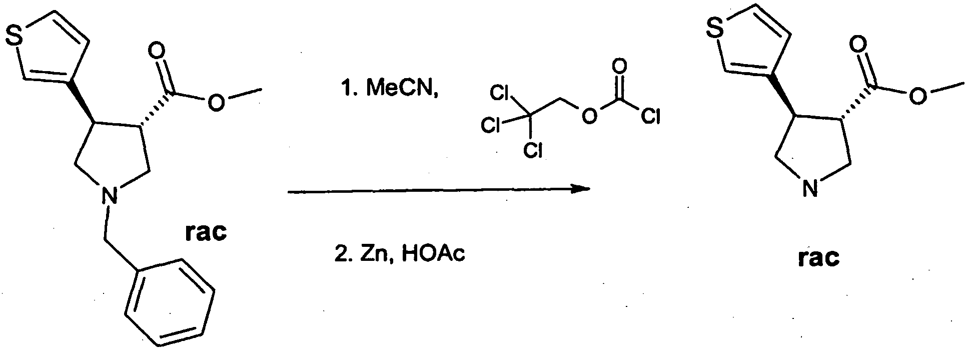

2.0 g 1-Benzyl-4-thiophen-3-yl-pyrrolidin-3-carbonsäuremethylester (Hergestellt analog zu Beispiel 1 aus den kommerziell erhältlichen Reagenzien Thiophen-2-carbaldehyd, Methyl(triphenylphosphoranyliden)acetat und N-(Methoxymethyl)-N-(trimethylsilylmethyl)-benzylamin) werden in 30 ml trockenem Acetonitril gelöst und bei Raumtemperatur mit 0.90 ml 2,2,2-Trichlorethylchloroformat versetzt. Nach dreißig minütigem Rühren bei Raumtemperatur wird das Acetonitril im Vakuum entfernt und der Rückstand in 40 ml Eisessig aufgenommen. Man fügt 845 mg Zink hinzu und rührt das Reaktionsgemisch bei bei Raumtemperatur. Nach zwei Stunden wird das Reaktionsgemisch filtriert, mit Dichlormethan nachgewaschen, und anschließend das Filtrat im Vakuum eingeengt. Der Rückstand wird mit 100 ml Toluol versetzt und im Vakuum eingeengt. Der Rückstand wird mittels RP-HPLC gereinigt. Man erhält 2.2 g 3,4-trans-4-Thiophen-3-yl-pyrrolidin-3-carbonsäuremethylestertrifluoracetat als Öl.
C10H13NO2S. C2HF3O2 (325.30), LCMS(ESI): 212.2 (M+H⁺).

### 1-(3-Methoxy-benzolsulfonyl)-3,4-trans-4-thiophen-3-yl-pyrrolidin-3-carbonsäuremethylester

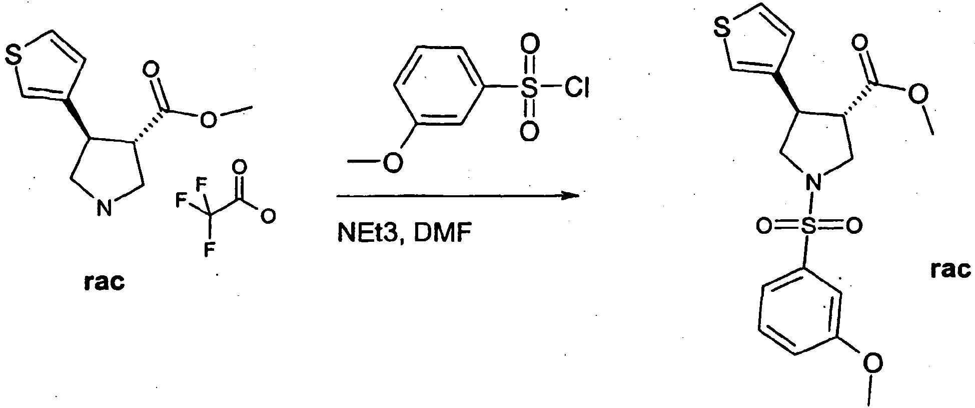

1.1 g 3,4-trans-4-Thiophen-3-yl-pyrrolidin-3-carbonsäuremethylester-trifluoracetat werden in 20 ml N,N-Dimethylformamid gelöst und mit 2.7 ml Triethylamin und 1.0 g 3-Methoxy-benzolsulfonylchlorid versetzt. Nach dreißig Minuten Rühren bei Raumtemperatur wird das Reaktionsgemisch durch Zugabe von 100 ml Ethylacetat verdünnt und dreimal mit je 30 ml gesättigter Natriumhydrogemncarbonatlösung gewaschen. Die organische Phase wird über MgS04 getrocknet und anschließend das Lösungsmittel im Vakuum entfernt. Man erhält 900 mg 1-(3-Methoxy-benzolsulfonyl)-3,4-trans-4-thiophen-3-yl-pyrrolidin-3-carbonsäuremethylester als Öl.
C17H19NO5S2 (381.47), LCMS(ESI): 382.3 (M+H⁺).

### 1-(3-Methoxy-benzolsulfonyl)-3,4-trans-4-thiophen-3-yl-pyrrolidin-3-carbonsäure

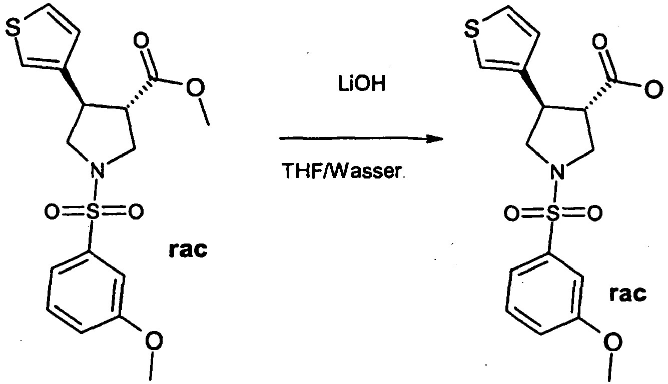

900 mg 1-(3-Methoxy-benzolsulfonyl)-3,4-trans-4-thiophen-3-yl-pyrrolidin-3-carbonsäuremethylester werden in einem Gemisch aus 10 ml Tetrahydrofuran und 5 ml Wasser gelöst und mit 280 mg Lithiumhydroxid versetzt. Das Reaktionsgemisch wird bei Raumtemperatur gerührt. Nach einer Stunde wird das Reaktionsgemisch durch Zugabe von wenigen Tropfen konzentrierter Salzsäure angesäuert. Das Reaktionsgemisch wird dreimal mit je 50 Ethylacetat extrahiert. Die vereinigten organischen Phasen werden über MgSo4 getrocknet und anschließend das Lösungsmittel im Vakuum entfernt. Man erhält 800 mg 1-(3-Methoxy-benzolsulfonyl)-3,4-trans-4-thiophen-3-yl-pyrrolidin-3-carbonsäure als Öl. C16H17NO5S2 (367.45), LCMS(ESI): 368.3 (M+H⁺).

### [4-(2,5-Dimethyl-phenyl)-piperazin-1-yl]-[1-(3-methoxy-benzolsulfonyl)-3,4-trans-4-thiophen-3-yl- pyrrolidin-3-yl]-methanon-trifluoracetat

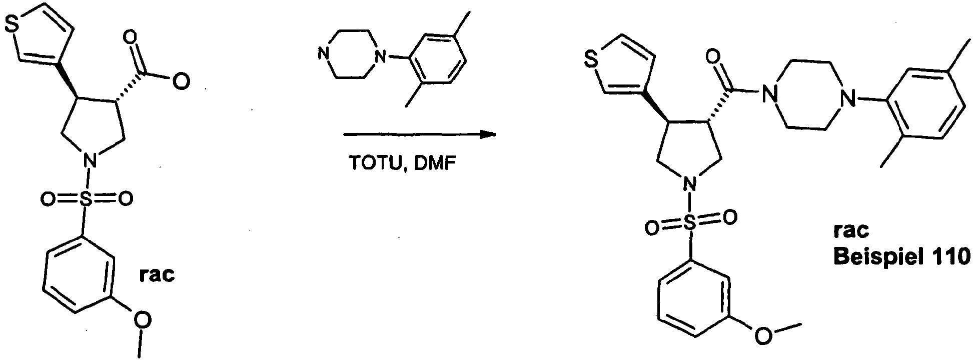

800 mg 1-(3-Methoxy-benzolsulfonyl)-3,4-trans-4-thiophen-3-yl-pyrrolidin-3-carbonsäure, 0.4 ml Triethylamin und 440 mg 1-(2,5-Dimethylphenyl)-piperazin werden in 15 ml N,N-Dimethylformamid gelöst und portionsweise mit 750 mg O-[Cyan(ethoxycarbonyl)methylenamino]-1,1,3,3-tetramethyluronium-tetrafluoroborat versetzt. Das Reaktionsgemisch wird bei Raumtemperatur gerührt. Nach einer Stunde wird das Reaktionsgemisch durch Zugabe von 100 ml Ethylacetat verdünnt und fünfmal mit je 30 ml gesättigter Natriumhydrogencarbonatlösung gewaschen. Die organische Phase wird über MgS04 getrocknet und anschließend das Lösungsmittel im Vakuum entfernt. Der Rückstand wird mittels RP-HPLC gereinigt. Man erhält 320 mg [4-(2,5-Dimethyl-phenyl)-piperazin-1-yl]-[1-(3-methoxy-benzolsulfonyl)-3,4-trans-4-thiophen-3-yl- pyrrolidin-3-yl]-methanontrifluoracetat als Lyophilisat.
C28H33N3O4S2. C2HF3O2 (653.74), LCMS(ESI): 540.6(M+H⁺).

### Beispiel 111

### [1-(3,5-Dimethyl-isoxazol-4-sulfonyl)-3,4-trans-4-thiophen-3-yl-pyrrolidin-3-yl]-[4-(2,5-dimethyl-phenyl)-piperazin-1-yl]-methanon-trifluoracetat

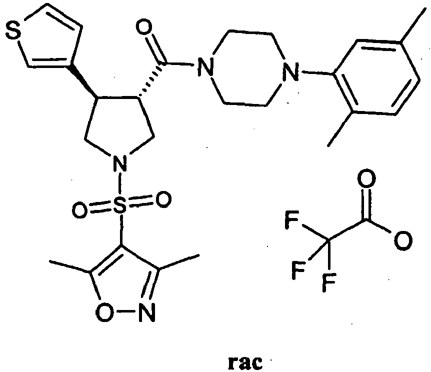

Analog zu Beispiel 110 erhält man aus den kommerziell erhältlichen Reagenzien Thiophen-2-carbaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3,5-Dimethylisoxazol-4-sulfonylchlorid [1-(3,5-Dimethyl-isoxazol-4-sulfonyl)-3,4-trans-4-thiophen-3-yl-pyrrolidin-3-yl]-[4-(2,5-dimethyl-phenyl)-piperazin-1-yl]-methanon-trifluoracetat.
C26H32N4O4S2.C2HF3O2 (642.72), LCMS(ESI): 529.3 (M+H⁺).

### Beispiel 112

### [1-(3,5-Dimethyl-isoxazol-4-sulfonyl)-3,4-trans-4-pyridin-3-yl-pyrrolidin-3-yl]-[4-(2,5-dimethyl-phenyl)-piperazin-1-yl]-methanon-trifluoracetat

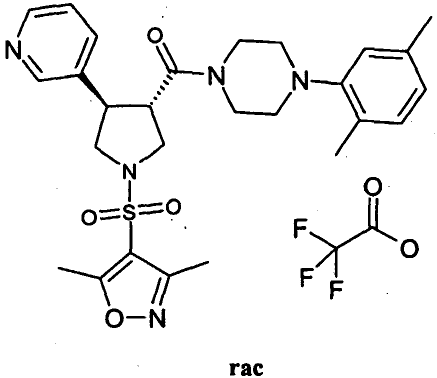

Analog zu Beispiel 110 erhält man aus den kommerziell erhältlichen Reagenzien Pyridin-3-carbaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3,5-Dimethylisoxazol-4-sulfonylchlorid [1-(3,5-Dimethyl-isoxazol-4-sulfonyl)-3,4-trans-4-pyridin-3-yl-pyrrolidin-3-yl]-[4-(2,5-dimethyl-phenyl)-piperazin-1-yl]-methanon-trifluoracetat.
C27H33N5O4S.C2HF3O2 (637.68), LCMS(ESI): 524.3 (M+H⁺).

### Beispiel 113

### [4-(2,5-Dimethyl-phenyl)-piperazin-1-yl]-[1-(pyridin-3-sulfonyl)-3,4-trans-4-pyridin-3-yl-pyrrolidin-3-yl]-methanon-trifluoracetat

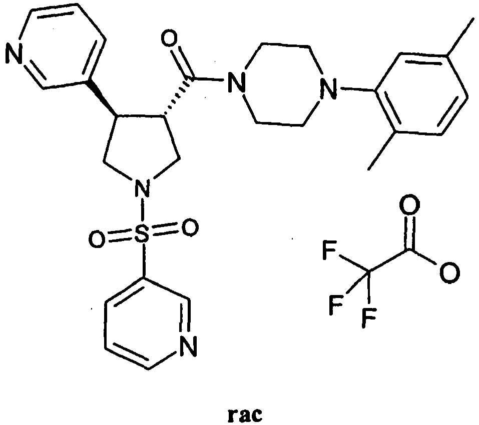

Analog zu Beispiel 110 erhält man aus den kommerziell erhältlichen Reagenzien Pyridin-3-carbaldehyd, Methyl(triphenylphosphoranyliden)acetat, N-(Methoxymethyl)-N-(trimethylsilylmethyl)-benzylamin, 1-(2,5-Dimethyl-phenyl)-piperazin und Pyridin-3-sulfonylchlorid [4-(2,5-Dimethyl-phenyl)-piperazin-1-yl]-[1-(pyridin-3-sulfonyl)-3,4-trans-4-pyridin-3-yl- pyrrolidin-3-yl]-methanon-trifluoracetat.
C27H31N5O3S.C2HF3O2 (619.67), LCMS(ESI): 506.3 (M+H⁺).

### Beispiel 114

### [4-(2,5-Dimethyl-phenyl)-piperazin-1-yl]-[3,4-trans-4-furan-3-yl-1-(3-methoxybenzolsulfonyl)- pyrrolidin-3-yl]-methanon -trifluoracetat

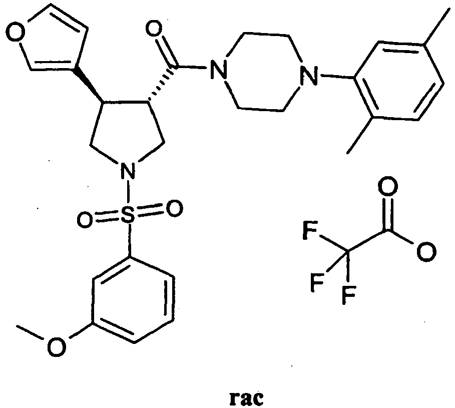

Analog zu Beispiel 110 erhält man aus den kommerziell erhältlichen Reagenzien Furan-2-carbaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3-Methoxy-benzolsulfonylchlorid [4-(2,5-Dimethyl-phenyl)-piperazin-1-yl]-[3,4-trans-4-furan-3-yl-1-(3-methoxy-benzolsulfonyl)- pyrrolidin-3-yl]-methanon -trifluoracetat.
C28H33N3O5S.C2HF3O2 (637.68), LCMS(ESI): 524.3 (M+H⁺).

### Beispiel 115

### [1-(3,5-Dimethyl-isoxazol-4-sulfonyl)-3,4-trans-4-furan-3-yl-pyrrolidin-3-yl]-[4-(2,5-dimethylphenyl)-piperazin-1-yl]-methanon-trifluoracetat

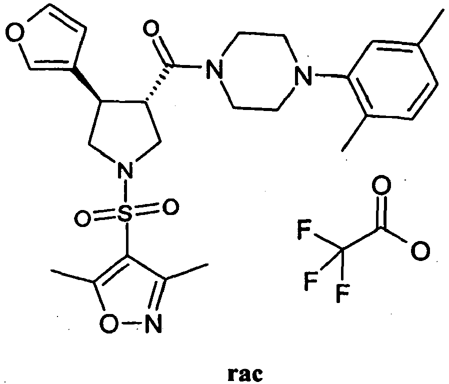

Analog zu Beispiel 110 erhält man aus den kommerziell erhältlichen Reagenzien Furan-2-carbaldehyd, Methyl(triphenylphosphoranyliden)acetat, N-(Methoxymethyl)-N-(trimethylsilylmethyl)-benzylamin, 1-(2,5-Dimethyl-phenyl)-piperazin und 3,5-Dimethylisoxazol-4-sulfonylchlorid [1-(3,5-Dimethyl-isoxazol-4-sulfonyl)-3,4-trans-4-furan-3-yl-pyrrolidin-3-yl]-[4-(2,5-dimethyl-phenyl)-piperazin-1-yl]-methanon-trifluoracetat.
C26H32N4O5S.C2HF3O2 (626.66), LCMS(ESI): 513.3 (M+H⁺).

### Beispiel 116

### [4-(2,5-Dimethyl-phenyl)-piperazin-1-yl]-[3,4-trans-4-furan-3-yl-1-(pyridin-3-sulfonyl)-pyrrolidin- 3-yl]-methanon-trifluoracetat

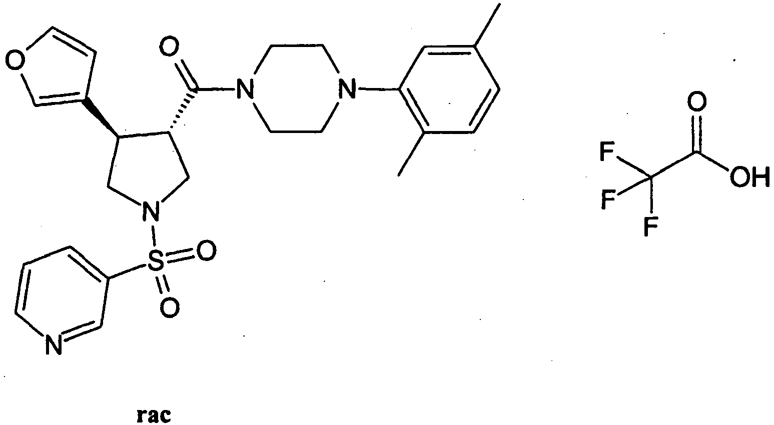

Analog zu Beispiel 110 erhält man aus den kommerziell erhältlichen Reagenzien Furan-2-carbaldehyd, Methyl(triphenylphosphoranyliden)acetat, N-(Methoxymethyl)-N-(trimethylsilylmethyl)-benzylamin, 1-(2,5-Dimethyl-phenyl)-piperazin und Pyridin-3-sulfonylchlorid [4-(2,5-Dimethyl-phenyl)-piperazin-1-yl]-[3,4-trans-4-furan-3-yl-1-(pyridin-3-sulfonyl)-pyrrolidin- 3-yl]-methanon-trifluoracetat.
C26H30N4O4S.C2HF3O2 (608.64), LCMS(ESI): 495.2 (M+H⁺).

### Beispielsynthesen nach Verfahren D:

### Beispiel 117

### [4-(2,5-Dimethyl-phenyl)-piperazin-1-yl]-[3,4-cis-4-phenyl-1-(thiophen-2-sulfonyl)-pyrrolidin-3- yl]-methanon

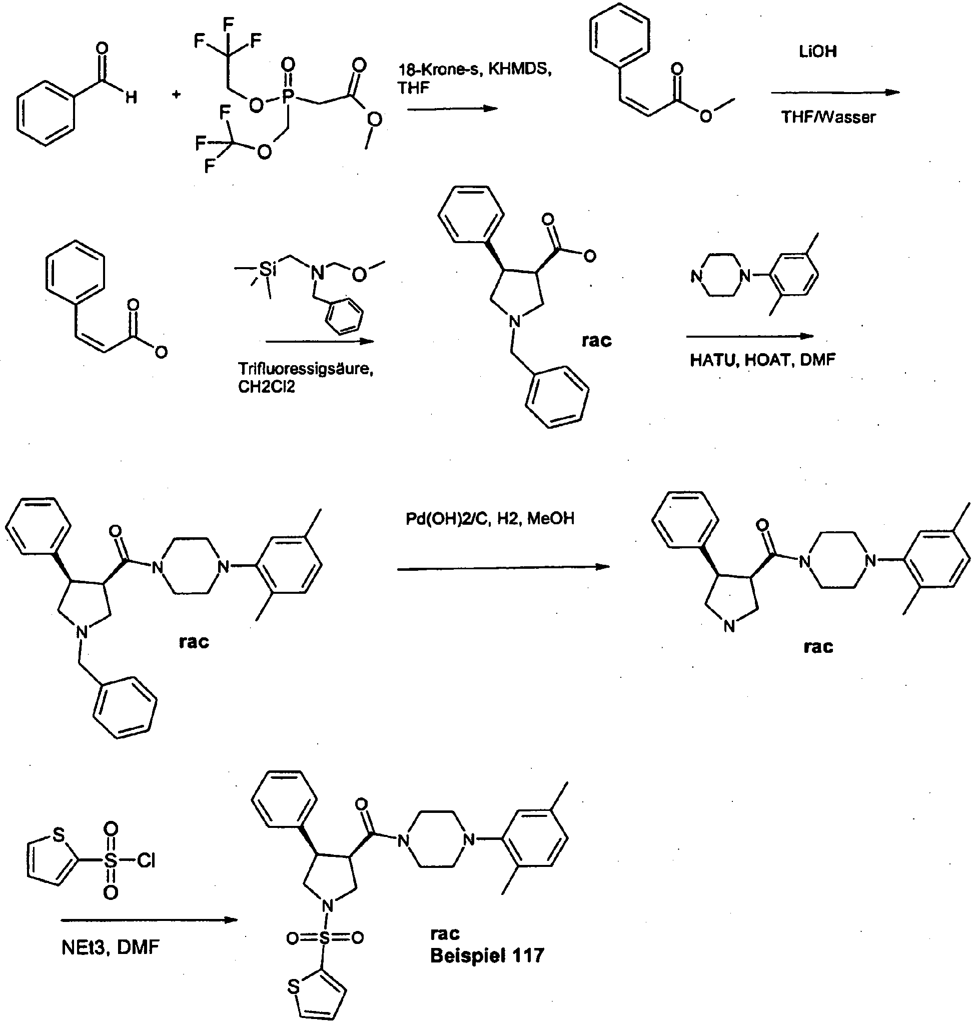

### (Z)-3-Phenyl-acrylsäuremethylester

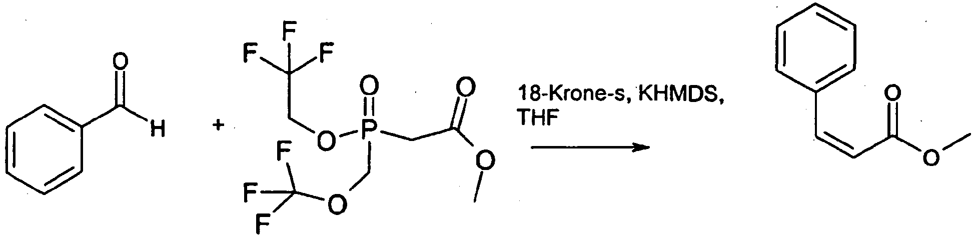

9.4 g 18-Krone-6 und 1,7 ml [Bis-(2,2,2-trifluorethoxy)-phosphoryl]-essigsäuremethylester werden unter Argon in 120 ml trockenem Tetrahydrofuran gelöst und auf -78°C abgekühlt. Zu diesem Gemisch werden 14.3 ml einer 0.5 molaren Lösung von Kalium-bis(trimethylsilyl)amid in Toluol gefolgt von 0.85 ml Benzaldehyd zugefügt. Nach dreißig minütigem Rühren bei - 78°C wird das Reaktionsgemisch durch Zugabe gesättigter Ammoniumchloridlösung gequenscht und mit fünf Portionen mit je 50 ml Ethylacetat extrahiert. Die vereinigten organischen Phasen werden über MgS04 getrocknet und anschließend das Lösungsmittel im Vakuum entfernt. Der Rückstand wird an Kieselgel mit dem Eluens n-Heptan:Ethylacetat = 20:1 gereinigt. Man erhält 1.1 g (Z)-3-Phenyl-acrylsäuremethylester als Öl.
C10H10O2 (162.19), LCMS(ESI): 163.2 (M+H⁺), Rf(n-Heptan:Ethylacetat = 2:1) = 0.62, Kopplungskonstante der olefinischen Protonen im 1H-NMR beträgt 12 Hz.

### (Z)-3-Phenyl-acrylsäure

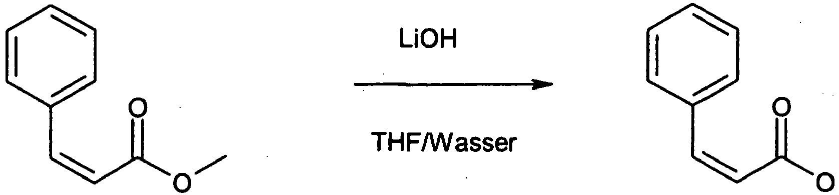

1.1 g (Z)-3-Phenyl-acrylsäuremethylester werden in einem Gemisch aus 30 ml Tetrahydrofuran und 10 ml Wasser gelöst und mit 340 mg Lithiumhydroxid versetzt. Man rührt bei 60°C nach. Nach vier Stunden wird das Reaktionsgemisch durch Zugabe von einmolarer Salzsäurelösung angesäuert und fünfmal mit je 50 ml Ethylacetat extrahiert. Die vereinigten organischen Phasen werden über MgSO4 getrocknet und anschließend das Lösungsmittel im Vakuum entfernt. Man erhält 900 mg (Z)-3-Phenyl-acrylsäure als Öl.

C9H8O2 (148.16), LCMS(ESI): 149.2 (M+H⁺), Kopplungskonstante der olefinischen Protonen im 1H-NMR beträgt 12 Hz.

### 1-Benzyl-3,4-cis-4-phenyl-pyrrolidin-3-carbonsäure-trifluoracetat

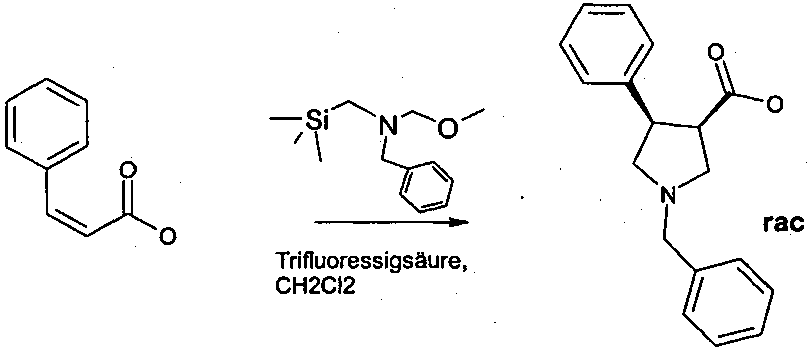

2 ml einer einmolaren Lösung von Trifluoressigsäure in Dichlormethan wird bei 0°C zu einer Lösung von 5.0 g N-(Methoxymethyl)-N-(trimethylsilylmethyl)-benzylamin und 3 g (Z)-3-Phenyl-acrylsäure in 50 ml Dichlormethan zugetropft. Nach einstündigem Rühren bei Raumtemperatur wird das Lösungsmittel im Vakuum entfernt und der Rückstand durch RP-HPLC gereinigt. Man erhält 1.14 g 1-Benzyl-3,4-cis-4-phenyl-pyrrolidin-3-carbonsäure-trifluoracetat als farbloses Öl.
C18H19N02. C2HF3O2 (395.38), LCMS(ESI): 282.2 (M+H⁺).

### (1-Benzyl-3,4-cis-4-phenyl-pyrrolidin-3-yl)-[4-(2,5-dimethyl-phenyl)-piperazin-1-yl]-methanon-trifluoracetat

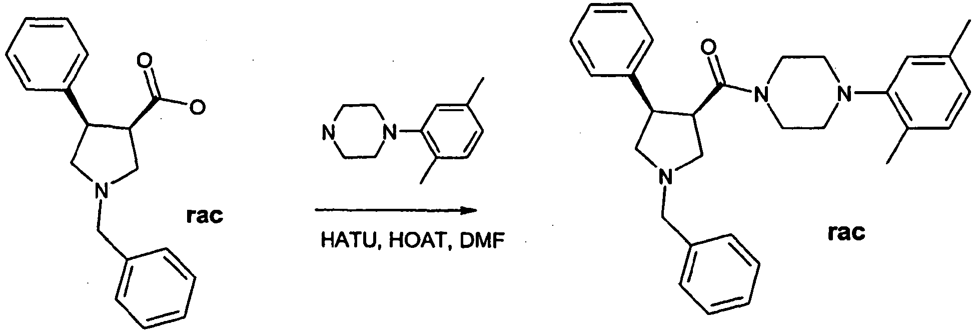

1.14 g 1-Benzyl-3,4-cis-4-phenyl-pyrrolidin-3-carbonsäure-trifluoracetat, 980 mg 1-(2,5-Dimethylphenyl)-piperazin, 3.0 ml N,N-Diisopropylethylamin,, 840 mg 1-Hydroxy-7-azabenzotriazol und 2,4 g O-(7-Azabenzotriazol-1-yl)-N,N,N',N'-tetramethyluroniumhexafluorophoaphat werden werden in 20 ml N,N-Dimethylformamid gelöst und eine Stunde bei Raumtemperatur gerührt. Danach wird das Reaktionsgemsisch durch Zugabe von 100 ml Ethylacetat verdünnt und mit gesättigter Natriumhydrogencarbonatlösung gewaschen. Die organische Phase wird abgetrennt, über MgS04 getrocknet und das Lösungsmittel im Vakuum entfernt. Der Rückstand wird mittels RP-HPLC gereinigt. Man erhält 940 mg (1-Benzyl-3,4-cis-4-phenyl-pyrrolidin-3-yl)-[4-(2,5-dimethyl-phenyl)-piperazin-1-yl]-methanon-trifluoracetat als farbloses Lyophilisat.
C30H35N30. C2HF3O2 (567.65), LCMS(ESI): 454.6 (M+H⁺).

### [4-(2,5-Dimethyl-phenyl)-piperazin-1-yl]-(3,4-cis-4-phenyl-pyrrolidin-3-yl)-methanontrifluoracetat

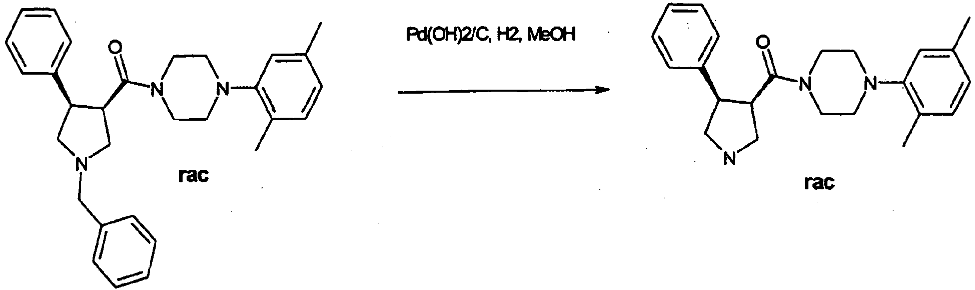

940 mg (1-Benzyl-3,4-cis-4-phenyl-pyrrolidin-3-yl)-[4-(2,5-dimethyl-phenyl)-piperazin-1-yl]-methanon-trifluoracetat werden in 10 ml Methanol gelöst und mit 50 mg Palladiumhydroxid auf Kohle, 20%, feucht, versetzt. Man rührt vierundzwanzig Stunden unter einer Wasserstoffatmosphäre von 5 bar Überdrucknach. Danach wird der Katalysator über Celiteabfiltriert, mit Ethylacetat nachgewaschen und das Filtrat im Vakuum eingeengt. Man erhält 1.1 g [4-(2,5-Dimethyl-phenyl)-piperazin-1-yl]-(3,4-cis-4-phenyl-pyrrolidin-3-yl)-methanon-trifluoracetat als farbloses Öl.
C23H29N30. C2HF302 (477.53), LCMS(ESI): 364.5 (M+H⁺).

### [4-(2,5-Dimethyl-phenyl)-piperazin-1-y1]-[3,4-cis-4-phenyl-1-(thiophen-2-sulfonyl)-pyrrolidin-3- yl]-methanon

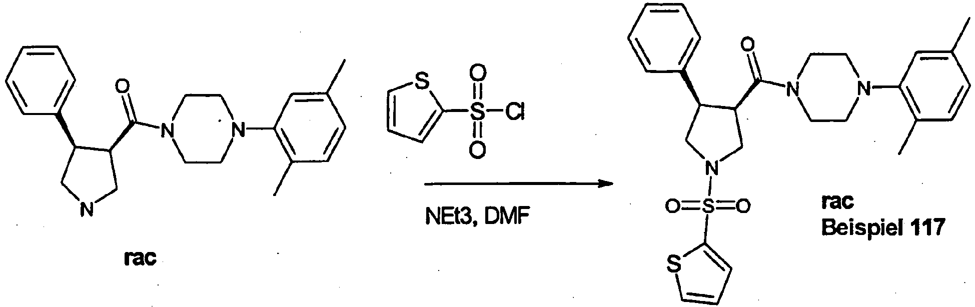

200 mg [4-(2,5-Dimethyl-phenyl)-piperazin-1-yl]-(3,4-cis-4-phenyl-pyrrolidin-3-yl)-methanontrifluoracetat, 200 µl N,N-Diisopropylethylamin und 100 mg 2-Thiophen-sulfonylchlorid werden in 10 ml N,N-Dimethylformamid gelöst und bei Raumtemperatur gerührt. Nach einer Stunde wird das Reaktionsgemsisch durch Zugabe von 100 ml Ethylacetat verdünnt und mit 50 ml gesättigter Natriumhydrogencarbonatlösung und dreimal mit je 50 ml Wasser gewaschen. Die organische Phase wird abgetrennt, über MgS04 getrocknet und das Lösungsmittel im Vakuum entfernt. Der Rückstand wird an Kieselgel mit dem Eluens n-Heptan:Ethylacetat = 20:1 => 10:1 => 5:1 => 2:1 gereinigt. Man erhält 120 mg [4-(2,5-Dimethyl-phenyl)-piperazin-1-yl]-[3,4-cis-4-phenyl-1-(thiophen-2-sulfonyl)-pyrrolidin-3- yl]-methanon als weißen amorphen Feststoff.
C27H31N3O3S2 (509.69), LCMS(ESI): 510.1 (M+H⁺).

Das Racemat [4-(2,5-Dimethyl-phenyl)-piperazin-1-yl]-[3,4-cis-4-phenyl-1-(thiophen-2-sulfonyl)-pyrrolidin-3- yl]-methanon wurde durch Chromatographie an chiraler Phase in die Enantiomere aufgetrennt. Man erhält [4-(2,5-Dimethyl-phenyl)-piperazin-1-yl]-[(3R,4R)/(3S,4S)-4-phenyl-1-(thiophen-2-sulfonyl)-pyrrolidin-3- yl]-methanon Beispiel 117A (Chiracel OJ/37 250x4.6 mm, Eluent Methanol, Rt = 9.4 min) und [4-(2,5-Dimethyl-phenyl)-piperazin-1-yl]-[(3S,4S)/(3R,4R)-4-phenyl-1-(thiophen-2-sulfonyl)-pyrrolidin-3- yl]-methanon Beispiel **117B** (Chiracel OJ/37 250x4.6 mm, Eluent Methanol, Rt = 14.9 min).

### Beispiel 118

### [1-(3,5-Dimethyl-isoxazole-4-sulfonyl)-3,4-cis-4-phenyl-pyrrolidin-3-yl]-[4-(2,5-dimethylphenyl)-piperazin-1-yl]-methanon-trifluoracetat

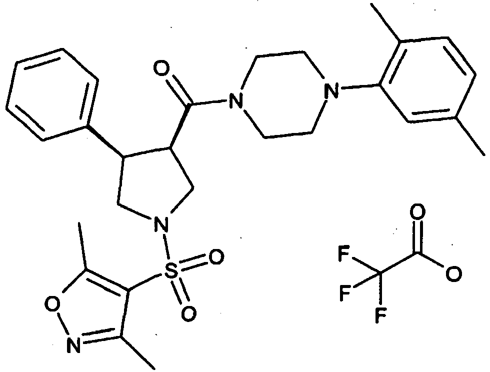

Analog zu Beispiel 117 erhält man aus den kommerziell erhältlichen Reagenzien Benzaldehyd, [Bis-(2,2,2-trifluorethoxy)-phosphoryl]-essigsäuremethylester, N-(Methoxymethyl)-N-(trimethylsilylmethyl)-benzylamin, 1-(2,5-Dimethyl-phenyl)-piperazin und 3,5-Dimethylisoxazol-4-sulfonylchlorid [1-(3,5-Dimethyl-isoxazole-4-sulfonyl)-3,4-cis-4-phenyl-pyrrolidin-3-yl]-[4-(2,5-dimethyl-phenyl)-piperazin-1-yl]-methanon-trifluoracetat.
C28H34N4O4S.C2HF3O2 (636.70), LCMS(ESI): 523.3 (M+H⁺).

### Beispiel 119

### [4-(2,5-Dimethyl-phenyl)-piperazin-1-yl]-[1-(3-methoxy-benzenesulfonyl)-3,4-cis-4-phenylpyrrolidin-3-yl]-methanon-trifluoracetat

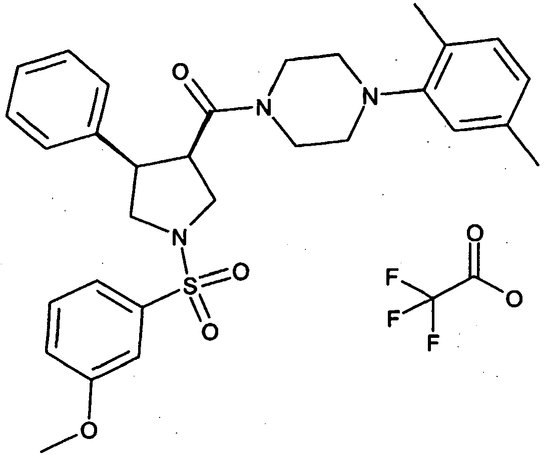

Analog zu Beispiel 117 erhält man aus den kommerziell erhältlichen Reagenzien Benzaldehyd, [Bis-(2,2,2-trifluorethoxy)-phosphoryl]-essigsäuremethylester, N-(Methoxymethyl)-N-(trimethylsilylmethyl)-benzylamin, 1-(2,5-Dimethyl-phenyl)-piperazin und 3-Methoxy-benzolsulfonylchlorid [4-(2,5-Dimethyl-phenyl)-piperazin-1-yl]-[1-(3-methoxybenzenesulfonyl)-3,4-cis-4-phenyl-pyrrolidin-3-yl]-methanon-trifluoracetat.
C30H35N3O4S.C2HF3O2 (647.72), LCMS(ESI): 534.3 (M+H⁺).

Das Racemat [[4-(2,5-Dimethyl-phenyl)-piperazin-1-yl]-[1-(3-methoxy-benzenesulfonyl)-3,4-cis-4-phenyl-pyrrolidin-3-yl]-methanon-trifluoracetat wurde durch Chromatographie an chiraler Phase in die Enantiomere aufgetrennt. Man erhält [4-(2,5-Dimethyl-phenyl)-piperazin-1-yl]-[1-(3-methoxy-benzenesulfonyl)-(3,R4R)/(3S,4S)-cis-4-phenyl-pyrrolidin-3-yl]-methanon Beispiel **119A** (Chiracel OJ/15 250x4.6 mm, Eluent Methanol, Rt = 9.8 min) und [4-(2,5-Dimethyl-phenyl)-piperazin-1-yl]-[1-(3-methoxy-benzenesulfonyl)- (3S,4S)/(3R,4R)-cis-4-phenyl-pyrrolidin-3-yl]-methanon Beispiel **119B** (Chiracel OJ/15 250x4.6 mm, Eluent Methanol, Rt = 17.3+ min).

### Beispiel 120

### [4-(2,5-Dimethyl-phenyl)-piperazin-1-yl]-[3,4-cis-4-phenyl-1-(pyridin-3-sulfonyl)-pyrrolidin-3-yl]-methanon-trifluoracetat

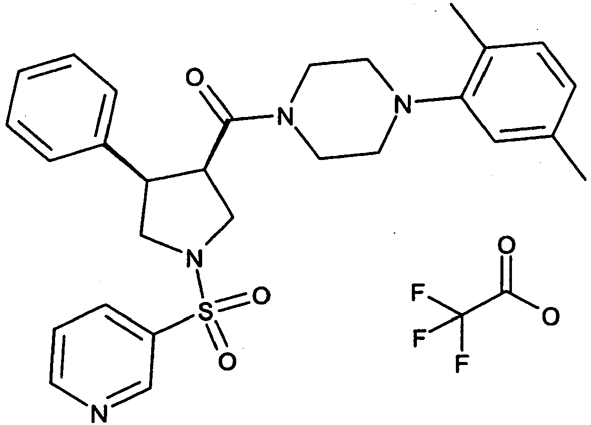

Analog zu Beispiel 117 erhält man aus den kommerziell erhältlichen Reagenzien Benzaldehyd, [Bis-(2,2,2-trifluorethoxy)-phosphoryl]-essigsäuremethylester, N-(Methoxymethyl)-N-(trimethylsilylmethyl)-benzylamin, 1-(2,5-Dimethyl-phenyl)-piperazin und Pyridin-3-sulfonylchlorid [4-(2,5-Dimethyl-phenyl)-piperazin-1-yl]-[3,4-cis-4-phenyl-1-(pyridin-3-sulfonyl)-pyrrolidin-3-yl]-methanon-trifluoracetat.
C28H32N4O3S.C2BF3O2 (618.68), LCMS(ESI): 505.2 (M+H⁺).

## Patentansprüche

1. Verbindungen der Formel I, 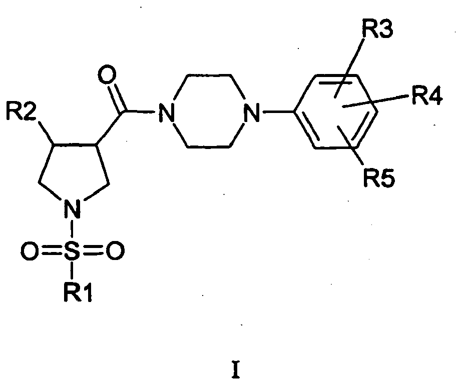 worin bedeuten
R1 (C₁-C₆)-Alkyl, wobei in dem Alkylrest ein oder mehrere Wasserstoffe durch Fluor ersetzt sein können, Phenyl, (C₁-C₈)-Alkylen-Phenyl, Heterocyclus, (C₁-C₈)-Akylen-Heterocyclus, wobei der Phenyl- oder Heterocyclus-rest ein oder mehrfach substituiert sein kann mit F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
R2 (C₁-C₆)-Alkyl, Phenyl, (C₁-C₈)-Alkylen-Phenyl, Heterocyclus, (C₁-C₈)-Alkylen-Heterocyclus, wobei der Phenyl- oder Heterocyclus-rest ein oder mehrfach substituiert sein kann mit F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
R3, R4, R5 unabhängig voneinander H, F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
wobei die Verbindungen der folgenden vier Formeln ausgeschlossen sind 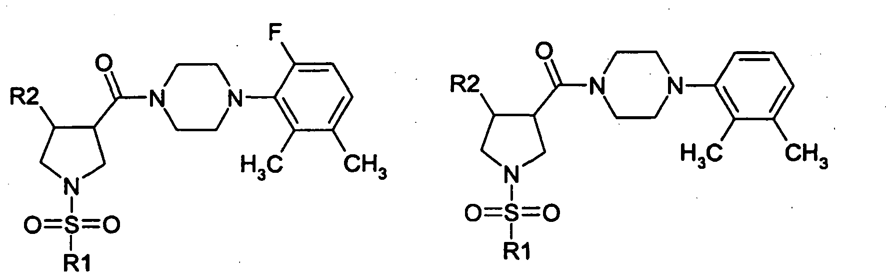 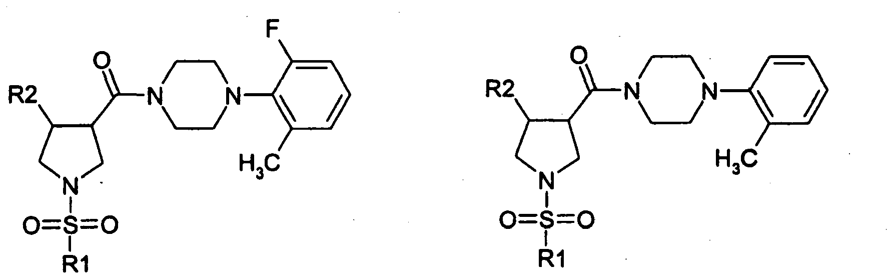 sowie deren physiologisch verträgliche Salze.

2. Verbindungen der Formel I, gemäß Anspruch 1, **dadurch gekennzeichnet, daß** darin bedeuten
R1 (C₁-C₆)-Alkyl, wobei in dem Alkylrest ein oder mehrere Wasserstoffe durch Fluor ersetzt sein können, Phenyl, (C₁-C₈)-Alkylen-Phenyl, Heterocyclus, (C₁-C₈)-Alkylen-Heterocyclus, wobei der Phenyl- oder Heterocyclus-rest ein oder mehrfach substituiert sein kann mit F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
R2 Phenyl, Heterocyclus, wobei der Phenyl- oder Heterocyclus-rest ein oder mehrfach substituiert sein kann mit F, Cl, Br, NO₂, COO-(C₁-C₆)-Alkyl, (C₁-C₆)- Alkylen-O-(C₁-C₆)-alkyl, OCF₃, (C₁-C₆)-Alkyl, CO-(C₁-C₆)-Alkyl, Phenyl, SCF₃, SF₅; R3, R4, R5 unabhängig voneinander H, F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
wobei die Verbindungen der folgenden vier Formeln ausgeschlossen sind 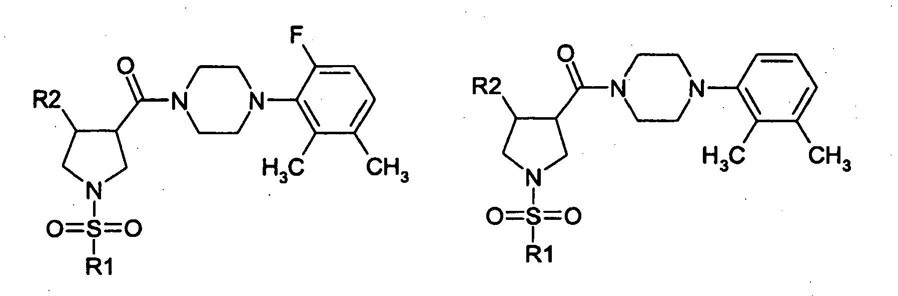 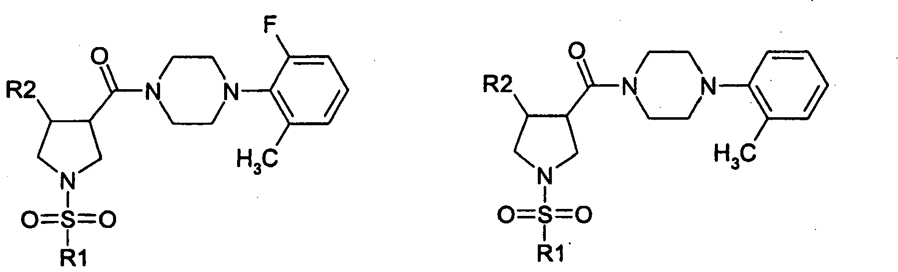 sowie deren physiologisch verträgliche Salze.

3. Verbindungen der Formel I, gemäß Anspruch 1 oder 2, **dadurch gekennzeichnet, daß** sie die Struktur Ia besitzen 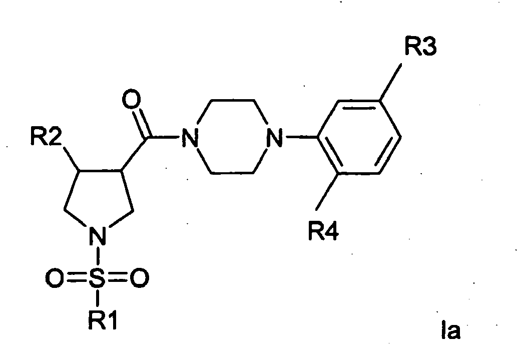 worin ein oder mehrere Reste folgende Bedeutung haben:
R1 (C₁-C₆)-Alkyl, wobei in dem Alkylrest ein oder mehrere Wasserstoffe durch Fluor ersetzt sein können, Phenyl, (C₁-C₈)-Alkylen-Phenyl, Heterocyclus, (C₁-C₈)-Alkylen-Heterocyclus, wobei der Phenyl- oder Heterocyclus-rest ein oder mehrfach substituiert sein kann mit F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
R2 Phenyl, Heterocyclus, wobei der Phenyl- oder Heterocyclus-rest ein oder mehrfach substituiert sein kann mit F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
R3, R4 unabhängig voneinander H, F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
wobei die Verbindung der folgenden Formel ausgeschlossen ist 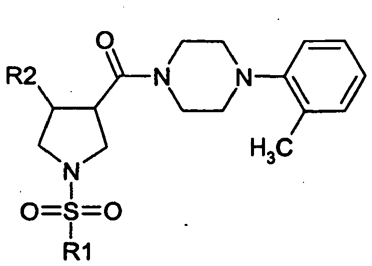 sowie deren physiologisch verträgliche Salze.

4. Verbindungen der Formel I, gemäß Anspruch 1 oder 2, oder der Formel Ia gemäß Anspruch 3, **dadurch gekennzeichnet, daß** darin ein mehrere Reste folgende Bedeutung haben:
R1 (C₁-C₆)-Alkyl, wobei in dem Alkylrest ein oder mehrere Wasserstoffe durch Fluor ersetzt sein können, Phenyl, (C₁-C₈)-Alkylen-Phenyl, Heterocyclus, (C₁-C₈)-Alkylen-Heterocyclus, wobei der Phenyl- oder Heterocyclus-rest ein oder mehrfach substituiert sein kann mit F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅ und der Heterocylus aus der Gruppe Thiophen, Quinolin, Oxadiazol, Isoxazol und Pyridin ausgewählt ist und mit einem Benzolring anneliert sein kann.
R2 Phenyl, Heterocyclus, wobei der Phenyl- oder Heterocyclus-rest ein oder mehrfach substituiert sein kann mit F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅ und der Heterocylus aus der Gruppe Dioxol, Tetrahydrofuran, Isoxazol, Oxazin, Thiophen und Pyridin ausgewählt ist und mit einem Benzolring anneliert sein kann.
R3, R4 unabhängig voneinander H, F, Cl, Br, NO₂, COO-(C₁-C₆)-Alkyl, (C₁-C₆)-Alkylen-O-(C₁-C₆)-alkyl, OCF₃, (C₁-C₆)-Alkyl, CO-(C₁-C₆)-Alkyl, Phenyl, SCF₃, SF₅;
wobei die Verbindung der folgenden Formel ausgeschlossen ist 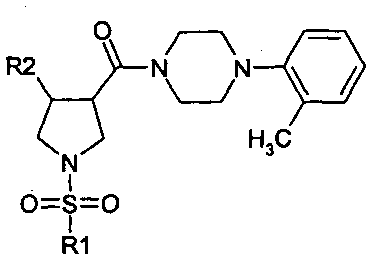 sowie deren physiologisch verträgliche Salze.

5. Verwendung der Verbindungen gemäß einem oder mehreren der Ansprüche 1 bis 4 zur Herstellung eines Arzneimittels.

6. Arzneimittel enthaltend eine oder mehrere der Verbindungen gemäß einem oder mehreren der Ansprüche 1 bis 4.

7. Arzneimittel enthaltend eine oder mehrere der Verbindungen gemäß einem oder mehreren der Ansprüche 1 bis 4 und mindestens einen weiteren Wirkstoff.

8. Arzneimittel, gemäß Anspruch 7, **dadurch gekennzeichnet, daß** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylharnstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

9. Verwendung der Verbindungen gemäß einem oder mehreren der Ansprüche 1 bis 3 zur Herstellung eines Medikamentes zur HDL Erhöhung im Blut.

10. Verwendung der Verbindungen gemäß einem oder mehreren der Ansprüche 1 bis 4 zur Herstellung eines Medikamentes zur Behandlung von Dyslipidämie.

11. Verwendung der Verbindungen gemäß einem oder mehreren der Ansprüche 1 bis 4 zur Herstellung eines Medikamentes zur Behandlung von Erkrankungen des Herz-Kreislaufsystems.

12. Verwendung der Verbindungen gemäß einem oder mehreren der Ansprüche 1 bis 4 zur Herstellung eines Medikaments zur Behandlung arteriosklerotischer Erscheinungen.

13. Verwendung der Verbindungen gemäß einem oder mehreren der Ansprüche 1 bis 4 zur Herstellung eines Medikaments zur Behandlung des metabolischen Syndroms.

14. Verwendung der Verbindungen gemäß einem oder mehreren der Ansprüche 1 bis 4 zur Herstellung eines Medikaments zur Behandlung von Diabetes.

15. Verfahren zur Herstellung eines Arzneimittels enthaltend eine oder mehrere der Verbindungen gemäß einem oder mehreren der Ansprüche 1 bis 4, **dadurch gekennzeichnet, daß** der Wirkstoff mit einem pharmazeutisch geeigneten Träger vermischt wird und diese Mischung in eine für die Verabreichung geeignete Form gebracht wird.

## Claims

1. A compound of the formula I 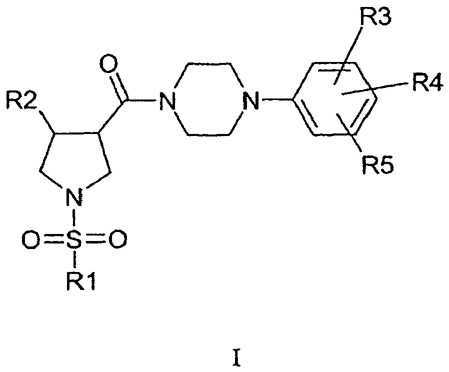 in which the meanings are
R1 (C₁-C₆)-alkyl, where one or more hydrogens in the alkyl radical may be replaced by fluorine, or phenyl, (C₁-C₈)-alkylene-phenyl, heterocycle, (C₁-C₈)-alkylene-heterocycle, where the phenyl or heterocycle radical may be substituted one or more times by F, Cl, Br, NO₂, COO-(C₁-C₆)-alkyl, (C₁-C₆)-alkylene-O-(C₁-C₆)-alkyl, OCF₃, (C₁-C₆)-alkyl, CO-(C₁-C₆)-alkyl, phenyl, SCF₃, SF₅;
R2 (C₁-C₆)-alkyl, phenyl, (C₁-C₈)-alkylene-phenyl, heterocycle, (C₁-C₈)-alkylene-heterocycle, where the phenyl or heterocycle radical may be substituted one or more times by F, Cl, Br, NO₂, COO-(C₁-C₆)-alkyl, (C₁-C₆)-alkylene-O-(C₁-C₆)-alkyl, OCF₃, (C₁-C₆)-alkyl, CO-(C₁-C₆)-alkyl, phenyl, SCF₃, SF₅;
R3, R4, R5 independently of one another H, F, Cl, Br, NO₂, COO-(C₁-C₆)-alkyl, (C₁-C₆)-alkylene-O-(C₁-C₆)-alkyl, OCF₃, (C₁-C₆)-alkyl, CO-(C₁-C₆)-alkyl, phenyl, SCF₃, SF₅;
where the compounds of the following four formulae are excluded 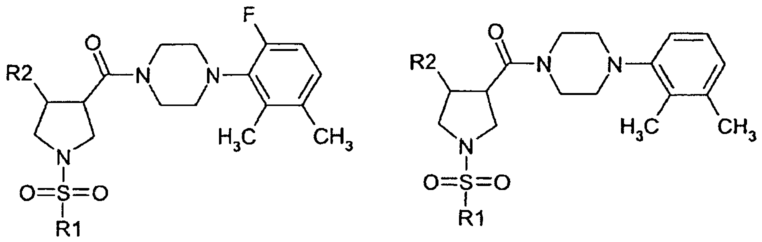 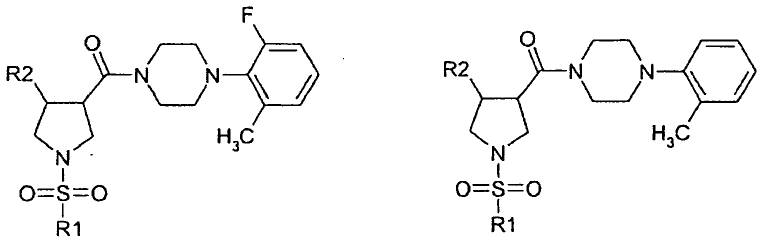 and the physiologically tolerated salts thereof.

2. A compound of the formula I as claimed in claim 1, wherein the meanings are
R1 (C₁-C₆)-alkyl, where one or more hydrogens in the alkyl radical may be replaced by fluorine, or phenyl, (C₁-C₈)-alkylene-phenyl, heterocycle, (C₁-C₈)-alkylene-heterocycle, where the phenyl or heterocycle radical may be substituted one or more times by F, Cl, Br, NO₂, COO-(C₁-C₆)-alkyl, (C₁-C₆)-alkylene-O-(C₁-C₆)-alkyl, OCF₃, (C₁-C₆)-alkyl, CO-(C₁-C₆)-alkyl, phenyl, SCF₃, SF₅;
R2 phenyl, heterocycle, where the phenyl or heterocycle radical may be substituted one or more times by F, Cl, Br, NO₂, COO-(C₁-C₆)-alkyl, (C₁-C₆)-alkylene-O-(C₁-C₆)-alkyl, OCF₃, (C₁-C₆)-alkyl, CO-(C₁-C₆)-alkyl, phenyl, SCF₃, SF₅;
R3, R4, R5 independently of one another H, F, Cl, Br, NO₂, COO-(C₁-C₆)-alkyl, (C₁-C₆)-alkylene-O-(C₁-C₆)-alkyl, OCF₃, (C₁-C₆)-alkyl, CO-(C₁-C₆)-alkyl, phenyl, SCF₃, SF₅;
where the compounds of the following four formulae are excluded 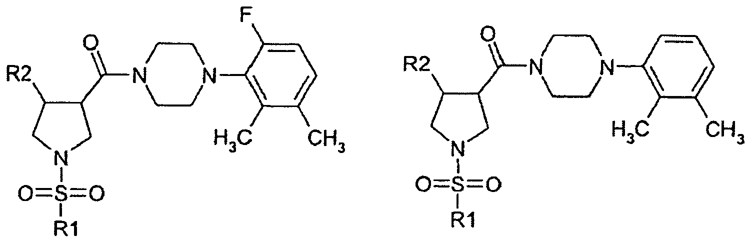 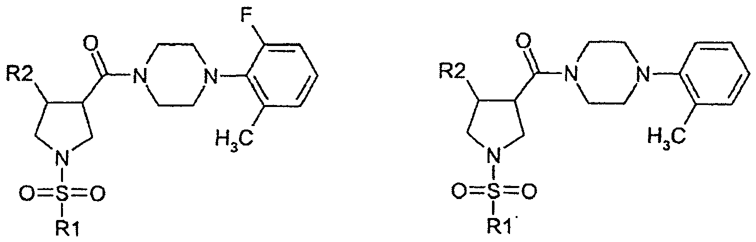 and the physiologically tolerated salts thereof.

3. A compound of the formula I as claimed in claim 1 or 2, which has the structure la 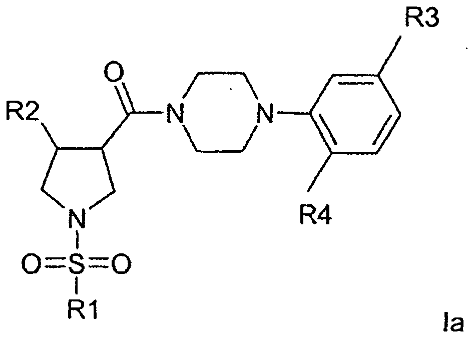 in which one or more radicals have the following meaning:
R1 (C₁-C₆)-alkyl, where one or more hydrogens in the alkyl radical may be replaced by fluorine, or phenyl, (C₁-C₈)-alkylene-phenyl, heterocycle, (C₁-C₈)-alkylene-heterocycle, where the phenyl or heterocycle radical may be substituted one or more times by F, Cl, Br, NO₂, COO-(C₁-C₆)-alkyl, (C₁-C₆)-alkylene-O-(C₁-C₆)-alkyl, OCF₃, (C₁-C₆)-alkyl, CO-(C₁-C₆)-alkyl, phenyl, SCF₃, SF₅;
R2 phenyl, heterocycle, where the phenyl or heterocycle radical may be substituted one or more times by F, Cl, Br, NO₂, COO-(C₁-C₆)-alkyl, (C₁-C₆)-alkylene-O-(C₁-C₆)-alkyl, OCF₃, (C₁-C₆)-alkyl, CO-(C₁-C₆)-alkyl, phenyl, SCF₃, SF₅;
R3, R4, independently of one another H, F, Cl, Br, NO₂, COO-(C₁-C₆)-alkyl, (C₁-C₆)-alkylene-O-(C₁-C₆)-alkyl, OCF₃, (C₁-C₆)-alkyl, CO-(C₁-C₆)-alkyl, phenyl, SCF₃, SF₅;
where the compound of the following formula is excluded 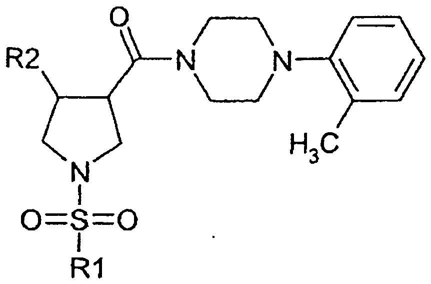 and the physiologically tolerated salts thereof.

4. A compound of the formula I as claimed in claim 1 or 2, or of the formula la as claimed in claim 3, wherein one or more radicals have the following meaning:
R1 (C₁-C₆)-alkyl, where one or more hydrogens in the alkyl radical may be replaced by fluorine, or phenyl, (C₁-C₈)-alkylene-phenyl, heterocycle, (C₁-C₈)-alkylene-heterocycle, where the phenyl or heterocycle radical may be substituted one or more times by F, Cl, Br, NO₂, COO-(C₁-C₆)-alkyl, (C₁-C₆)-alkylene-O-(C₁-C₆)-alkyl, OCF₃, (C₁-C₆)-alkyl, CO-(C₁-C₆)-alkyl, phenyl, SCF₃, SF₅ and the heterocycle is selected from the group of thiophene, quinoline, oxadiazole, isoxazole and pyridine and may be fused to a benzene ring.
R2 phenyl, heterocycle, where the phenyl or heterocycle radical may be substituted one or more times by F, Cl, Br, NO₂, COO-(C₁-C₆)-alkyl, (C₁-C₆)-alkylene-O-(C₁-C₆)-alkyl, OCF₃, (C₁-C₆)-alkyl, CO-(C₁-C₆)-alkyl, phenyl, SCF₃, SF₅ and the heterocycle is selected from the group of dioxole, tetrahydrofuran, isoxazole, oxazine, thiophene and pyridine and may be fused to a benzene ring.
R3, R4, independently of one another H, F, Cl, Br, NO₂, COO-(C₁-C₆)-alkyl, (C₁-C₆)-alkylene-O-(C₁-C₆)-alkyl, OCF₃, (C₁-C₆)-alkyl, CO-(C₁-C₆)-alkyl, phenyl, SCF₃, SF₅;
where the compound of the following formula is excluded 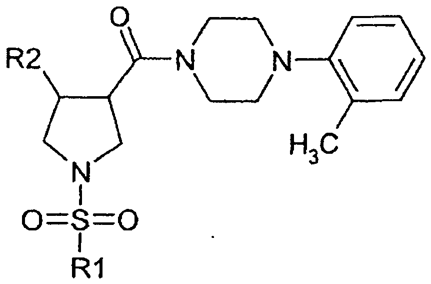 and the physiologically tolerated salts thereof.

5. The use of the compounds as claimed in one or more of claims 1 to 4 for producing a medicament.

6. A medicament comprising one or more of the compounds as claimed in one or more of claims 1 to 4.

7. A medicament comprising one or more of the compounds as claimed in one or more of claims 1 to 4 and at least one other active ingredient.

8. The medicament as claimed in claim 7, wherein the other active ingredient comprises one or more antidiabetics, hypoglycemic active ingredients, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients which act on the ATP-dependent potassium channel of the beta cells, CART agonists, NPY agonists, MC4 agonists, orexin agonists, H3 agonists, TNF agonists, CRF agonists, CRF BP antagonists, urocortin agonists, β3 agonists, MSH (melanocyte-stimulating hormone) agonists, CCK agonists, serotonin reuptake inhibitors, mixed sertoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

9. The use of the compounds as claimed in one or more of claims 1 to 3 for producing a medicament for increasing HDL in the blood.

10. The use of the compounds as claimed in one or more of claims 1 to 4 for producing a medicament for the treatment of dyslipidemia.

11. The use of the compounds as claimed in one or more of claims 1 to 4 for producing a medicament for the treatment of disorders of the cardiovascular system.

12. The use of the compounds as claimed in one or more of claims 1 to 4 for producing a medicament for the treatment of arteriosclerotic manifestations.

13. The use of the compounds as claimed in one or more of claims 1 to 4 for producing a medicament for the treatment of the metabolic syndrome.

14. The use of the compounds as claimed in one or more of claims 1 to 4 for producing a medicament for the treatment of diabetes.

15. A process for producing a medicament comprising one or more of the compounds as claimed in one or more of claims 1 to 4, which comprises mixing the active ingredient with a pharmaceutically suitable carrier and converting this mixture into a form suitable for administration.

## Revendications

1. Composés de formule I 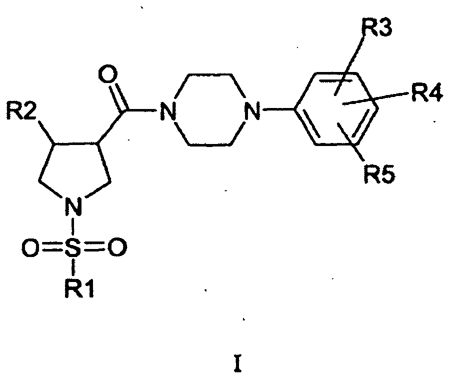 dans laquelle
R1 signifie alkyle en C₁-C₆, un ou plusieurs hydrogènes dans le radical alkyle pouvant être remplacés par du fluor, phényle, alkylène en C₁-C₈-phényle, hétérocycle, alkylène en C₁-C₈-hétérocycle, le radical phényle ou hétérocycle pouvant être substitué une ou plusieurs fois par F, CI, Br, NO₂, COO-alkyle en C₁-C₆, alkylène en C₁-C₆-O-alkyle en C₁-C₆, OCF₃, alkyle en C₁-C₆, CO-alkyle en C₁-C₆, phényle, SCF₃, SF₅ ;
R2 signifie alkyle en C₁-C₆, phényle, alkylène en C₁-C₈-phényle, hétérocycle, alkylène en C₁-C₈-hétérocycle, le radical phényle ou hétérocycle pouvant être substitué une ou plusieurs fois par F, CI, Br, NO₂, COO-alkyle en C₁-C₆, alkylène en C₁-C₆-O-alkyle en C₁-C₆, OCF₃, alkyle en C₁-C₆, CO-alkyle en C₁-C₆, phényle, SCF₃, SF₅ ;
R3, R4, R5 signifient indépendamment les uns des autres H, F, CI, Br, NO₂, COO-alkyle en C₁-C₆, alkylène en C₁-C₆-O-alkyle en C₁-C₆, OCF₃, alkyle en C₁-C₆, CO-alkyle en C₁-C₆, phényle, SCF₃, SF₅ ;
les composés des quatre formules suivantes étant exclus 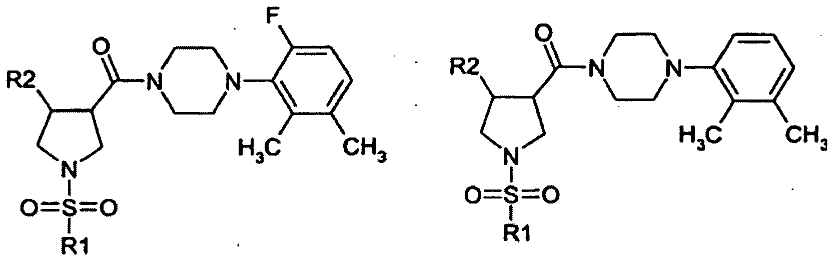 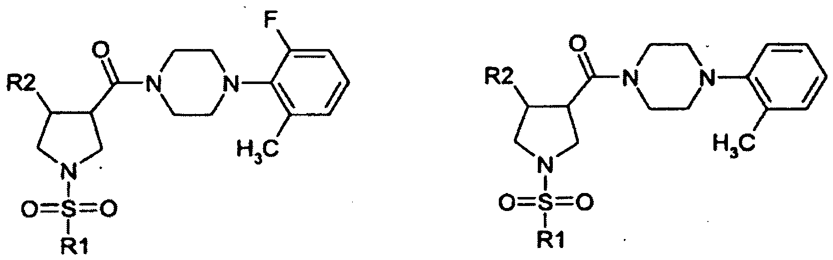 ainsi que leurs sels physiologiquement compatibles.

2. Composés de formule I selon la revendication 1, **caractérisés en ce que**
R1 signifie alkyle en C₁-C₆, un ou plusieurs hydrogènes dans le radical alkyle pouvant être remplacés par du fluor, phényle, alkylène en C₁-C₈-phényle, hétérocycle, alkylène en C₁-C₈-hétérocycle, le radical phényle ou hétérocycle pouvant être substitué une ou plusieurs fois par F, CI, Br, NO₂, COO-alkyle en C₁-C₆, alkylène en C₁-C₆-O-alkyle en C₁-C₆, OCF₃, alkyle en C₁-C₆, CO-alkyle en C₁-C₆, phényle, SCF₃, SF₅ ;
R2 signifie phényle, hétérocycle, le radical phényle ou hétérocycle pouvant être substitué une ou plusieurs fois par F, Cl, Br, NO₂, COO-alkyle en C₁-C₆, alkylène en C₁-C₆-O-alkyle en C₁-C₆, OCF₃, alkyle en C₁-C₆, CO-alkyle en C₁-C₆, phényle, SCF₃, SF₅ ;
R3, R4, R5 signifient indépendamment les uns des autres H, F, CI, Br, NO₂, COO-alkyle en C₁-C₆, alkylène en C₁-C₆-O-alkyle en C₁-C₆, OCF₃, alkyle en C₁-C₆, CO-alkyle en C₁-C₆, phényle, SCF₃, SF₅ ;
les composés des quatre formules suivantes étant exclus 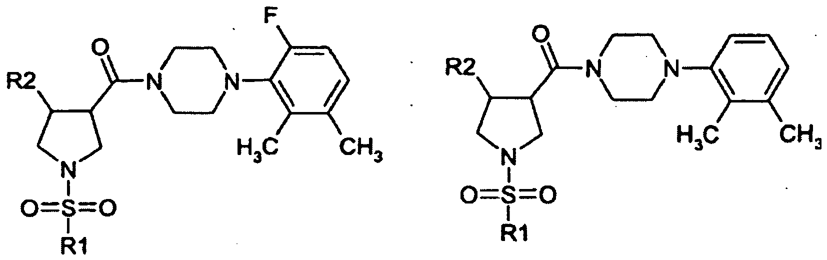 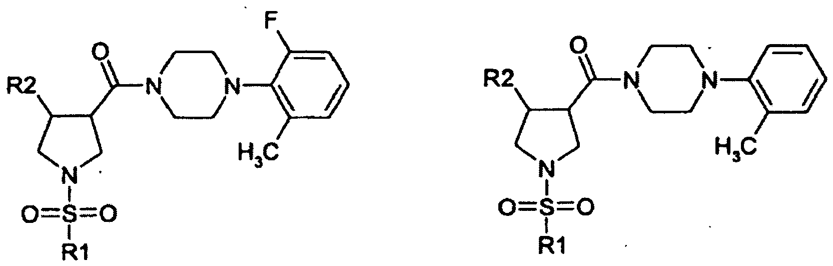 ainsi que leurs sels physiologiquement compatibles.

3. Composés de formule I selon la revendication 1 ou 2, **caractérisés en ce qu'**ils possèdent la structure la 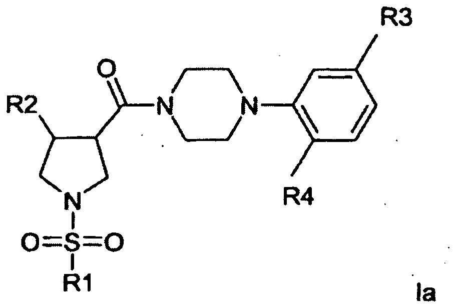 dans laquelle un ou plusieurs des radicaux ont la signification suivante :
R1 alkyle en C₁-C₆, un ou plusieurs hydrogènes dans le radical alkyle pouvant être remplacés par du fluor, phényle, alkylène en C₁-C₈-phényle, hétérocycle, alkylène en C₁-C₈-hétérocycle, le radical phényle ou hétérocycle pouvant être substitué une ou plusieurs fois par F, CI, Br, NO₂, COO-alkyle en C₁-C₆, alkylène en C₁-C₆-O-alkyle en C₁-C₆, OCF₃, alkyle en C₁-C₆, CO-alkyle en C₁-C₆, phényle, SCF₃, SF₅ ;
R2 phényle, hétérocycle, le radical phényle ou hétérocycle pouvant être substitué une ou plusieurs fois par F, CI, Br, NO₂, COO-alkyle en C₁-C₆, alkylène en C₁-C₆-O-alkyle en C₁-C₆, OCF₃, alkyle en C₁-C₆, CO-alkyle en C₁-C₆, phényle, SCF₃, SF₅ ;
R3, R4 indépendamment l'un de l'autre H, F, Cl, Br, NO₂, COO-alkyle en C₁-C₆, alkylène en C₁-C₆-O-alkyle en C₁-C₆, OCF₃, alkyle en C₁-C₆, CO-alkyle en C₁-C₆, phényle, SCF₃, SF₅ ;
le composé de la formule suivante étant exclu 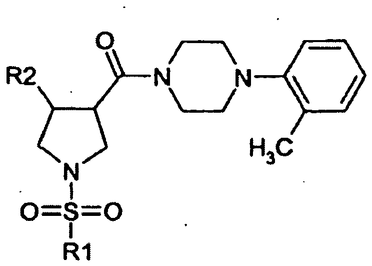 ainsi que leurs sels physiologiquement compatibles.

4. Composés de formule I selon la revendication 1 ou 2, ou de formule la selon la revendication 3, **caractérisés en ce qu'**un ou plusieurs radicaux dans celles-ci ont la signification suivante :
R1 alkyle en C₁-C₆, un ou plusieurs hydrogènes dans le radical alkyle pouvant être remplacés par du fluor, phényle, alkylène en C₁-C₈-phényle, hétérocycle, alkylène en C₁-C₈-hétérocycle, le radical phényle ou hétérocycle pouvant être substitué une ou plusieurs fois par F, CI, Br, NO₂, COO-alkyle en C₁-C₆, alkylène en C₁-C₆-O-alkyle en C₁-C₆, OCF₃, alkyle en C₁-C₆, CO-alkyle en C₁-C₆, phényle, SCF₃, SF₅, et l'hétérocycle étant choisi dans le groupe constitué par thiophène, quinoline, oxadiazole, isoxazole et pyridine, et pouvant être annelé avec un cycle benzène,
R2 phényle, hétérocycle, le radical phényle ou hétérocycle pouvant être substitué une ou plusieurs fois par F, CI, Br, NO₂, COO-alkyle en C₁-C₆, alkylène en C₁-C₆-O-alkyle en C₁-C₆, OCF₃, alkyle en C₁-C₆, CO-alkyle en C₁-C₆, phényle, SCF₃, SF₅, et l'hétérocycle étant choisi dans le groupe constitué par dioxole, tétrahydrofurane, isoxazole, oxazine, thiophène et pyridine, et pouvant être annelé avec un cycle benzène,
R3, R4 indépendamment l'un de l'autre H, F, CI, Br, NO₂, COO-alkyle en C₁-C₆, alkylène en C₁-C₆-O-alkyle en C₁-C₆, OCF₃, alkyle en C₁-C₆, CO-alkyle en C₁-C₆, phényle, SCF₃, SF₅ ;
le composé de la formule suivante étant exclu 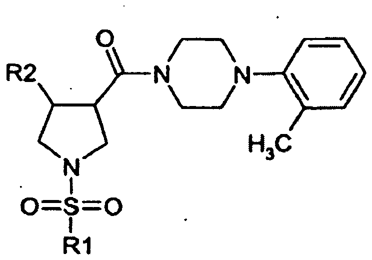 ainsi que leurs sels physiologiquement compatibles.

5. Utilisation des composés selon une ou plusieurs des revendications 1 à 4 pour la fabrication d'un médicament.

6. Médicament contenant un ou plusieurs des composés selon une ou plusieurs des revendications 1 à 4.

7. Médicament contenant un ou plusieurs des composés selon une ou plusieurs des revendications 1 à 4 et au moins un agent actif supplémentaire.

8. Médicament selon la revendication 7, **caractérisé en ce qu'**il contient en tant qu'agent actif supplémentaire un ou plusieurs antidiabétiques, agents actifs hypoglycémiques, inhibiteurs d'HMGCoA-réductase, inhibiteurs de résorption du cholestérol, agonistes de PPAR gamma, agonistes de PPAR alpha, agonistes de PPAR alpha/gamma, fibrates, inhibiteurs de MTP, inhibiteurs de résorption d'acides biliaires, inhibiteurs de CETP, adsorbeurs polymères d'acides biliaires, inducteurs des récepteurs de LDL, inhibiteurs d'ACAT, antioxydants, inhibiteurs de lipoprotéine-lipase, inhibiteurs d'ATP-citrate-lyase, inhibiteurs de squalène synthétase, antagonistes de lipoprotéine (a), inhibiteurs de lipase, insulines, sulfonylurées, biguanides, méglitinides, thiazolidinedione, inhibiteurs d'α-glucosidase, agents actifs agissant sur le canal calcique des cellules bêta dépendant de l'ATP, agonistes de CART, agonistes de NPY, agonistes de MC4, agonistes d'orexine, agonistes d'H3, agonistes de TNF, agonistes de CRF, antagonistes de CRF BP, agonistes d'urocortine, agonistes de β3, agonistes de MSH (hormone stimulant les mélanocytes), agonistes de CCK, inhibiteurs du recaptage de sérotonine, composés de sertonine et noradrénergiques mixtes, agonistes de 5HT, agonistes de bombésine, antagonistes de galanine, hormones de croissance, composés libérant des hormones de croissance, agonistes de TRH, modulateurs de protéine découplante 2 ou 3, agonistes de leptine, agonistes de DA (bromocriptine, doprexine), inhibiteurs de lipase/amylase, modulateurs de PPAR, modulateurs de RXR ou agonistes de TR-β ou amphétamines.

9. Utilisation des composés selon une ou plusieurs des revendications 1 à 3 pour la fabrication d'un médicament pour l'augmentation de l'HDL dans le sang.

10. Utilisation des composés selon une ou plusieurs des revendications 1 à 4 pour la fabrication d'un médicament pour le traitement de la dyslipidémie.

11. Utilisation des composés selon une ou plusieurs des revendications 1 à 4 pour la fabrication d'un médicament pour le traitement de maladies du système cardiovasculaire.

12. Utilisation des composés selon une ou plusieurs des revendications 1 à 4 pour la fabrication d'un médicament pour le traitement des phénomènes artério-sclérotiques.

13. Utilisation des composés selon une ou plusieurs des revendications 1 à 4 pour la fabrication d'un médicament pour le traitement du syndrome métabolique.

14. Utilisation des composés selon une ou plusieurs des revendications 1 à 4 pour la fabrication d'un médicament pour le traitement du diabète.

15. Procédé de fabrication d'un médicament contenant un ou plusieurs composés selon une ou plusieurs des revendications 1 à 4, **caractérisé en ce que** l'agent actif est mélangé avec un véhicule pharmaceutiquement approprié et ce mélange est mis sous une forme appropriée pour l'administration.
